# EUROPEAN PATENT APPLICATION

(11) **EP 4 663 226 A2**
(43) Date of publication of application: **17.12.2025**
(21) Application number: 25204590.1
(22) Date of filing: 29.07.2021
(51) Int. Cl.: A61M 16/16

(54) **A CONNECTION MEMBER**

(30) Priority: 30.07.2020 AU 2020902667
(62) Divisional of application: 21849481.3
(71) Applicant: ResMed Pty Ltd, Bella Vista, NSW 2153 (AU)
(72) Inventor: The designation of the inventor has not yet been filed
(74) Representative: Vossius & Partner Patentanwälte Rechtsanwälte mbB

(57) **Abstract**

The present invention relates to a connection member (6000) configured to connect an air circuit (4170) to a patient interface (3000) to convey a flow of pressurised breathable gas from the air circuit (4170) to the patient interface (3000) for breathing by a patient (1000). The connection member (6000) comprises a first tube portion (6110) having a patient-proximal end configured to connect to the patient interface (3000) and having a patient-distal end; and a second tube portion (6120) having a patient-proximal end and a patient-distal end configured to connect to the air circuit (4170), wherein at least a portion of the patient-proximal end of the second tube portion (6120) is positioned inside at least a portion of the patient-distal end of the first tube portion (6110), the flow of breathable gas being conveyed from the air circuit (4170) to the patient interface (3000) through the first tube portion (6110) and the second tube portion (6120). The connection member further comprises a vent (6100) for venting gas exhaled by the patient to the ambient air. The vent is formed between the first tube portion (6110) and the second tube portion (6120). The connection member also comprises an anti-asphyxia valve (AAV) (6200) comprising an opening (6210) to the ambient air and a closure (6220) configured to move between a first position in which the closure covers the opening and a second position in which the opening is uncovered. The AAV (6200) is located closer to the patient-proximal end of the first tube portion (6110) than the vent (6100).

## Description

### 1 BACKGROUND OF THE TECHNOLOGY

### 1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO₂ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 1.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 1.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

### 1.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired CO₂ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched gas at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 1.2.2.3 Supplementary oxygen

For certain patients, oxygen therapy may be combined with a respiratory pressure therapy or HFT by adding supplementary oxygen to the pressurised flow of air. When oxygen is added to respiratory pressure therapy, this is referred to as RPT with supplementary oxygen. When oxygen is added to HFT, the resulting therapy is referred to as HFT with supplementary oxygen.

### 1.2.3 Respiratory therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 1.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 1.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFT^{™} nasal pillows mask, SWIFT^{™} II nasal pillows mask, SWIFT^{™} LT nasal pillows mask, SWIFT^{™} FX nasal pillows mask and MIRAGE LIBERTY^{™} full-face mask. The following patent applications, assigned to ResMed Limited, describe examples of nasal pillows masks: International Patent Application WO2004/073,778 (describing amongst other things aspects of the ResMed Limited SWIFT^{™} nasal pillows), US Patent Application 2009/0044808 (describing amongst other things aspects of the ResMed Limited SWIFT^{™} LT nasal pillows); International Patent Applications WO 2005/063,328 and WO 2006/130,903 (describing amongst other things aspects of the ResMed Limited MIRAGE LIBERTY^{™} full-face mask); International Patent Application WO 2009/052,560 (describing amongst other things aspects of the ResMed Limited SWIFT^{™} FX nasal pillows).

### 1.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 1.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

### 1.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 1.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air. Humidifiers therefore often have the capacity to heat the flow of air was well as humidifying it.

### 1.2.3.5 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

Table of noise of prior masks (ISO 17510-2:2007, 10 cmH₂O pressure at 1m)

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage^{™} (*) | nasal | 29.5 | 21.5 | 1998 |
| ResMed UltraMirage^{™} | nasal | 36 (3) | 28 (3) | 2000 |
| ResMed Mirage Activa^{™} | nasal | 32 (3) | 24 (3) | 2002 |
| ResMed Mirage Micro^{™} | nasal | 30 (3) | 22 (3) | 2008 |
| ResMed Mirage^{™} SoftGel | nasal | 29 (3) | 22 (3) | 2008 |
| ResMed Mirage^{™} FX | nasal | 26 (3) | 18 (3) | 2010 |
| ResMed Mirage Swift^{™} (*) | nasal pillows | 37 | 29 | 2004 |
| ResMed Mirage Swift^{™} II | nasal pillows | 28 (3) | 20 (3) | 2005 |
| ResMed Mirage Swift^{™} LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| (* one specimen only, measured using test method specified in ISO 3744 in CPAP mode at 10 cmH₂O) | | | | |

Sound pressure values of a variety of objects are listed below

| Object | A-weighted sound pressure dB(A) | Notes |
|---|---|---|
| Vacuum cleaner: Nilfisk | 68 | ISO 3744 at 1m distance |
| Walter Broadly Litter Hog: B+ Grade | | |
| Conversational speech | 60 | 1m distance |
| Average home | 50 | |
| Quiet library | 40 | |
| Quiet bedroom at night | 30 | |
| Background in TV studio | 20 | |

### 2 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

An aspect of the present technology provides a connection member configured to connect an air circuit to a patient interface to convey a flow of pressurised breathable gas to a patient.

An aspect of the present technology provides a connection member configured to connect an air circuit to a patient interface to convey a flow of pressurised breathable gas to a patient, the connection member comprising a vent for venting gas exhaled by the patient to the ambient air and an anti-asphyxia valve (AAV).

An aspect of the present technology provides a connection member configured to connect an air circuit to a patient interface to convey a flow of pressurised breathable gas to a patient, the connection member comprising a vent for venting gas exhaled by the patient to the ambient air and an anti-asphyxia valve (AAV), wherein in use the AAV is located closer to the patient than the vent.

An aspect of the present technology provides a connection member configured to connect an air circuit to a patient interface to convey a flow of pressurised breathable gas to a patient, the connection member comprising a vent for venting gas exhaled by the patient to the ambient air and an anti-asphyxia valve (AAV), wherein the connection member is substantially straight and/or is compact in size.

An aspect of one form of the present technology is a connection member configured to connect an air circuit to a patient interface to convey a flow of pressurised breathable gas from the air circuit to the patient interface for breathing by a patient, the connection member comprising:
a first tube portion having a patient-proximal end configured to connect to the patient interface and having a patient-distal end, the first tube portion having a first longitudinal central axis that is substantially straight between the patient-proximal end and the patient-distal end;
a second tube portion having a patient-proximal end and a patient-distal end configured to connect to the air circuit, the second tube portion having a second longitudinal central axis that is substantially straight between the patient-proximal end and the patient-distal end, wherein the first tube portion and the second tube portion are arranged with the first longitudinal central axis of the first tube portion substantially parallel to the second longitudinal central axis of the second tube portion and at least a portion of the patient-proximal end of the second tube portion positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion; and
an anti-asphyxia valve (AAV) comprising an opening to the ambient air and a closure configured to move between a first position in which the closure covers the opening and a second position in which the opening is uncovered.

In examples: a) the AAV is located in a position on the connection member closer to the patient-proximal end of the first tube portion than the vent; b) the AAV is located proximate the patient-proximal end of the first tube portion; c) the vent is located proximate the patient-distal end of the first tube portion; d) the closure comprises a hingedly mounted flap; e) the flap is mounted to the first tube portion; f) the flap comprises a base portion configured to fit into a first aperture in the first tube portion; g) the base portion is configured to fit into the first aperture via a snap-fit connection; h) the opening is positioned to face an anterior direction when in use; i) the connection member further comprises a tube connector configured to connect the second tube portion to an outer surface of the first tube portion; j) the tube connector comprises a third tube portion mounted to an outer surface of the second tube portion, wherein the third tube portion surrounds the patient-distal end of the first tube portion; k) the first tube portion comprises a connecting portion configured to fit into a second aperture in the third tube portion; 1) the third tube portion comprises a connecting portion configured to fit into a second aperture in the first tube portion; m) the connecting portion fits into the second aperture via a snap-fit connection; n) the vent comprises a plurality of vent slots arranged in a region between the first tube portion and the second tube portion; o) the plurality of vent slots are arranged around the second tube portion; p) the vent slots are formed between a plurality of ribs extending between an outer surface of the second tube portion and an inner surface of the first tube portion; q) the vent comprises four vent slots; r) the plurality of vent slots are equally spaced apart; s) a plane of the patient-proximal end of the first tube portion is arranged at a substantially non-perpendicular angle to the first longitudinal central axis; t) the plane of the patient-proximal end of the first tube portion is angled with respect to the first longitudinal central axis so that, in use, the connection member is directed in an anterior-inferior direction; u) the first tube portion is cylindrical in shape; v) the second tube portion is cylindrical in shape; w) the vent slots are arranged around an annular region between the first and second tube portions; x) the first longitudinal central axis and the second longitudinal central axis are axially aligned; y) when the closure is in the second position, the closure substantially covers the patient-proximal end of the second tube portion thereby preventing flow of gas between the first tube portion and the second tube portion during use; z) in the second position the hingedly mounted flap contacts the patient-proximal end of the second tube portion; and/or aa) the patient-proximal end of the second tube portion is at a second tube angle to the second longitudinal central axis and, when the hingedly mounted flap contacts the patient-proximal end of the second tube portion, the hingedly mounted flap is oriented at substantially the second tube angle to the second longitudinal central axis, the second tube angle being substantially non-perpendicular.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient-proximal end configured to connect to the patient interface and having a patient-distal end;
a second tube portion having a patient-proximal end and a patient-distal end configured to connect to the air circuit, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion; and
an anti-asphyxia valve (AAV) comprising an opening to the ambient air and a closure configured to move between a first position in which the closure covers the opening and a second position in which the opening is uncovered;
wherein the AAV is located in a position on the connection member closer to the patient interface than the vent.

In examples: a) the AAV is located proximate the patient-proximal end of the first tube portion; b) the closure comprises a hingedly mounted flap; c) the flap is mounted to the first tube portion; d) the flap comprises a base portion configured to fit into a first aperture in the first tube portion; e) the base portion is configured to fit into the first aperture via a snap-fit connection; f) the opening is positioned to face an anterior direction when in use; g) the connection member further comprises a tube connector configured to connect the second tube portion to an outer surface of the first tube portion; h) the tube connector comprises a third tube portion mounted to an outer surface of the second tube portion, wherein the third tube portion surrounds the patient-distal end of the first tube portion; i) the first tube portion comprises a connecting portion configured to fit into a second aperture in the third tube portion; j) the third tube portion comprises a connecting portion configured to fit into a second aperture in the first tube portion; k) the connecting portion fits into the second aperture via a snap-fit connection; 1) the vent comprises a plurality of vent slots arranged in a region between the first tube portion and the second tube portion; m) the plurality of vent slots are arranged around the second tube portion; n) the vent slots are formed between a plurality of ribs extending between an outer surface of the second tube portion and an inner surface of the first tube portion; o) the vent comprises four vent slots; p) the plurality of vent slots are equally spaced apart; q) a plane of the patient-proximal end of the first tube portion is arranged at a substantially non-perpendicular angle to the first longitudinal central axis; r) the plane of the patient-proximal end of the first tube portion is angled with respect to the first longitudinal central axis so that, in use, the connection member is directed in an anterior-inferior direction; s) the first tube portion is cylindrical in shape; t) the second tube portion is cylindrical in shape; u) the vent slots are arranged around an annular region between the first and second tube portions; v) the first longitudinal central axis and the second longitudinal central axis are axially aligned; w) when the closure is in the second position, the closure substantially covers the patient-proximal end of the second tube portion thereby preventing flow of gas between the first tube portion and the second tube portion during use; x) in the second position the hingedly mounted flap contacts the patient-proximal end of the second tube portion; and/or y) the patient-proximal end of the second tube portion is at a second tube angle to the second longitudinal central axis and, when the hingedly mounted flap contacts the patient-proximal end of the second tube portion, the hingedly mounted flap is oriented at substantially the second tube angle to the second longitudinal central axis, the second tube angle being substantially non-perpendicular.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient proximal end configured to connect to the patient interface and having a patient-distal end;
a second tube portion having a patient-proximal end and a patient-distal end configured to connect to the air circuit, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion; and
an AAV comprising an opening to the ambient air and a closure configured to move between a first position in which the closure covers the opening and a second position in which the opening is uncovered;
wherein the connection member has a substantially straight central longitudinal axis.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient proximal end configured to connect to the patient interface and having a patient-distal end, the first tube portion having a first longitudinal central axis that is substantially straight between the patient-proximal end and the patient-distal end;
a second tube portion having a patient-proximal end and a patient-distal end configured to connect to the air circuit, the second tube portion having a second longitudinal central axis that is substantially straight between the patient-proximal end and the patient end, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion; and
an AAV comprising an opening to the ambient air and a closure configured to move between a first position in which the closure covers the opening and a second position in which the opening is uncovered;
wherein the first longitudinal central axis passes through an opening of the second tube portion and the second longitudinal central axis passes through an opening of the first tube portion.

Another aspect of one form of the present technology is an air circuit assembly configured to fluidly connect a respiratory therapy device to a patient interface, the air circuit assembly comprising
an air circuit; and
a connection member as described in any one of the other aspects of the technology above.

Another aspect of one form of the present technology is a patient interface assembly comprising:
a connection member as described in any one of the other aspects of the technology above; and
a patient interface, comprising:
   a plenum chamber pressurisable to a therapeutic pressure of at least 6 cmH₂O above ambient air pressure, said plenum chamber including a plenum chamber inlet port sized and structured to receive a flow of air at the therapeutic pressure for breathing by a patient; and
   a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use.

In examples: a) the plenum chamber is formed from a flexible material; b) the plenum chamber is formed from silicone; c) the seal-forming structure is configured to form a seal around both the patient's nose and mouth in use; d) the seal-forming structure is configured to form a seal over the bridge of the patient's nose in use; e) the seal-forming structure comprises an oral portion and a nasal portion; f) the nasal portion comprises a lip superior portion which contacts the lip superior of the patient in use; g) the nasal portion comprises a superior-facing medial portion which contacts the inferior and partially anterior surfaces of the patient's pronasale in use; h) the oral portion comprises a lip inferior portion which contacts the chin region of the patient in use; and/or i) the oral portion comprises an oral hole peripheral portion which contacts the cheeks of the patient in use.

An aspect of one form of the present technology is a connection member configured to connect an air circuit to a patient interface to convey a flow of pressurised breathable gas from the air circuit to the patient interface for breathing by a patient, the connection member comprising:
a first tube portion having a patient-proximal end configured to connect to the patient interface and having a patient-distal end, the first tube portion having a first longitudinal central axis that is substantially straight between the patient-proximal end and the patient-distal end;
a second tube portion having a patient-proximal end and a patient-distal end configured to connect to the air circuit, the second tube portion having a second longitudinal central axis that is substantially straight between the patient-proximal end and the patient-distal end, wherein the first tube portion and the second tube portion are arranged with the first longitudinal central axis of the first tube portion substantially parallel to the second longitudinal central axis of the second tube portion and at least a portion of the patient-proximal end of the second tube portion positioned inside at least a portion of the patient-distal end of the first tube portion, the flow of breathable gas being conveyed from the air circuit to the patient interface through the first tube portion and the second tube portion;
an anti-asphyxia valve (AAV) comprising an opening to the ambient air and a closure configured to move between a first position in which the closure covers the opening and a second position in which the opening is uncovered;
a vent for venting gas exhaled by the patient to the ambient air, the vent formed between the first tube portion and the second tube portion; wherein the vent comprises a vent structure comprising:
   a vent housing; and
   a plurality of partitions inside the vent housing, the plurality of partitions forming therebetween a plurality of vent slots, wherein the vent slots comprise:
      a vent inlet configured to receive an air flow; and
      a vent outlet configured to allow the air flow to exit into the surrounding ambient air,
   wherein the partitions are formed such that each vent slot has a cross-sectional area at a first region of the vent slots that is smaller than a cross-sectional area at a second region of the vent slots, wherein the first region is closer to the vent inlet than the second region.

An aspect of one form of the present technology is a connection member configured to connect an air circuit to a patient interface to convey a flow of pressurised breathable gas from the air circuit to the patient interface for breathing by a patient, the connection member comprising:
a first tube portion having a patient-proximal end configured to connect to the patient interface and having a patient-distal end, the first tube portion having a first longitudinal central axis that is substantially straight between the patient-proximal end and the patient-distal end;
a second tube portion having a patient-proximal end and a patient-distal end configured to connect to the air circuit, the second tube portion having a second longitudinal central axis that is substantially straight between the patient-proximal end and the patient-distal end, wherein the first tube portion and the second tube portion are arranged with the first longitudinal central axis of the first tube portion substantially parallel to the second longitudinal central axis of the second tube portion and at least a portion of the patient-proximal end of the second tube portion positioned inside at least a portion of the patient-distal end of the first tube portion, the flow of breathable gas being conveyed from the air circuit to the patient interface through the first tube portion and the second tube portion;
an anti-asphyxia valve (AAV) comprising an opening to the ambient air and a closure configured to move between a first position in which the closure covers the opening and a second position in which the opening is uncovered;
a vent for venting gas exhaled by the patient to the ambient air, the vent formed between the first tube portion and the second tube portion; wherein the vent comprises a vent structure comprising:
   a vent housing defining a plurality of vent slots, the vent slots comprising:
   a vent inlet configured to receive an air flow; and
   a vent outlet configured to allow the air flow to exit into the surrounding ambient air; and
a plurality of deflectors configured to deflect the air flow through each vent slot towards a part of the vent housing and/or another component in the respiratory system.

An aspect of one form of the present technology is a connection member configured to connect an air circuit to a patient interface to convey a flow of pressurised breathable gas from the air circuit to the patient interface for breathing by a patient, the connection member comprising:
a first tube portion having a patient-proximal end configured to connect to the patient interface and having a patient-distal end, the first tube portion having a first longitudinal central axis that is substantially straight between the patient-proximal end and the patient-distal end;
a second tube portion having a patient-proximal end and a patient-distal end configured to connect to the air circuit, the second tube portion having a second longitudinal central axis that is substantially straight between the patient-proximal end and the patient-distal end, wherein the first tube portion and the second tube portion are arranged with the first longitudinal central axis of the first tube portion substantially parallel to the second longitudinal central axis of the second tube portion and at least a portion of the patient-proximal end of the second tube portion positioned inside at least a portion of the patient-distal end of the first tube portion, the flow of breathable gas being conveyed from the air circuit to the patient interface through the first tube portion and the second tube portion;
an anti-asphyxia valve (AAV) comprising an opening to the ambient air and a closure configured to move between a first position in which the closure covers the opening and a second position in which the opening is uncovered;
a vent for venting gas exhaled by the patient to the ambient air, the vent formed between the first tube portion and the second tube portion; wherein the vent comprises a vent structure comprising:
   a vent housing defining a plurality of vent slots, the vent slots comprising:
   a vent inlet configured to receive an air flow; and
   a vent outlet configured to allow the air flow to exit into the surrounding ambient air; and
a projecting portion structured and arranged to inhibit generation of flow layer instabilities, the projecting portion extending outwardly from the vent outlet.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient-proximal end configured to connect to the patient interface and having a patient-distal end;
a second tube portion having a patient-proximal end and a patient-distal end configured to connect to the air circuit, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion; wherein the vent comprises a vent structure comprising:
   a vent housing; and
   a plurality of partitions inside the vent housing, the plurality of partitions forming therebetween a plurality of vent slots, wherein the vent slots comprise:
      a vent inlet configured to receive an air flow; and
      a vent outlet configured to allow the air flow to exit into the surrounding ambient air,
      wherein the partitions are formed such that each vent slot has a cross-sectional area at a first region of the vent slots that is smaller than a cross-sectional area at a second region of the vent slots, wherein the first region is closer to the vent inlet than the second region; and
wherein the connection member further comprises an anti-asphyxia valve (AAV) comprising an opening to the ambient air and a closure configured to move between a first position in which the closure covers the opening and a second position in which the opening is uncovered;
wherein the AAV is located in a position on the connection member closer to the patient interface than the vent.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient-proximal end configured to connect to the patient interface and having a patient-distal end;
a second tube portion having a patient-proximal end and a patient-distal end configured to connect to the air circuit, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion; wherein the vent comprises a vent structure comprising:
   a vent housing defining a plurality of vent slots, the vent slots comprising:
   a vent inlet configured to receive an air flow; and
   a vent outlet configured to allow the air flow to exit into the surrounding ambient air; and
a plurality of deflectors configured to deflect the air flow through each vent slot towards a part of the vent housing and/or another component in the respiratory system; and
wherein the connection member further comprises an anti-asphyxia valve (AAV) comprising an opening to the ambient air and a closure configured to move between a first position in which the closure covers the opening and a second position in which the opening is uncovered;
wherein the AAV is located in a position on the connection member closer to the patient interface than the vent.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient-proximal end configured to connect to the patient interface and having a patient-distal end;
a second tube portion having a patient-proximal end and a patient-distal end configured to connect to the air circuit, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion; wherein the vent comprises a vent structure comprising:
   a vent housing defining a plurality of vent slots, the vent slots comprising:
      a vent inlet configured to receive an air flow; and
      a vent outlet configured to allow the air flow to exit into the surrounding ambient air; and
   a projecting portion structured and arranged to inhibit generation of flow layer instabilities, the projecting portion extending outwardly from the vent outlet; and
   wherein the connection member further comprises an anti-asphyxia valve (AAV) comprising an opening to the ambient air and a closure configured to move between a first position in which the closure covers the opening and a second position in which the opening is uncovered;
   wherein the AAV is located in a position on the connection member closer to the patient interface than the vent.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient proximal end configured to connect to the patient interface and having a patient-distal end;
a second tube portion having a patient-proximal end and a patient-distal end configured to connect to the air circuit, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion, the vent comprising a vent structure comprising:
   a vent housing; and
   a plurality of partitions inside the vent housing, the plurality of partitions forming therebetween a plurality of vent slots, wherein the vent slots comprise:
      a vent inlet configured to receive an air flow; and
      a vent outlet configured to allow the air flow to exit into the surrounding ambient air,
   wherein the partitions are formed such that each vent slot has a cross-sectional area at a first region of the vent slots that is smaller than a cross-sectional area at a second region of the vent slots, wherein the first region is closer to the vent inlet than the second region; and
wherein the connection member further comprises an AAV comprising an opening to the ambient air and a closure configured to move between a first position in which the closure covers the opening and a second position in which the opening is uncovered;
wherein the connection member has a substantially straight central longitudinal axis.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient proximal end configured to connect to the patient interface and having a patient-distal end;
a second tube portion having a patient-proximal end and a patient-distal end configured to connect to the air circuit, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion, the vent comprising a vent structure comprising:
   a vent housing defining a plurality of vent slots, the vent slots comprising:
      a vent inlet configured to receive an air flow; and
      a vent outlet configured to allow the air flow to exit into the surrounding ambient air; and
a plurality of deflectors configured to deflect the air flow through each vent slot towards a part of the vent housing and/or another component in the respiratory system; and
wherein the connection member further comprises an AAV comprising an opening to the ambient air and a closure configured to move between a first position in which the closure covers the opening and a second position in which the opening is uncovered;
wherein the connection member has a substantially straight central longitudinal axis.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient proximal end configured to connect to the patient interface and having a patient-distal end;
a second tube portion having a patient-proximal end and a patient-distal end configured to connect to the air circuit, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion, the vent comprising a vent structure comprising:
   a vent housing defining a plurality of vent slots, the vent slots comprising:
   a vent inlet configured to receive an air flow; and
   a vent outlet configured to allow the air flow to exit into the surrounding ambient air; and
a projecting portion structured and arranged to inhibit generation of flow layer instabilities, the projecting portion extending outwardly from the vent outlet; and
wherein the connection member further comprises an AAV comprising an opening to the ambient air and a closure configured to move between a first position in which the closure covers the opening and a second position in which the opening is uncovered;
wherein the connection member has a substantially straight central longitudinal axis.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient proximal end configured to connect to the patient interface and having a patient-distal end, the first tube portion having a first longitudinal central axis that is substantially straight between the patient-proximal end and the patient-distal end;
a second tube portion having a patient-proximal end and a patient-distal end configured to connect to the air circuit, the second tube portion having a second longitudinal central axis that is substantially straight between the patient-proximal end and the patient end, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion, the vent comprising a vent structure comprising:
   a vent housing; and
   a plurality of partitions inside the vent housing, the plurality of partitions forming therebetween a plurality of vent slots, wherein the vent slots comprise:
      a vent inlet configured to receive an air flow; and
      a vent outlet configured to allow the air flow to exit into the surrounding ambient air,
   wherein the partitions are formed such that each vent slot has a cross-sectional area at a first region of the vent slots that is smaller than a cross-sectional area at a second region of the vent slots, wherein the first region is closer to the vent inlet than the second region; and
wherein the connection member further comprises an AAV comprising an opening to the ambient air and a closure configured to move between a first position in which the closure covers the opening and a second position in which the opening is uncovered;
wherein the first longitudinal central axis passes through an opening of the second tube portion and the second longitudinal central axis passes through an opening of the first tube portion.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient proximal end configured to connect to the patient interface and having a patient-distal end, the first tube portion having a first longitudinal central axis that is substantially straight between the patient-proximal end and the patient-distal end;
a second tube portion having a patient-proximal end and a patient-distal end configured to connect to the air circuit, the second tube portion having a second longitudinal central axis that is substantially straight between the patient-proximal end and the patient end, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion, the vent comprising a vent structure comprising:
   a vent housing defining a plurality of vent slots, the vent slots comprising:
   a vent inlet configured to receive an air flow; and
   a vent outlet configured to allow the air flow to exit into the surrounding ambient air; and
a plurality of deflectors configured to deflect the air flow through each vent slot towards a part of the vent housing and/or another component in the respiratory system; and
wherein the connection member further comprises an AAV comprising an opening to the ambient air and a closure configured to move between a first position in which the closure covers the opening and a second position in which the opening is uncovered;
wherein the first longitudinal central axis passes through an opening of the second tube portion and the second longitudinal central axis passes through an opening of the first tube portion.

Another aspect of one form of the present technology is a connection member configured to connect an air circuit to a patient interface to convey a flow of pressurised breathable gas from the air circuit to the patient interface for breathing by a patient, the connection member comprising:
a first tube portion having a patient proximal end configured to connect to the patient interface and having a patient-distal end, the first tube portion having a first longitudinal central axis that is substantially straight between the patient-proximal end and the patient-distal end;
a second tube portion having a patient-proximal end and a patient-distal end configured to connect to the air circuit, the second tube portion having a second longitudinal central axis that is substantially straight between the patient-proximal end and the patient end, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
a vent for venting gas exhaled by the patient to the ambient air, the vent formed between the first tube portion and the second tube portion, the vent comprising a vent structure comprising:
   a vent housing defining a plurality of vent slots, the vent slots comprising:
   a vent inlet configured to receive an air flow; and
   a vent outlet configured to allow the air flow to exit into the surrounding ambient air; and
a projecting portion structured and arranged to inhibit generation of flow layer instabilities, the projecting portion extending outwardly from the vent outlet; and
wherein the connection member further comprises an AAV comprising an opening to the ambient air and a closure configured to move between a first position in which the closure covers the opening and a second position in which the opening is uncovered;
wherein the first longitudinal central axis passes through an opening of the second tube portion and the second longitudinal central axis passes through an opening of the first tube portion.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 3 BRIEF DESCRIPTION OF THE DRAWINGS

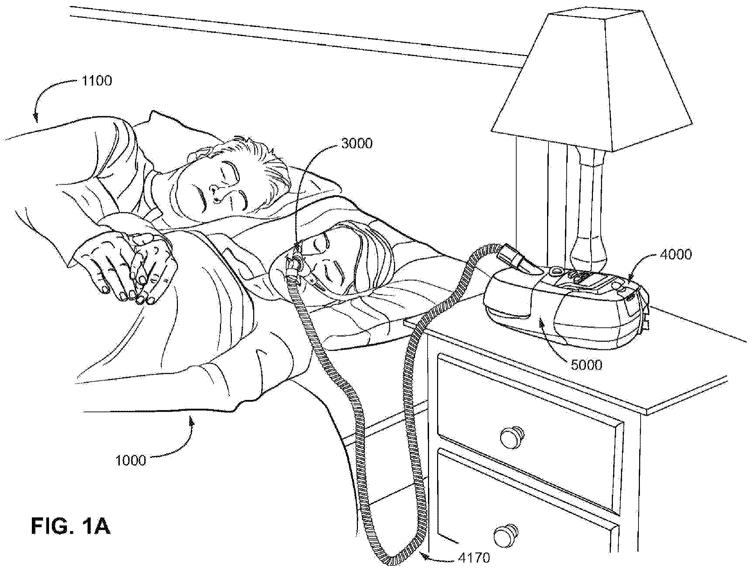

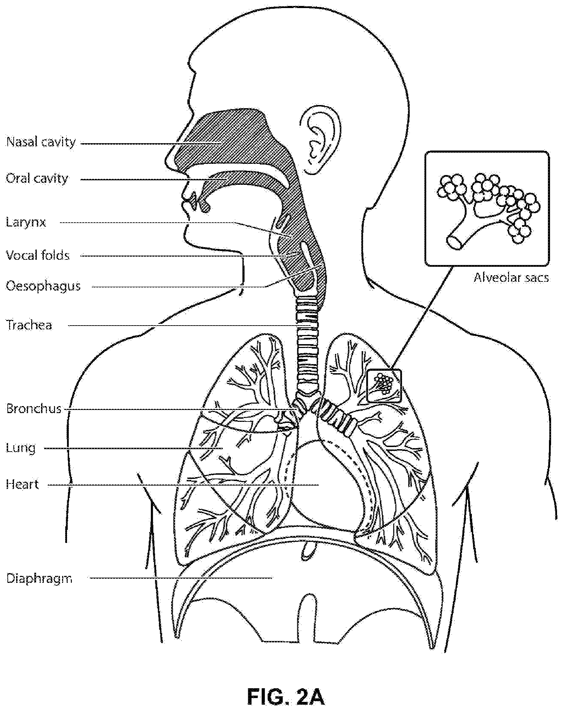

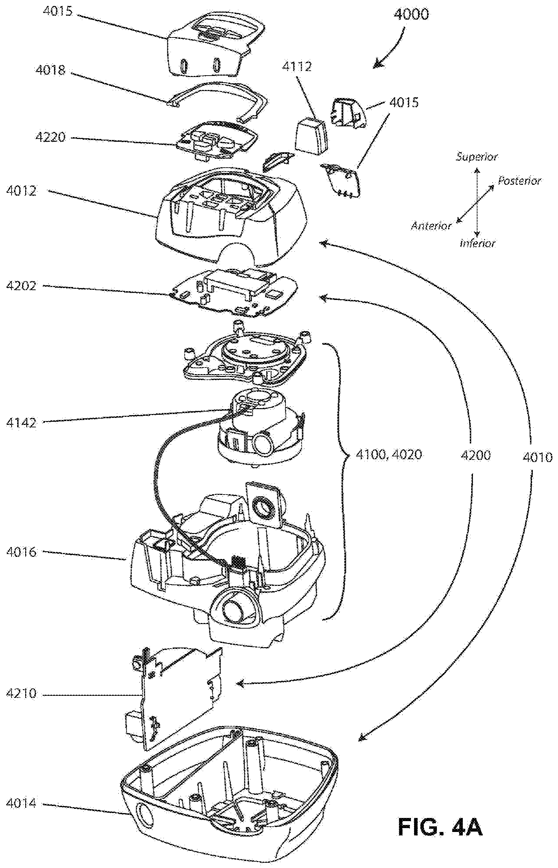

The present technology is illustrated by way of example, and not by way of limitation, in the figures of the accompanying drawings, in which like reference numerals refer to similar elements including:
3.1 RESPIRATORY THERAPY SYSTEMS
   Fig. 1A shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.

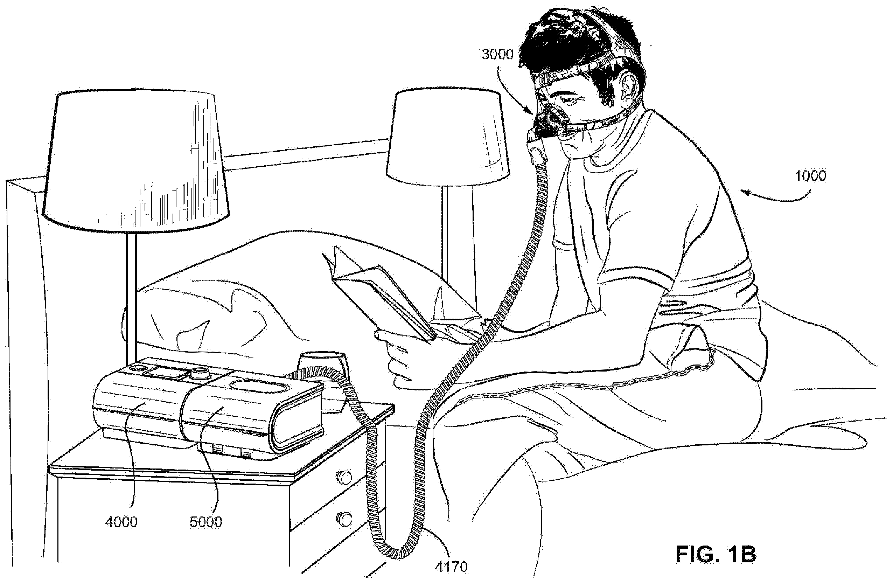

   Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

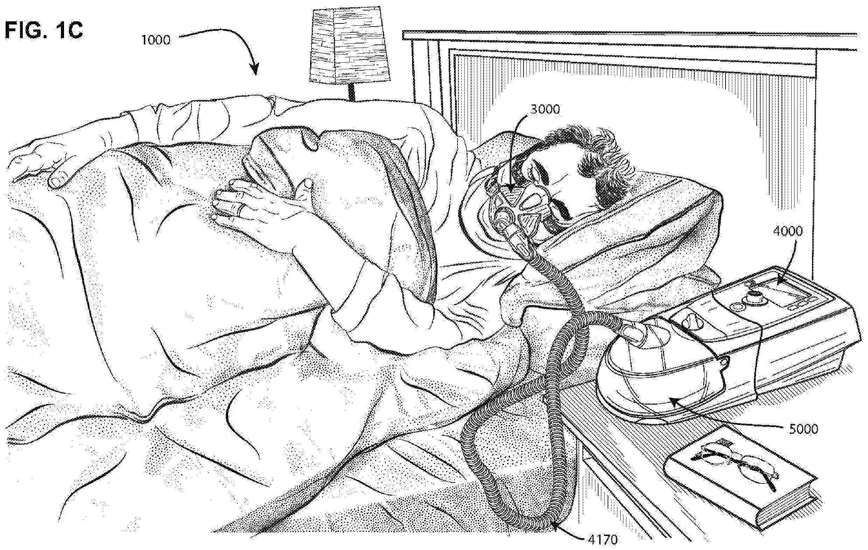

   Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.
3.2 RESPIRATORY SYSTEM AND FACIAL ANATOMY
   Fig. 2A shows an overview of a human respiratory system including the nasal and oral cavities, the larynx, vocal folds, oesophagus, trachea, bronchus, lung, alveolar sacs, heart and diaphragm.

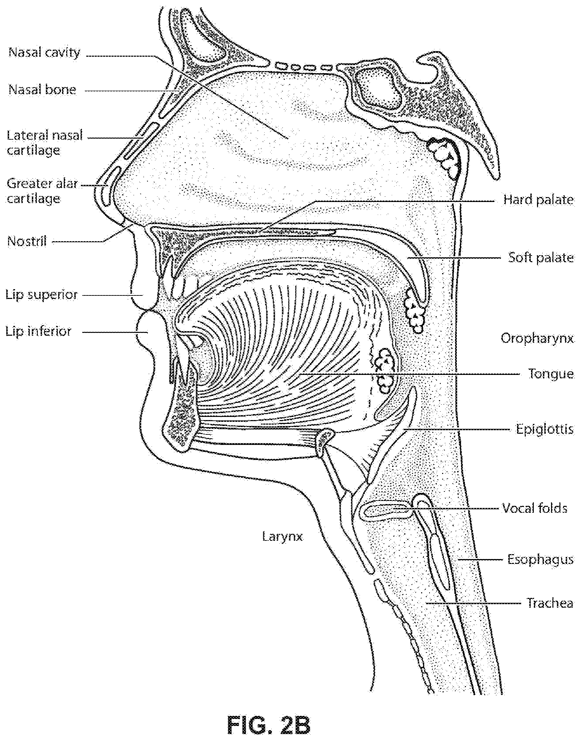

   Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

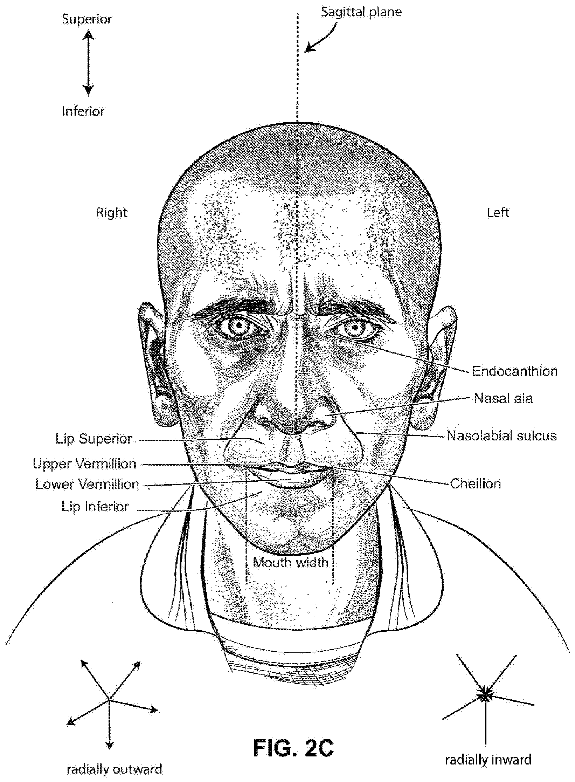

   Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

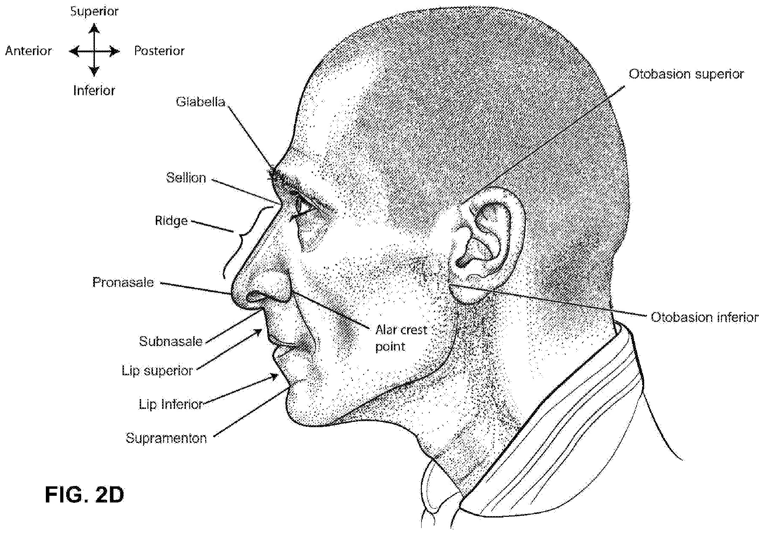

   Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

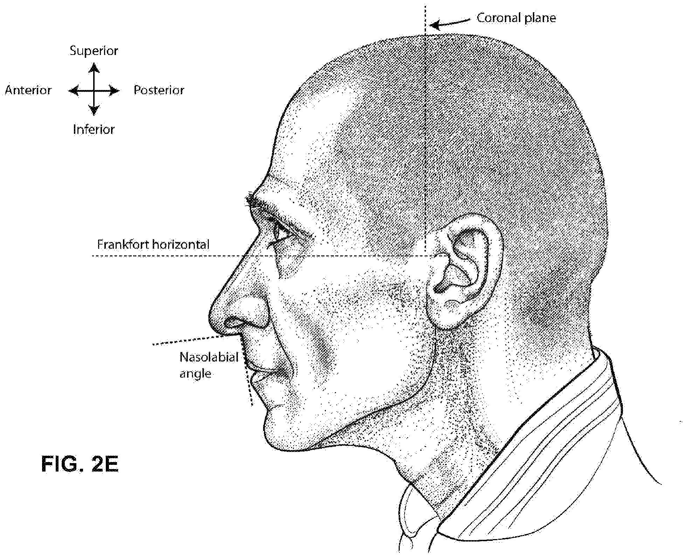

   Fig. 2E is a further side view of a head. The approximate locations of the Frankfort horizontal and nasolabial angle are indicated. The coronal plane is also indicated.

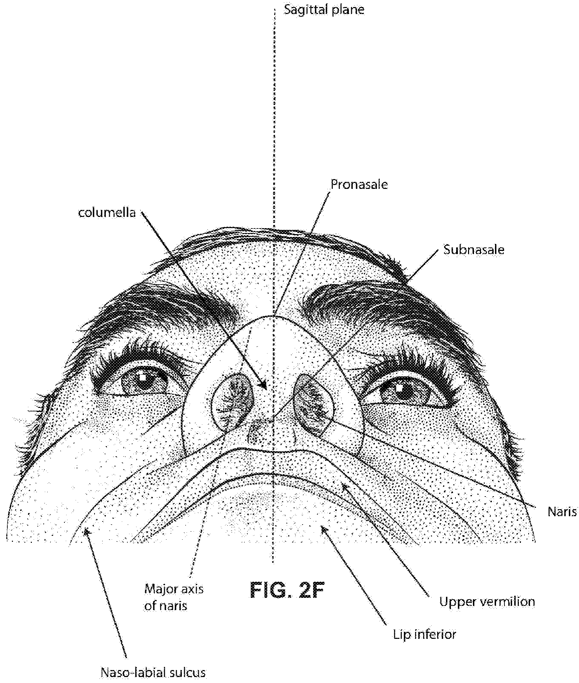

   Fig. 2F shows a base view of a nose with several features identified including naso-labial sulcus, lip inferior, upper Vermilion, naris, subnasale, columella, pronasale, the major axis of a naris and the midsagittal plane.
3.3 PATIENT INTERFACE
   Fig. 3A shows a patient interface in the form of a nasal mask in accordance with one form of the present technology.
3.4 RPT DEVICE
   Fig. 4A shows an RPT device in accordance with one form of the present technology.

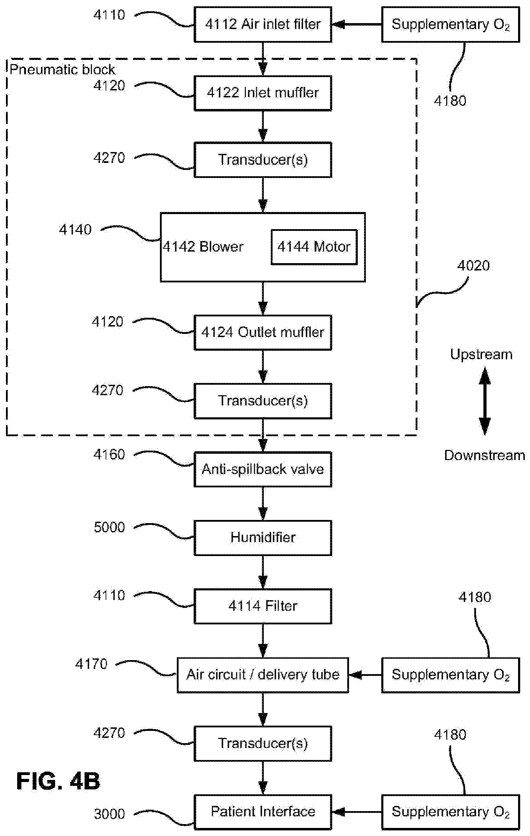

   Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.
3.5 HUMIDIFIER
   Fig. 5A shows an isometric view of a humidifier in accordance with one form of the present technology.
   Fig. 5B shows an isometric view of a humidifier in accordance with one form of the present technology, showing a humidifier reservoir 5110 removed from the humidifier reservoir dock 5130.
3.6 BREATHING WAVEFORMS
   Fig. 6A shows a model typical breath waveform of a person while sleeping.
3.7 CONNECTION MEMBER
   Fig. 7A shows a perspective view of a connection member connected at one end to an air circuit (partially shown) in accordance with one form of the present technology.
   Fig. 7B shows a top view of the connection member of Fig. 7A.
   Fig. 7C shows a bottom view of the connection member of Fig. 7A.
   Fig. 7D shows a front view of the connection member of Fig. 7A.
   Fig. 7E shows a back view of the connection member of Fig. 7A.
   Fig. 7F shows a left-hand view of the connection member of Fig. 7A.
   Fig. 7G shows a right-hand view of the connection member of Fig. 7A.
   Fig. 7H shows an exploded view of the connection member of Fig. 7A.
   Fig. 7I shows a cross-sectional view of the connection member along the plane A-A shown in Fig. 7B.
   Fig. 7J shows a perspective view of the connection member of Fig. 7A connected at one end to an air circuit (partially shown) and the opposite end to a plenum chamber of a patient interface.
   Fig. 8 shows a perspective view of a connection member according to another form of the present technology.
   Fig. 9 shows an exploded perspective view of the connection member of Fig. 8.
   Fig. 10 shows a perspective view of part of the connection member of Fig. 8.
   Fig. 11 shows a top view of the connection member of Fig. 8.
   Fig. 12 shows a top view of part of the connection member of Fig. 8.
   Fig. 13 shows an enlarged view of region A of the connection member of Fig. 12.
   Fig. 14 shows a left side view of the connection member of Fig. 8.
   Fig. 15 shows a right side view of the connection member of Fig. 8.
   Fig. 16 shows a cross-sectional view along the plane X1-X1 of Fig. 11.
   Fig. 17 shows an enlarged view of region B of the connection member of Fig. 16.
   Fig. 18 shows a perspective view of a connection member according to another form of the present technology.
   Fig. 19 shows an exploded perspective view of the connection member of Fig. 18.
   Fig. 20 shows a perspective view of part of the connection member of Fig. 18.
   Fig. 21 shows a top view of the connection member of Fig. 18.
   Fig. 22 shows a top view of part of the connection member of Fig. 18.
   Fig. 23 shows an enlarged view of region C of the connection member of Fig. 22.
   Fig. 24 shows a left side view of the connection member of Fig. 18.
   Fig. 25 shows a right side view of the connection member of Fig. 18.
   Fig. 26 shows a cross-sectional view along the plane X2-X2 of Fig. 21.
   Fig. 27 shows an enlarged view of region D of the connection member of Fig. 28.
   Fig. 28 shows a perspective view of a connection member according to another form of the present technology.
   Fig. 29 shows an exploded perspective view of the connection member of Fig. 28.
   Fig. 30 shows a perspective view of part of the connection member of Fig. 28.
   Fig. 31 shows a top view of the connection member of Fig. 28.
   Fig. 32 shows a top view of the connection member of Fig. 28 without the outer tube portion.
   Fig. 33 shows an enlarged view of region E of the connection member of Fig. 32.
   Fig. 34 shows a left side view of the connection member of Fig. 28.
   Fig. 35 shows a right side view of the connection member of Fig. 28.
   Fig. 36 shows a cross-sectional view along the line X3-X3 of Fig. 31.
   Fig. 37 shows an enlarged view of region F of the connection member of Fig. 36.
   Fig. 38 shows a perspective view of a connection member according to another form of the present technology.
   Fig. 39 shows a front view of the connection member of Fig. 38.
   Fig. 40 shows a front view of a sealing member according to a form of the present technology.
   Fig. 41 shows a perspective view of the sealing member of Fig. 40.
   Fig. 42 shows a perspective view of an end portion of a connection member according to another form of the present technology with the sealing member of Fig. 40.
   Fig. 43 shows a front view of the connection member of Fig. 42.
   Fig. 44 shows an enlarged cross-sectional view of an alternative form of a connection member.

### 4 DETAILED DESCRIPTION OF EXAMPLES OF THE

### TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 4.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 4.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 3800.

### 4.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

An unsealed patient interface 3800, in the form of a nasal cannula, includes nasal prongs 3810a, 3810b which can deliver air to respective nares of the patient 1000 via respective orifices in their tips. Such nasal prongs do not generally form a seal with the inner or outer skin surface of the nares. The air to the nasal prongs may be delivered by one or more air supply lumens 3820a, 3820b that are coupled with the nasal cannula 3800. The lumens 3820a, 3820b lead from the nasal cannula 3800 to a respiratory therapy device via an air circuit. The unsealed patient interface 3800 is particularly suitable for delivery of flow therapies, in which the RPT device generates the flow of air at controlled flow rates rather than controlled pressures. The "vent" at the unsealed patient interface 3800, through which excess airflow escapes to ambient, is the passage between the end of the prongs 3810a and 3810b of the cannula 3800 via the patient's nares to atmosphere.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH₂O with respect to ambient.

The patient interface 3000 in accordance with one form of the present technology is constructed and arranged to be able to provide a supply of air at a positive pressure of at least 10 cmH₂O with respect to ambient.

The patient interface 3000 in accordance with one form of the present technology is constructed and arranged to be able to provide a supply of air at a positive pressure of at least 20 cmH₂O with respect to ambient.

### 4.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example, the system may comprise one form of a seal-forming structure 3100 suitable for a large sized head, but not a small sized head and another suitable for a small sized head, but not a large sized head.

### 4.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200 and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 4.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 4.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 4.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 4.3.1.5 Forehead region

In one form, the seal-forming structure that forms a seal in use on a forehead region of the patient's face. In such a form, the plenum chamber may cover the eyes in use.

### 4.3.1.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 4.3.1.7 Oro-Nasal Mask

In one form of the technology the patient interface 3000 may be an oro-nasal mask. The patient interface 3000 of an oro-nasal mask comprises a seal-forming structure 3100 having an oral portion 3260 configured to form a seal around the patient's mouth and a nasal portion configured to form a seal around the patient's nasal openings. The oral portion 3260 and the nasal portion may be attached, for example they may be integrally formed.

### 4.3.1.7.1 Ultra-compact full face mask

In one form of the technology the patient interface 3000 may be a type of oro-nasal mask that may be referred to as an "ultra-compact full face mask" (UCFFM). A UCFFM mask is disclosed in Australian Provisional Patent Application No. 2019903948, the contents of which are hereby incorporated by reference.

In certain forms of the present technology, a seal-forming structure 3100 of an UCFFM comprises a nasal portion configured to form a seal to inferior surfaces of the patient's nose. The nasal portion may seal to an inferior periphery of the patient's nose (e.g., surrounding the patient's nares and to the patient's lip superior). In examples, the nasal portion of the seal-forming structure 3100 may be configured to contact the patient's face below the bridge of the nose or below the pronasale.

The nasal portion of the seal-forming structure 3100 may also comprise a medial portion configured to form a seal with an inferior surface of the patient's nose between the patient's nares, for example the columella. The medial portion may be configured so that it is substantially superior-facing in use.

In some examples a majority of the seal formed by the seal-forming surface 3100 to the inferior periphery of the patient's nose may be made by the superior-facing medial portion and lip superior portion. The superior-facing medial portion superior and anterior to the nasal holes of the seal-forming structure may seal against the inferior and partially anterior surfaces of the patient's pronasale. A lip superior portion of the seal-forming structure 3100 may seal against the lip superior. The lip superior portion is provided medially and inferior and posterior to the nasal holes.

In one form of an UCFFM the patient interface 3000 comprises a seal-forming structure 3100 that forms a seal in use around the patient's mouth at an oral portion 3260. The oral portion 3260 comprises a lip inferior portion which forms a seal against the chin region of the patient in use. The lip inferior portion of the seal-forming structure may seal against the lip inferior and supramenton of the patient. Additionally, the oral portion 3260 may comprise an oral hole peripheral portion which contacts the patient's cheeks. The lip inferior portion may be connected to (e.g., contiguous with) the lip superior portion via the oral hole peripheral portion.

### 4.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface and help improve compliance with therapy.

In certain forms of the present technology, the plenum chamber 3200 is constructed at least in part from a flexible material, e.g. silicone. In certain forms, the entire plenum chamber 3200 is formed from a flexible material. In certain forms a part of the plenum chamber 3200 comprising a connection port 3600 (allowing for connection to the air circuit 4170) is constructed from a flexible material. For example, an anterior portion of the plenum chamber 3200 may be constructed from a flexible material.

### 4.3.3 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example, the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example, the system may comprise one form of positioning and stabilizing structure 3300 suitable for a large sized head, but not a small sized head, and another. suitable for a small sized head, but not a large sized head.

### 4.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO₂ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

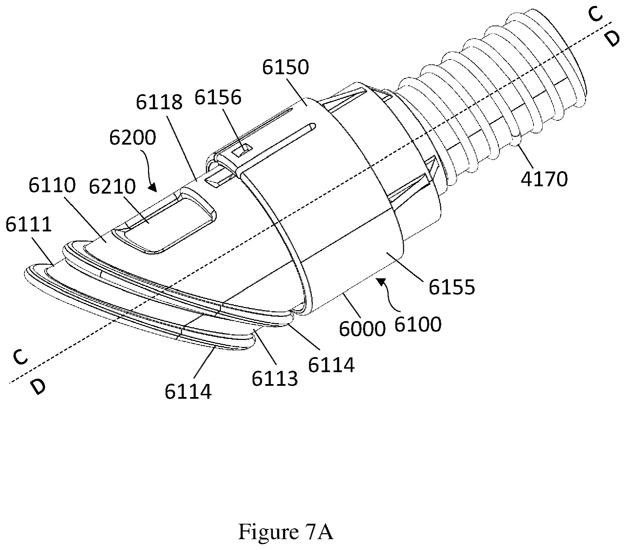

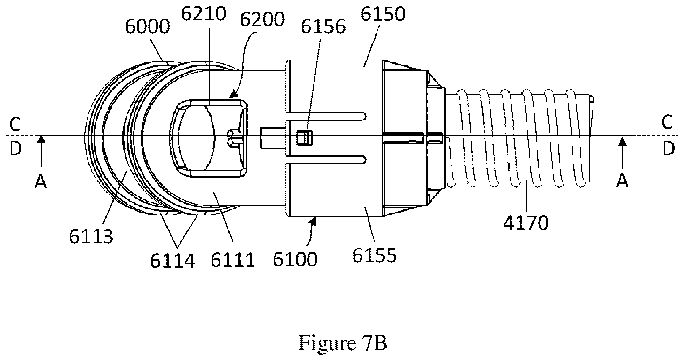

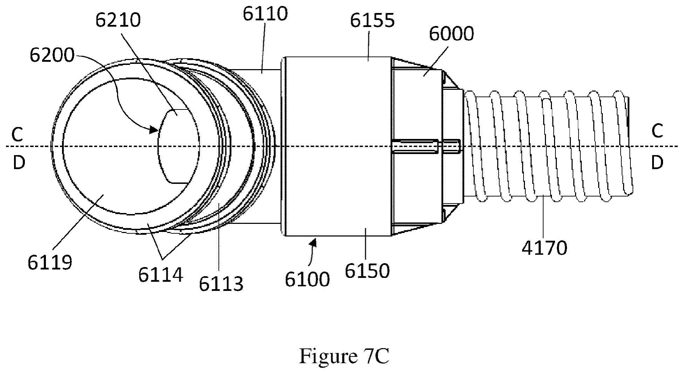

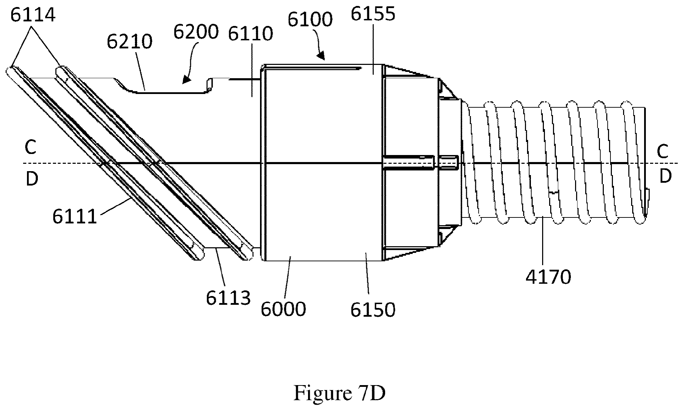

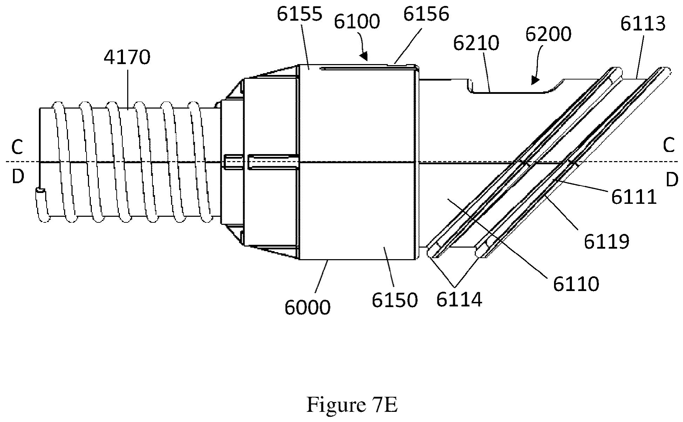

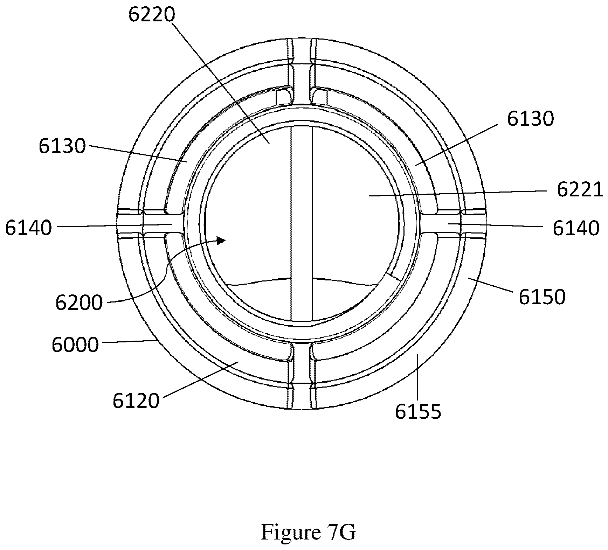

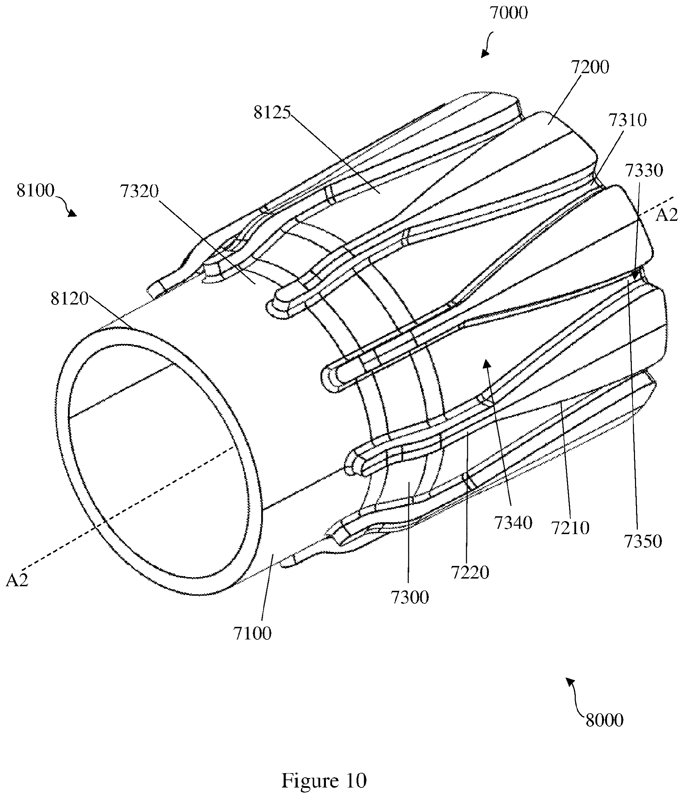

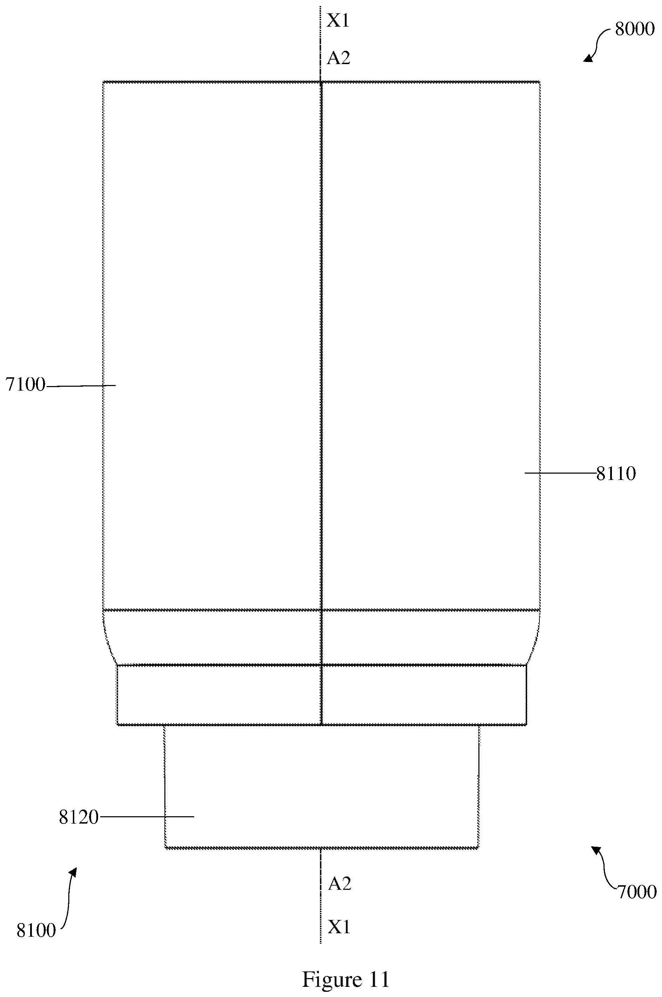

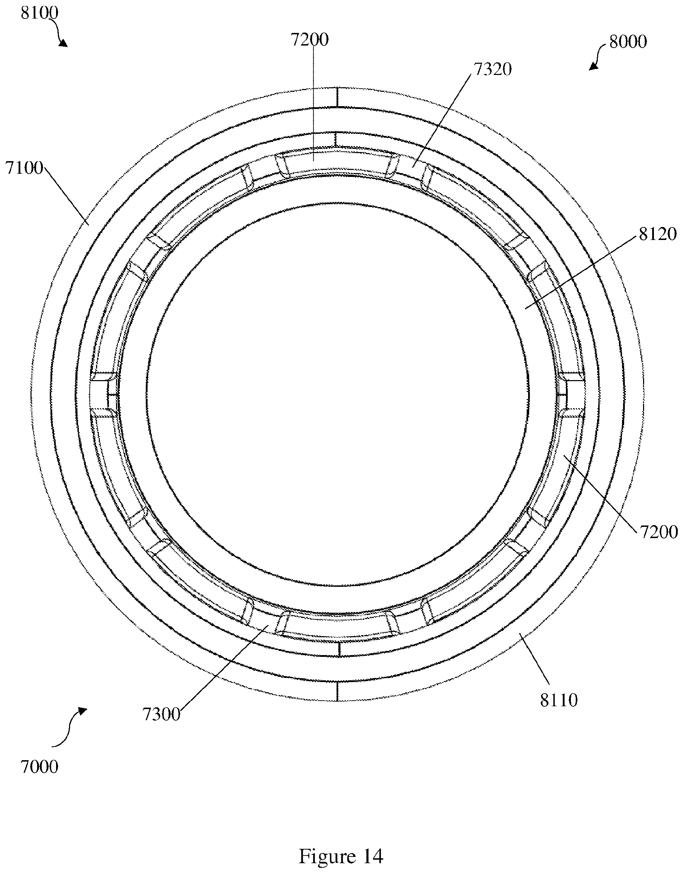

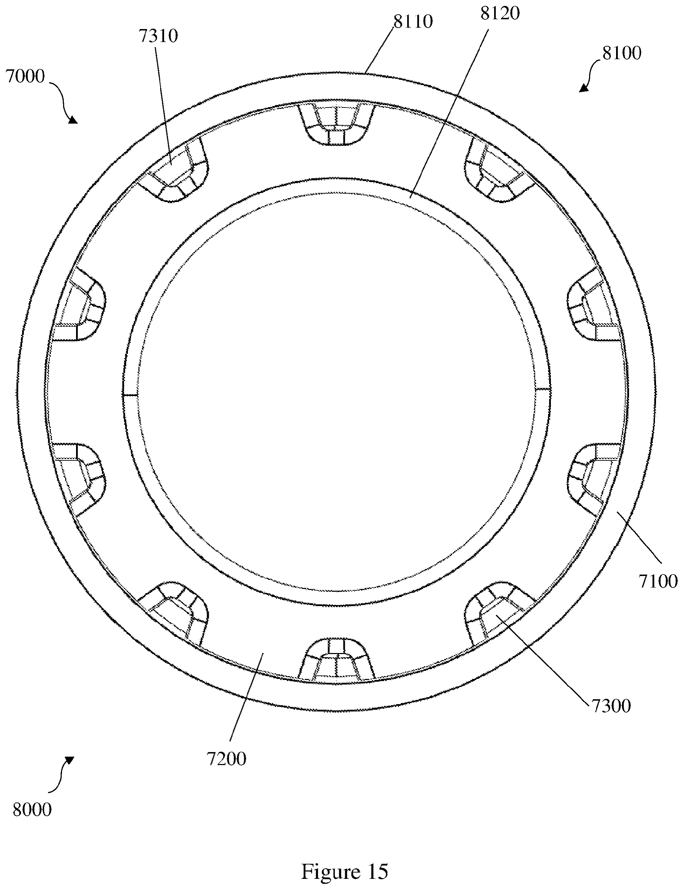

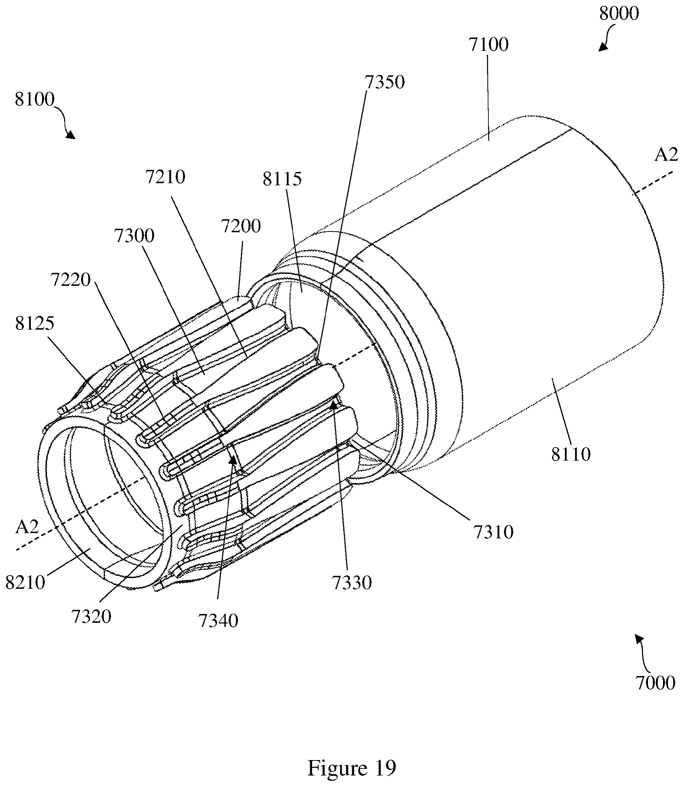

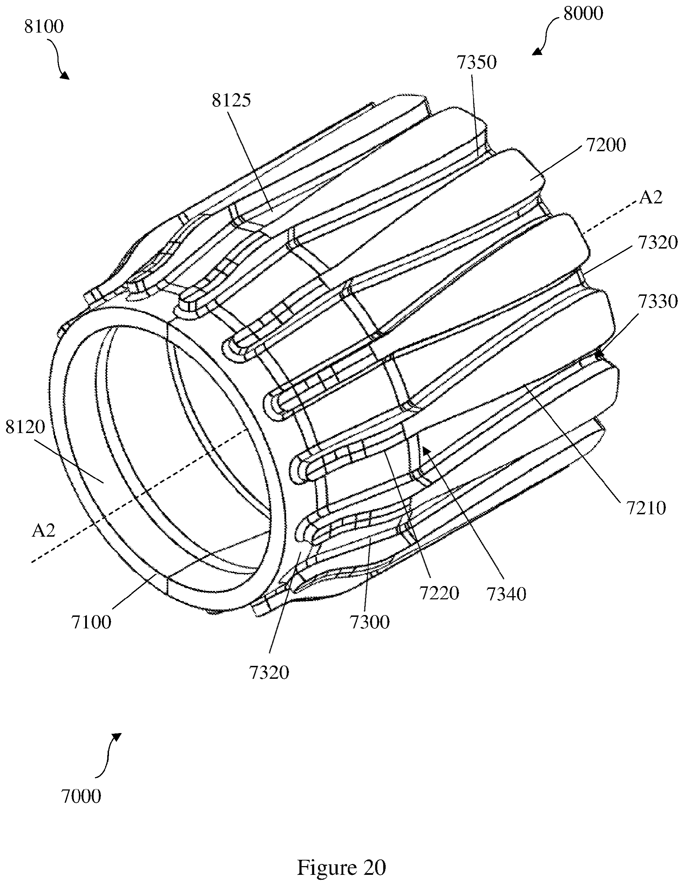

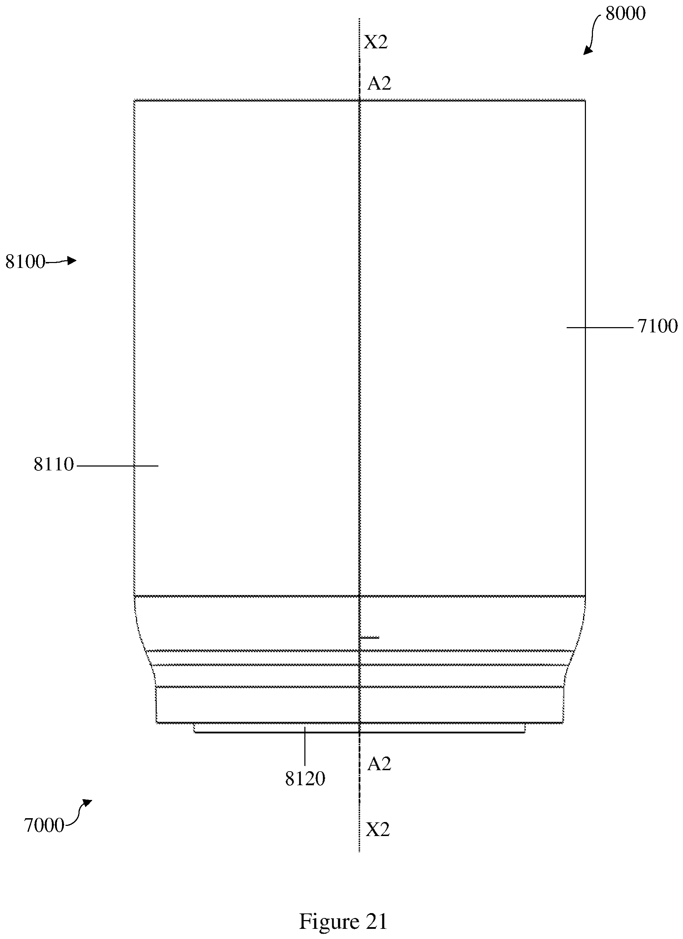

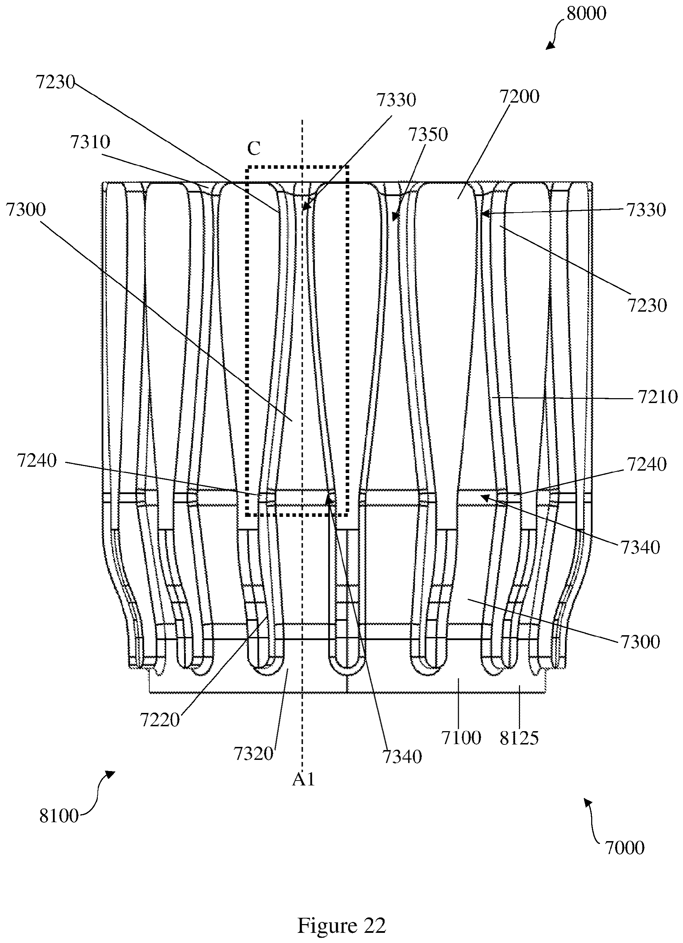

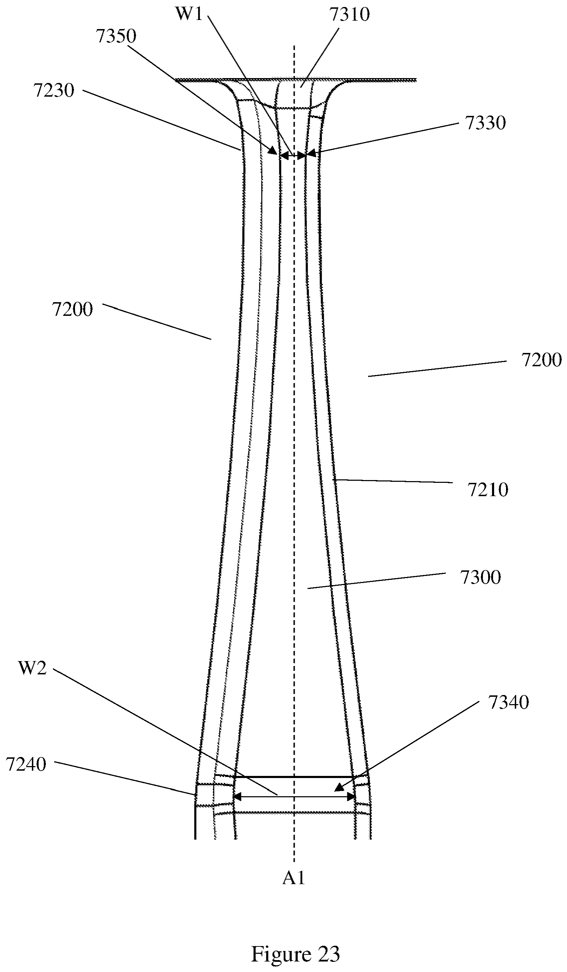

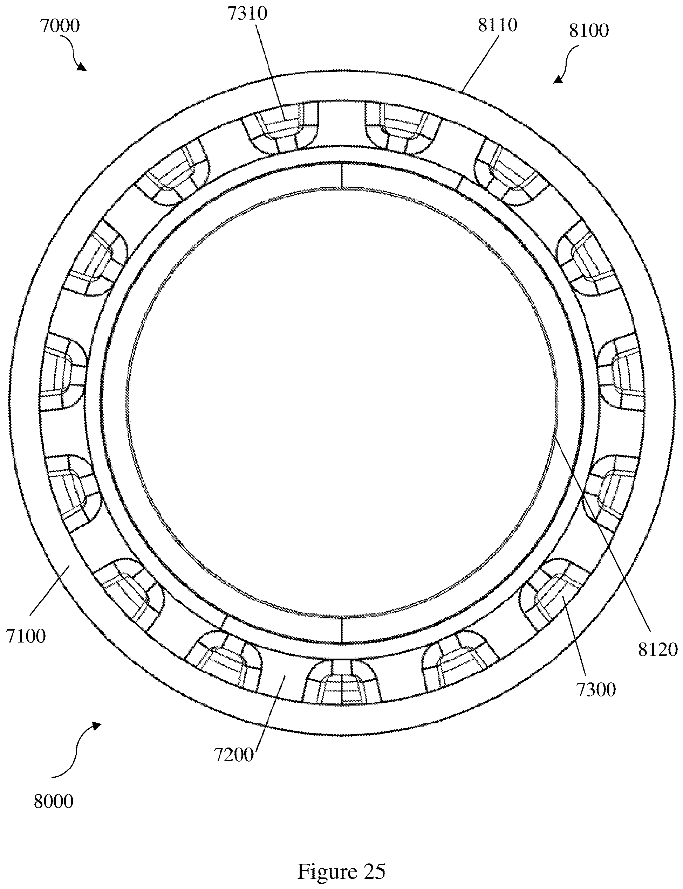

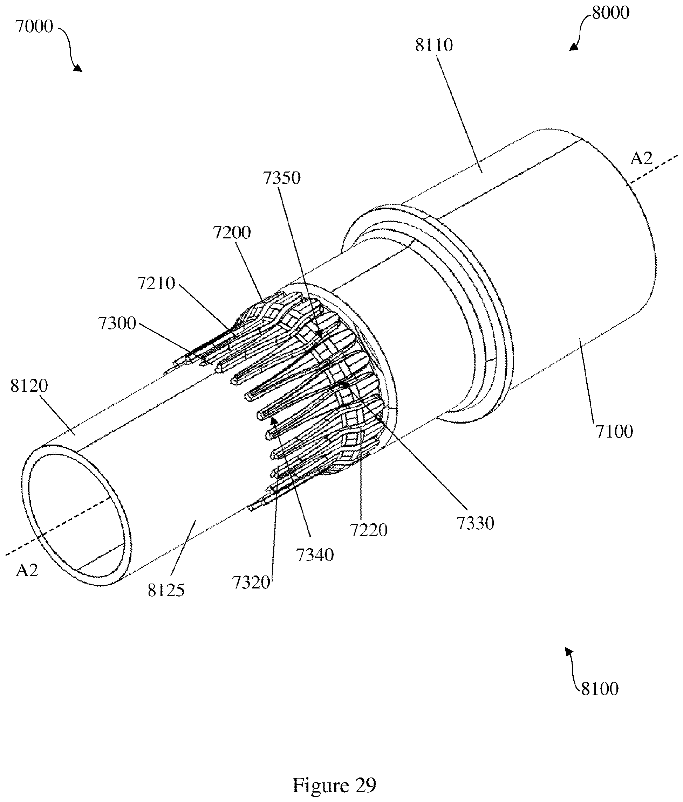

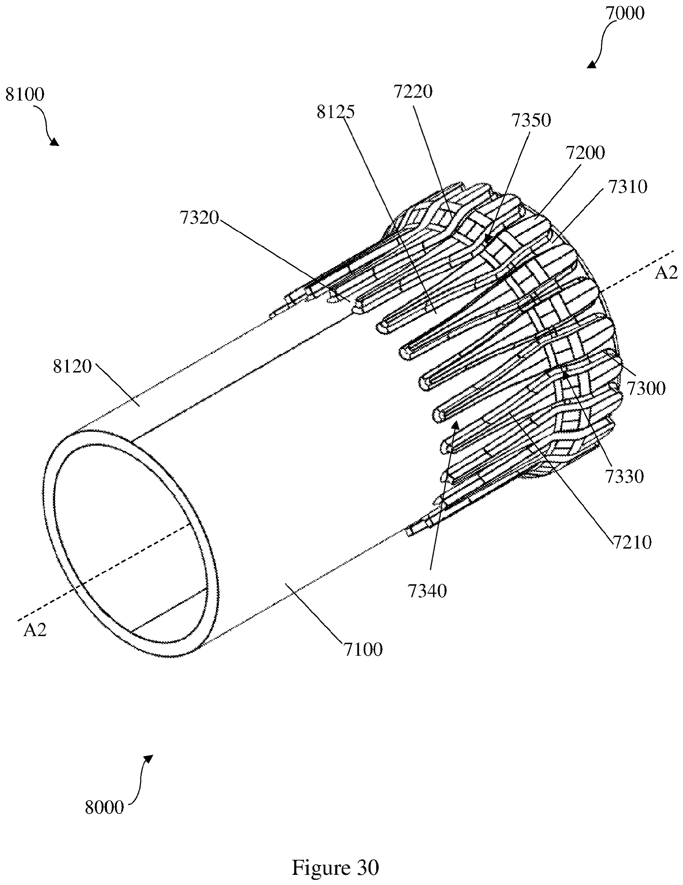

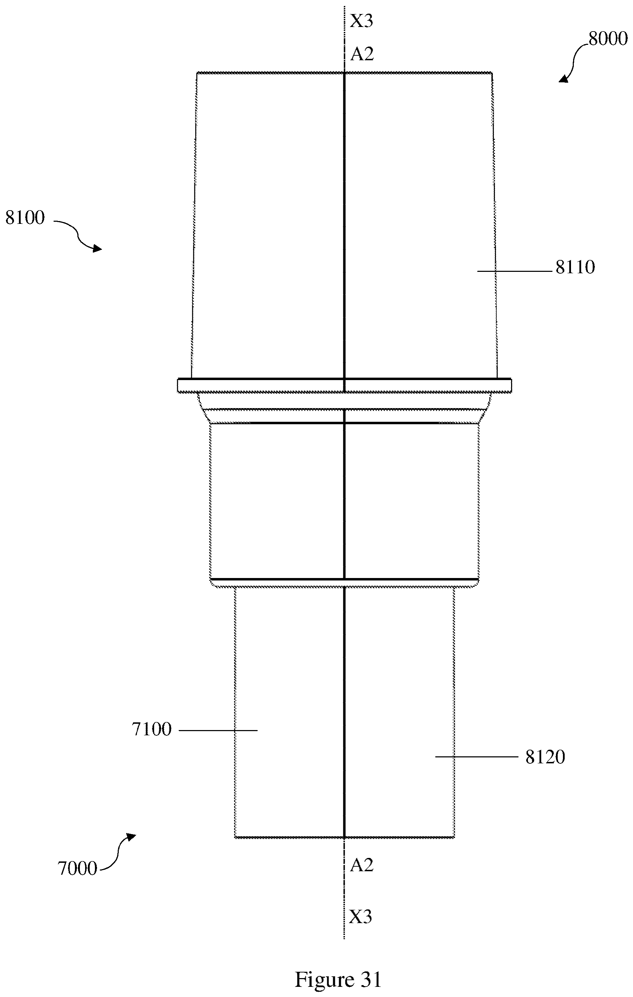

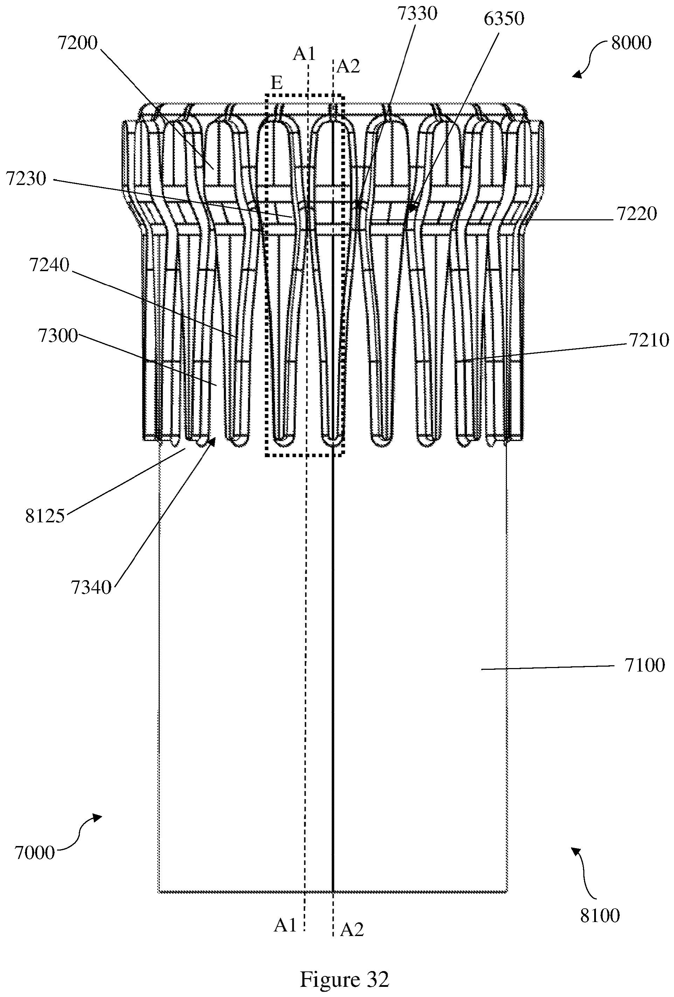

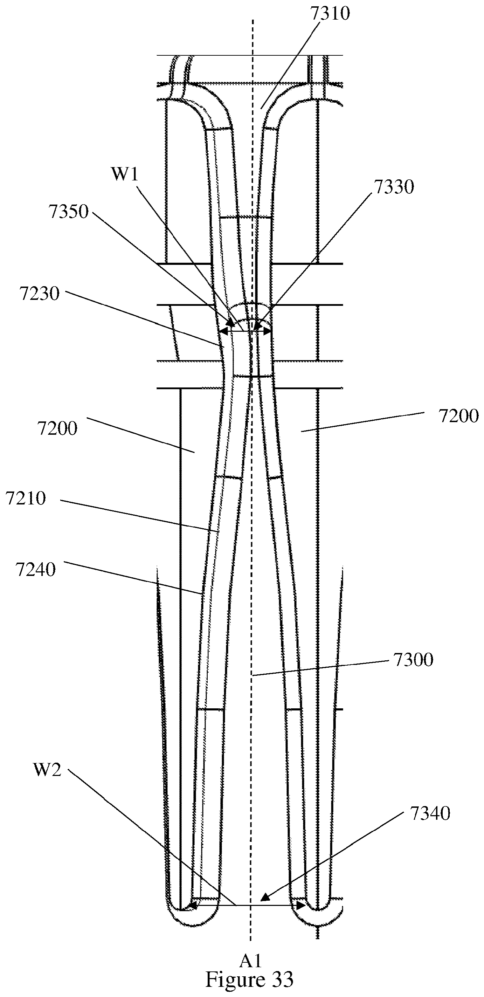

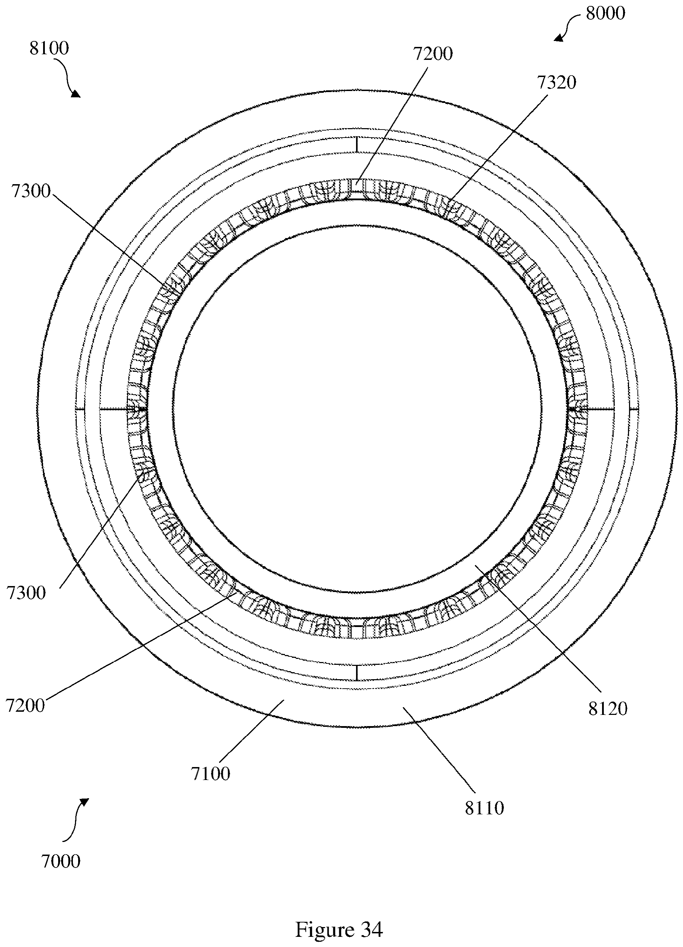

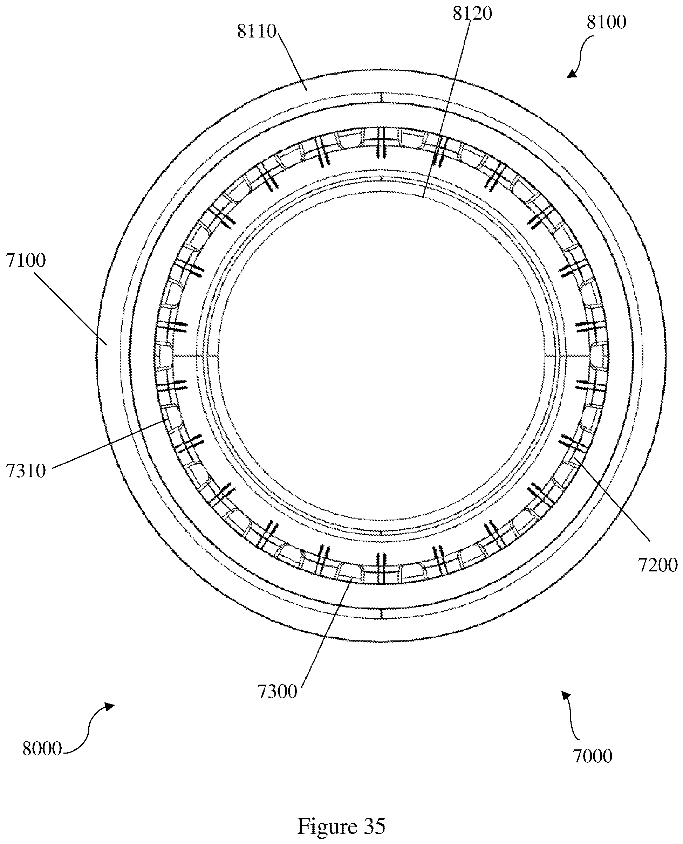

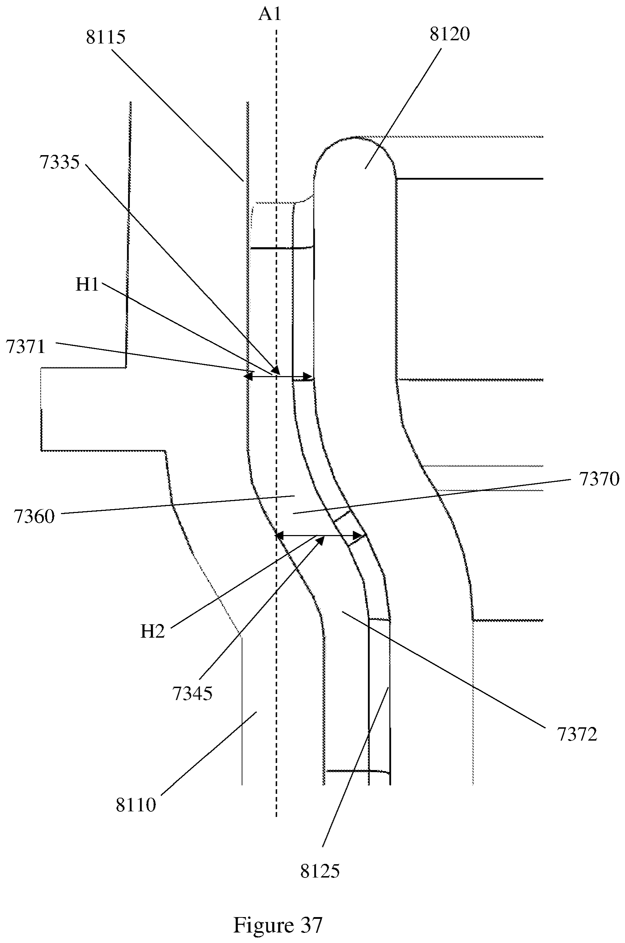

In one form the vent 3400 is located in a connection member 6000 as described in section 4.6 and shown in Figures 7A-44.

### 4.3.5 Connection port

Connection port 3600 allows for connection to the air circuit 4170.

### 4.3.6 Forehead support

In one form, the patient interface 3000 includes a forehead support 3700.

### 4.3.7 Anti-asphyxia valve

In certain forms of the technology, the patient interface assembly comprises an anti-asphyxia valve (AAV) 6200.

As explained in section 4.10.4, an anti-asphyxia valve (AAV) 6200 provides a mechanism to enable the patient 1000 to breathe in situations where the blower 4142 stops providing a flow of pressurised air. In certain forms, the AAV 6200 comprises an opening which forms an airflow path between the patient 1000 and the ambient air to provide the patient 1000 with a supply of fresh air. This reduces the risk of excessive CO₂ rebreathing by a patient.

In one form, the patient interface 3000 includes an anti-asphyxia valve. Alternatively, in one form the AAV 6200 is located in a connection member 6000 as described in section 4.6 herein.

### 4.3.8 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 4.3.9 Patient interface assembly

In some forms of the technology, a patient interface 3000 together with a connection member 6000 form a patient interface assembly.

### 4.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH₂O, or at least 10cmH₂O, or at least 20 cmH₂O.

### 4.4.1 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

In other forms of the present technology, some portion or all of the algorithms 4300 may be implemented by a controller of an external device such as the local external device 4288 or the remote external device 4286. In such forms, data representing the input signals and / or intermediate algorithm outputs necessary for the portion of the algorithms 4300 to be executed at the external device may be communicated to the external device via the local external communication network 4284 or the remote external communication network 4282. In such forms, the portion of the algorithms 4300 to be executed at the external device may be expressed as computer programs stored in a non-transitory computer readable storage medium accessible to the controller of the external device. Such programs configure the controller of the external device to execute the portion of the algorithms 4300.

In such forms, the therapy parameters generated by the external device via the therapy engine module 4320 (if such forms part of the portion of the algorithms 4300 executed by the external device) may be communicated to the central controller 4230 to be passed to the therapy control module 4330.

### 4.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

In particular, the air circuit 4170 may be in fluid connection with the outlet of the pneumatic block 4020 and/or the patient interface. The air circuit may be referred to as an air delivery tube. In some cases, there may be separate limbs of the circuit for inhalation and exhalation. In other cases, a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349, which is incorporated here within in its entirety by reference.

### 4.5.1 Air circuit assembly

In some forms of the technology, an air circuit 4170 together with a connection member 6000, as described in section 4.6, may form an air circuit assembly that is configured to fluidly connect RPT device 4000 to patient interface 3000.

### 4.6 CONNECTION MEMBER

In some forms of the technology the patient interface assembly comprises a connection member 6000 configured to connect an air circuit 4170 to a patient interface 3000 to convey a flow of pressurised breathable gas from the air circuit 4170 to the patient interface 3000 for breathing by a patient 1000.

To create a compact design for the patient interface assembly the connection member 6000 comprises both a vent structure 6100 and an anti-asphyxia valve (AAV) 6200. A further advantage of the connection member 6000 comprising a vent structure 6100, rather than providing the vent as part of the plenum chamber 3200, is that the patient interface 3000 may have a lower manufacturing cost due to the reduced complexity of this component. This may be especially beneficial as the patient interface 3000, or parts thereof (for example, a cushion formed by the plenum chamber 3200 and seal-forming structure 3100), is typically a frequently replaced product.

In some forms of the technology the connection member 6000 is formed from a substantially rigid material, for instance a polycarbonate.

### 4.6.1 Components of the connection member

### 4.6.1.1 Vent structure

In one form of the technology, the connection member 6000 comprises a vent structure 6100 to vent a flow of air exhaled from the patient 1000 to the surrounding ambient air. An example of a connection member with a vent structure 6100 is shown in Figures 7A-7I and is described in the following paragraphs.

The connection member 6000 comprises a first tube portion 6110 and a second tube portion 6120. The first tube portion 6110 has a patient-proximal end 6111 and a patient-distal end 6112. In use, the patient-proximal end 6111 is located closer to the patient than the patient-distal end 6112. The second tube portion 6120 has a patient-proximal end 6121 and a patient-distal end 6122. In use, the patient-proximal end 6121 is located closer to the patient than the patient-distal end 6122.

In use, the flow of breathable gas is conveyed from the air circuit 4170 to the patient interface 3000 through the first tube portion 6110 and the second tube portion 6120.

At least a portion of the patient-proximal end 6121 of the second tube portion 6120 is positioned inside at least a portion of the patient-distal end 6112 of the first tube portion 6110.

A vent structure 6100 for venting gas exhaled by the patient 1000 to the ambient air is formed between the first tube portion 6110 and the second tube portion 6120. The vent structure 6100 may be located around the outer periphery of the patient-proximal end 6121 of the second tube portion 6120 in the region where at least a portion of the patient-proximal end 6121 of the second tube portion 6120 is positioned inside at least a portion of the first tube portion 6110.

The vent structure 6100 may comprise a plurality of vent slots 6130 arranged in a region between the first tube portion 6110 and the second tube portion 6120. Some of the air that is exhaled by the patient 1000 flows into the connection member 6000 from the plenum chamber 3200, through the vent slots 6130 and into the surrounding ambient air. The vent slots 6130 may be arranged around the second tube portion 6120. The vent slots may be formed between a plurality of ribs 6140 extending between an outer surface 6125 of the second tube portion 6120 and an inner surface 6115 of the first tube portion 6110. In one form the ribs 6140 are provided on the outer surface 6125 of the second tube portion 6120, for example integrally formed (e.g. moulded) to the outer surface 6125. In another form the ribs 6140 are provided on the inner surface 6115 of the first tube portion 6110, for example integrally formed (e.g. moulded) to the inner surface 6115.

In the form of the technology shown in Figures 7A-7I the vent structure 6100 comprises four ribs 6140 forming four vent slots 6130. In other forms of the technology there may be a different number of ribs 6140 forming vent slots 6130. In the form of the technology shown in Figures 7A-7I the ribs 6140 are equally spaced apart around the outer periphery of the first tube portion 6110 and the vent slots 6130 are of equal size. The length of the vent slots in the form shown in Figures 7A-7I is between 15mm and 30mm, for example 20mm. In other forms of the technology the vent slots have a different length. The length of the vent slots is a trade-off between being as short as possible to provide a compact connection member 6000 whilst also being long enough to provide the necessary flow resistance to reduce the flow velocity.

In the form of the technology shown in Figures 7A-7I the first tube portion 6110 and the second tube portion 6120 have a circular cross-section and the first tube portion 6110 and the second tube portion 6120 are substantially cylindrical in shape. In other forms of the technology the cross-sections of the first tube portion 6110 and the second tube portion 6120 may be another shape for instance oval, rectangular (including square), or rectangular with rounded corners. In the form of the technology shown in Figures 7A-7I the second tube portion 6120 has a smaller diameter than the first tube portion 6110 and the vent slots 6130 are arranged in an annular region between the first tube portion 6110 and the second tube portion 6120.

The connection member 6000 may comprise a tube connector 6150 configured to connect the second tube portion 6120 to the first tube portion 6110. The tube connector 6150 may connect the second tube portion 6120 to the outer surface 6118 of the first tube portion 6110, although in other forms the tube connector 6150 may connect the second tube portion 6120 to another part of the first tube portion 6110. In the embodiment shown in Figures 7A-7I the tube connector 6150 comprises a third tube portion 6155 mounted to an outer surface 6125 of the second tube portion 6120. The third tube portion 6155 may be substantially cylindrical in shape. The third tube portion 6155 surrounds the patient-distal end 6112 of the first tube portion 6110. The first tube portion 6110 comprises a connecting portion 6116 to which the third tube portion 6155 is configured to connect. For example, the connecting portion 6116 may comprise a projection configured to fit into a corresponding second aperture 6156 in the third tube portion 6155. The connecting portion 6116 may fit into the second aperture 6156 via a snap-fit connection. In other forms of the technology the third tube portion 6155 may comprise a connecting portion 6116 configured to fit into a corresponding second aperture 6156 in the first tube portion 6110. In other forms of the technology the third tube portion 6155 may connect to the first tube portion 6110 via an alternative means, for instance an adhesive, or friction fit connection.

In alternative forms of the technology the first tube portion 6110 and second tube portion 6120 may be integrally formed, for example co-moulded.

The vent structure 6100 according to forms of the technology described above may provide a number of benefits. For example, the vent structure 6100 may be low cost to manufacture compared to some prior art vent structures. The vent structure 6000 may provide a sufficient level of quietness so as to not disturb the patient 1000 or bed partner 1100. The vent structure 6100 may be configured so that the vent slots 6130 are orientated so that vented gases flow adjacent to the outside of the second tube portion 6120 after exiting the vent structure 6100. This may mean the vented gases are unlikely to be directed towards the patient 1000 or bed partner 1100.

In some forms the Coanda effect may act to assist in ensuring that the air flow of vented gases attaches to the air circuit 4170 downstream of the vent structure 6100, even if the air circuit 4170 is bent. If there is a bend in the air circuit 4170 the air flowing along the outer curve of the bend will continue to follow the air circuit 4170 under the Coanda effect rather than continuing along a straight path in a direction away from the air circuit 4170. This effect may provide a number of advantages. It may help to avoid air flowing towards the patient 1000 or bed partner 1100 as the flow of air exiting the vent structure 6100 tends to stay attached to the outer surface of the air circuit 4170. The attachment of the flow to the outer surface of the air circuit 4170 where it is subject to friction from the air circuit 4170 may also help to further slow the flow of air, reducing noise.

### 4.6.1.1.1 Vent with noise reducing features

In alternative forms of the technology, the vent structure 6100 may comprise one or more features to reduce the noise caused by the flow of air through the vent and/or reduce the force of the air flow exiting the vent which may disturb, or be uncomfortable for, the patient 1000 and/or bed partner 1100. Examples of such vent features are disclosed in PCT Patent Application No. PCT/AU2020/051211, published as PCT Publication No. WO2021/087570 on 14 May 2021, which is incorporated herein in its entirety by reference. The relevant sections of PCT Publication No. WO2021/087570 have been reproduced below.

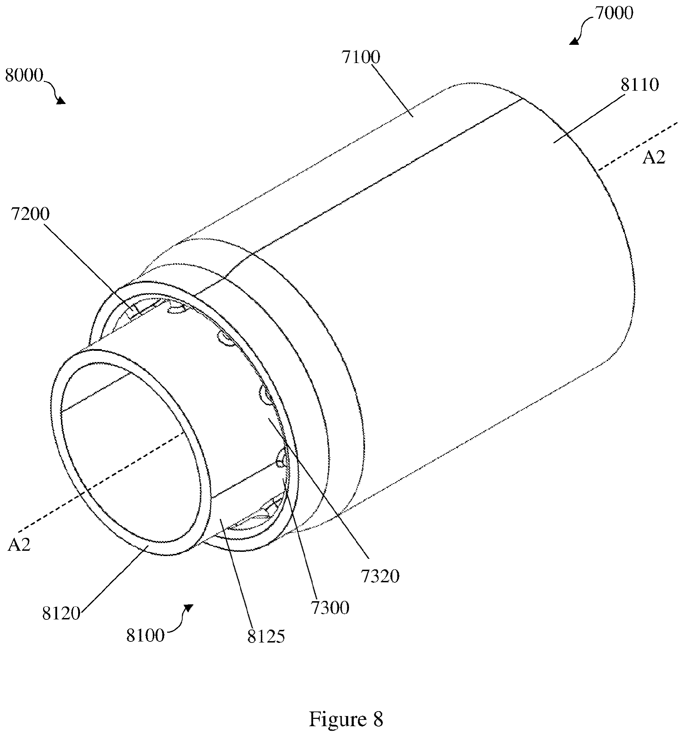

It should be appreciated that the concepts and/or features of the vents described in the proceeding sections and illustrated by way of example in Figs. 8 to 44 may be applied to the vent structure 6100 previously described, for example as shown in Figs. 7A to 7J, in certain forms of the technology. For example, in some forms of the technology, the ribs 6140 that form the vent slots 6130 of the vent structure 6100 may be formed in the manner of, or similar to, the partitions 7200 of vent structure 7000 described below such that each vent slot 6130 has a cross-sectional area at a first region of the vent slots that is smaller than a cross-sectional area at a second region of the vent slots. In some forms of the technology, the outer surface 6125 of the second tube portion 6120 and the inner surface 6115 of the first tube portion 6110 between which the vent slots 6130 are formed may be formed such that that each vent slot 6130 has a cross-sectional area at a first region of the vent slots that is smaller than a cross-sectional area at a second region of the vent slots. Various features and forms of the vent structure 7000 relevant to the change in cross-sectional area along the length of the vent slots 6130 are described in further detail below, including change in width, change in height, and acoustic attenuation chambers. Any one or more of the features described below, including in relation to the forms of the technology shown in Figs. 8 to 44, may be incorporated into the vent structure 6100 of Figs. 7A to 7J, including with regard the shape and structure of the ribs 6140, first tube portion 6110 and second tube portion 6120.

In other forms of the technology, the inner surface 6115 of the first tube portion 6110 of the form of the technology shown in Figs. 7A to 7J may form a deflector 7400 as described below to deflect the flow of air through each vent slot towards the second tube portion 6120.

In other forms of the technology, the vent structure 6100 of the form of the technology shown in Figs. 7A to 7J may comprise a projecting portion 7500, as described in detail below, extending outwardly from the patient-distal end 6112 of the first tube portion 6110 and structured and arranged to inhibit generation of shear layer instabilities in air flowing out of the vent structure 6100.

### 4.6.1.1.1.1 Vent overview

As noted above a vent 3400 may be provided to a part of the respiratory system to allow for the washout of exhaled gases, e.g. carbon dioxide. Venting of the exhaled gas may result in noise caused by the flow of air through the vent 3400 which can disturb the patient 1000 and/or the bed partner 1100. Furthermore, the force of the air flow exiting the vent may disrupt, or be uncomfortable for, the patient 1000 and/or bed partner 1100. Air exiting the vent directly into the surrounding ambient air may also result in flow separation, particularly at high velocities. Flow separation causes an increase in turbulence in the flow of air and therefore noise. The faster the flow of air exiting the vent, the louder the noise associated with the vent 3400. Slowing down the flow of air in the vent will therefore reduce the noise associated with the vent 3400.

In certain forms of the present technology a vent 3400 may be provided in the form of a vent structure 7000 provided to a part of the respiratory system and configured to reduce the noise produced by venting gases and/or to reduce the speed of the air flow as it exits the vent. In some forms, the vent structure 7000 may be formed from a plastics material, for example polycarbonate. In some forms the vent structure 7000 is formed from an assembly of different materials.

In certain forms, the vent structure 7000 may have the advantage of not requiring any diffuser material as is present in prior art vents to reduce jetting of the air flow. Diffuser material is an additional component to a patient interface so adds complexity in manufacture, assembly and use, and may need to be cleaned and/or replaced regularly.

### 4.6.1.1.1.2 Connection member

In certain forms of the present technology the vent structure 7000 may be formed as part of a connection member 8000. Figures 8 to 39 illustrate several connection members 8000 according to different forms of the technology. The connection member 8000 is configured to connect the air circuit 4170 and the patient interface 3000. In some forms of the technology this may be an indirect connection, for instance there may be one or more additional components that connect between the connection member 8000 and the air circuit 4170 or patient interface 3000, including a length of conduit fluidly connecting the patient interface 3000 and the connection member 8000, for example. In other forms the connection member 8000 is directly connected to the air circuit 4170 and/or the patient interface 3000. In forms of the technology, the connection member 8000 creates both a physical connection and a fluid connection between the air circuit 4170 and the patient interface 3000.

In some forms of the technology the connection member 8000 may be a separate or separable component to the circuit 4170 and/or the patient interface 3000. In other forms of the technology the air circuit 4170 or the patient interface 3000 comprises the connection member 8000.

In one form the connection member 8000 may comprise a tube portion 8100 configured to fluidly connect the air circuit 4170 to the patient interface 3000. The tube portion 8100 is typically a circular hollow cylinder or assembly of components forming a generally cylindrical shape with an air path through it to convey air from the air circuit 4170 to the patient interface 3000. In other forms of the technology the tube portion may have a different cross-sectional shape, for example oval, D-shaped or polygonal.

The connection member 8000 may further comprise a vent structure 7000 comprising a vent housing 7100 and a plurality of partitions 7200 inside the vent housing 7100. The plurality of partitions 7200 form therebetween a plurality of vent slots 6300 through which exhaled air can flow. The vent slots 7300 may be partly defined by the vent housing 7100, for example parts of the vent housing 7100 may define the upper and lower walls of each vent slot 7300 while the partitions 7200 define the side walls of each vent slot 7300. The vent slots 7300 comprise a vent inlet 7310 configured to receive an air flow, for example air exhaled by the patient 1000. The vent slots 7300 also comprise a vent outlet 7320 configured to allow the air flow to exit into the surrounding ambient air. Exhaled air passing through the vent slots flows in a direction from the vent inlet 7310 to the vent outlet 7320. The parts of the vent housing 7100 forming the vent slots 7300 and the partitions 7200 may be substantially rigid and fixed in position relative to each other such that the shape of the vent slots does not change during use.

The number of vent slots 7300 in the vent structure 7000 may vary between different forms of the technology. Design features that may be varied to alter the number of vent slots 7300 include the size of the vent structure 7000, the number of partitions 7200 and the thickness of the partitions 7200 forming the vent slots 7300.

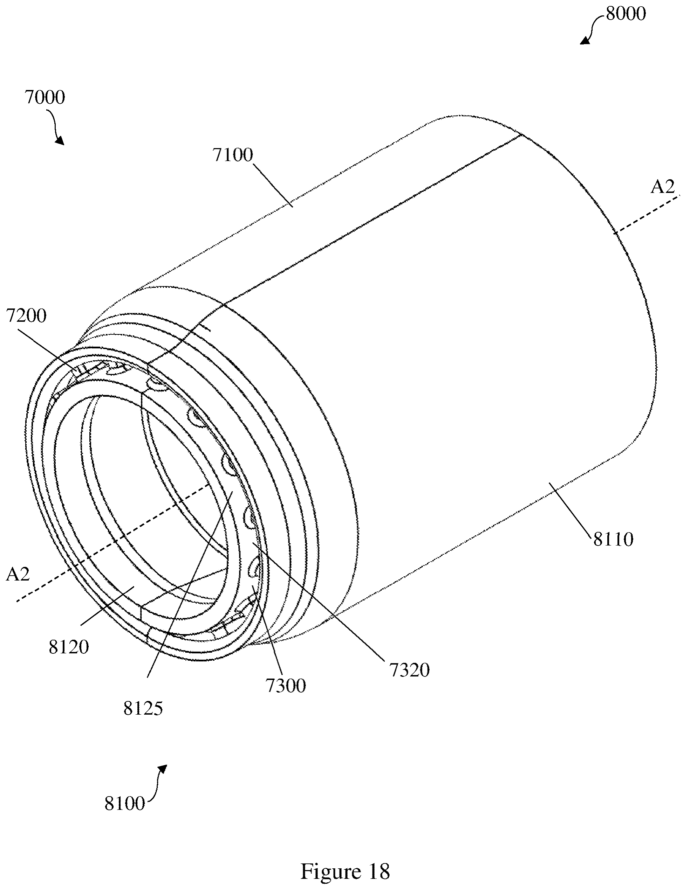

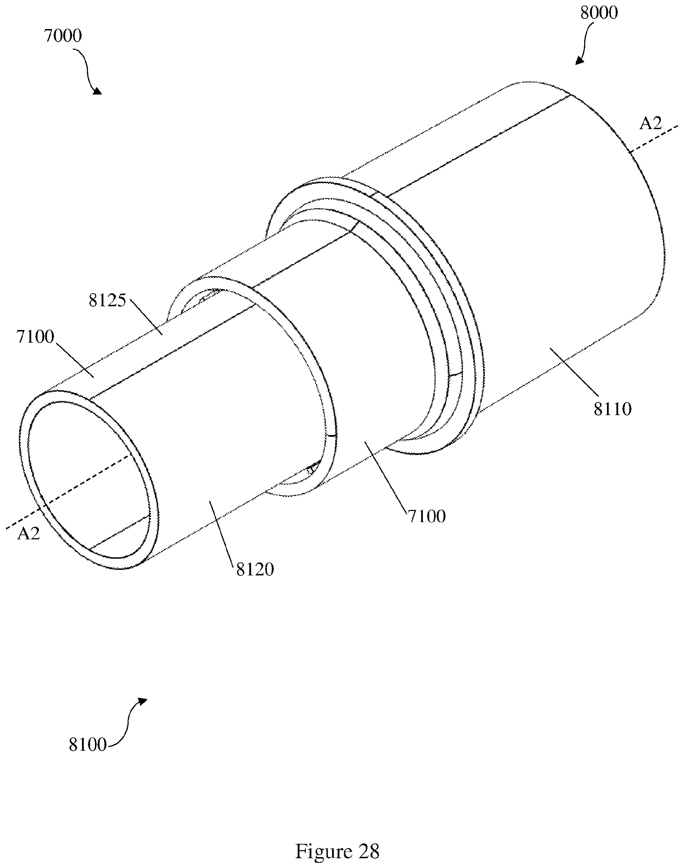

As mentioned above a vent 3400 according to forms of the technology is constructed and arranged to allow for the washout of exhaled gases, e.g. carbon dioxide from the patient interface 3000. The vent structure 7000 prevents rebreathing of the exhaled gases while maintaining the therapeutic pressure in the plenum chamber in use. The total size of the vent structure 7000 in particular the vent slots 7300 is therefore formed to be sufficient to allow for washout of the exhaled gases and also to maintain the therapeutic pressure in the plenum chamber 3200. In exemplary forms of the technology in which the vent structure 7000 is formed as part of a connection member 8000, the cross-sectional area of each vent inlet 7310 may be between 1.8mm² and 2.5mm², for example approximately 2.2mm². The total cross-sectional area of the vent outlets may be between 45mm² and 50mm². In the form of the technology shown in Figures 28 to 37 there are 22 vent inlets 7310 and the total cross-sectional area of the vent inlets is approximately 48mm². The cross-sectional area of each vent outlet 7320 may be between 2.0mm² and 2.8mm², for example approximately 2.4mm². The total cross-sectional area of the vent outlets 7320 may be between 50mm² and 55mm². In the form of the technology shown in Figures 28 to 37 there are 22 vent outlets 7320 and the total cross-sectional area of the vent outlets is approximately 52mm². Additionally, the ratio of the total cross-sectional area of the vent inlets 7310 to the total cross-sectional area of the tube portion 8100 may be in the range of 1:4-25. In the form of the technology shown in Figures 8 to 17 the ratio of the total cross-sectional area of the vent inlets 7310 to the total cross-sectional area of the tube portion 8100 is substantially 1:23. In the form shown in Figures 18 to 27 this ratio is substantially 1:18. In the form shown in Figures 28 to 37 this ratio is substantially 1:16.

As the air flows along the vent slots 7300 the air contacts the partitions 7200 and the vent housing 7100 and reduces in speed due to friction of the air flow with the partitions 7200 and walls of the vent housing 7100. Changes in the design of the vent structure 7000 may alter the amount of friction experienced by the air flow, and therefore the speed at which air exits the vent structure 7000.

The higher the total surface area of the vent slots 7300 that the air flow comes into contact with, the lower the speed of the air flow because of the greater frictional or drag forces when there is higher surface area. In general, the more vent slots 7300 there are the greater the overall surface area of the vent structure 7000 that contacts the air flow and therefore the greater the reduction in speed of the venting air. Therefore, forms of the technology having a relatively high number of vent slots 7300 may be more effective in reducing the speed of flow, and therefore reducing the amount of noise created by the air flow, than forms of the technology having a relatively low number of vent slots. However, forms of the technology having more vent slots 7300 may be more complex and costly to manufacture.

The longer the vent slots 7300, the greater the total surface area of the vent structure 7000 that contacts the air flow. Therefore, forms of the technology having relatively long vent slots 7300 may be more effective in reducing the speed of flow, and therefore reducing the amount of noise created by the air flow, than forms of the technology having relatively short vent slots. However, in some forms it may be advantageous for the connection member 8000 to have a relatively compact design. Also, since in some forms of the technology the connection member 8000 may be formed from a rigid material, the connection member 8000 therefore adds further rigidity to the overall rigidity of the mask system, and the longer the vent slots 7300, the greater the size of the rigid connection member 8000. Therefore, vent slots 7300 longer than a certain length will result in a connection member 8000 having a length that may make it difficult for the patient to move or position the patient interface 3000 or air circuit 4170. It will be appreciated that the "certain length" that makes the connection member 8000 too long for a patient's comfort or convenient use will depend on a variety of factors, including the patient's personal preference, the type of respiratory system the connection member 8000 is used with, and the context in which it is used. Nevertheless, it is to be understood that there may, in some circumstances, be an advantage in generally keeping the connection member 8000 relatively short. In certain forms of the technology, for example where the diameter of the air circuit 4170 is 19mm, the connection member 8000 may be approximately 25mm or less in length, and in some forms of the technology it may have a length of approximately 15mm. In other forms of the technology, for example where the diameter of the air circuit 4170 is 15mm, the connection member 8000 may have a length in the range of 10mm to 20mm.

In the forms of the technology shown in Figures 8 to 39, the tube portion 8100 of the connection member 8000 comprises an outer tube portion 8110 and an inner tube portion 8120. The outer tube portion 8110 is configured to connect to the patient interface 3000 and the inner tube portion 8120 is configured to connect to the air circuit 4170. In some forms of the technology a first end portion of the outer tube portion 8110 is configured to connect to the patient interface 3000 and a first end portion of the inner tube portion 8120 is configured to connect to the air circuit 4170.

In some forms of the technology (not illustrated), the outer tube portion 7110 may be configured to connect to the patient interface 3000 by a ball and socket arrangement or a swivel ring arrangement to allow a wide range of motion between the patient interface 3000 and the connection member 8000. This decoupling arrangement assists in hindering forces applied to the air circuit from being applied to the patient interface and disrupting the seal with the patient's face. In other forms of the technology, the first end portion of the outer tube portion 8110 may be integrally formed as part of the patient interface 3000, for instance integrally formed with the plenum chamber 3200. In other forms of the technology, the outer tube portion 8110 may be removably connected to the patient interface 3000. This connection may be achieved through a clip arrangement, a screw and thread arrangement, or a snap-fit arrangement. Similarly, in some forms of the technology the first end portion of the inner tube portion 8120 may be configured to connect to an end of the air circuit 4170 via a decoupling arrangement including a ball and socket or a swivel ring arrangement. In other forms of the technology the inner tube portion 8120 may be integrally formed with an end of the air circuit 4170. Alternatively, the inner tube portion 8120 may be removably connected by a clip arrangement, a screw and thread arrangement, or a snap-fit arrangement.

In some forms of the technology, as shown in Figures 8 to 37, the outer tube portion 8110 and the inner tube portion 8120 are coaxially arranged around a central longitudinal axis, A2. At least a portion of the inner tube portion 8120 is positioned inside at least a portion of the outer tube portion 8110. The outer surface 8125 of this portion of the inner tube portion 8120 is typically separated by a distance in the range of 0.5mm to 1mm from the inner surface 8115 of this portion of the outer tube portion 8110. In the form of the technology shown in Figures 8 to 37 the inner tube portion 8120 has a smaller diameter along its entire length compared to the outer tube portion 8110. Also, in the form of the technology shown in Figures 8 to 39, the portion of the inner tube portion 8120 positioned inside the outer tube portion 8110 is a second end portion of the inner tube portion 8120, the second end portion being opposite to the first end portion that connects to the air circuit 4170. Similarly, the portion of the outer tube portion 8110 in which the inner tube portion 8120 is positioned is a second end portion of the outer tube portion 8110, the second end portion being opposite to the first end portion that connects to the patient interface 3000. In the form of the technology shown in Figures 8-17 and 28-37 the first end portion of the inner tube portion 8120 extends longitudinally out from the second end portion of the outer tube portion 8110. Similarly, at the opposite end of the connection member 8000, the first end portion of the outer tube portion 8110 extends longitudinally out from the second end portion of the inner tube portion 8120.

In certain forms of the technology the vent housing 7100 comprises the outer tube portion 8110 and the inner tube portion 8120 and the partitions 7200 and vent slots 7300 are located between the outer tube portion 8110 and the inner tube portion 8120. For example, in the forms of the technology illustrated in Figures 8 to 39, the vent slots 7300 are arranged around an annular region between the outer tube portion 8110 and the inner tube portion 8120. The partitions 7200 may be equally spaced around the annular region between the outer tube portion 8110 and the inner tube portion 8120 so that the exhaled air vents generally evenly through the plurality of vent slots 7300. In other forms, the partitions may be distributed so that there is a higher concentration of exhaled air venting in one region of the vent compared to another, for example to urge exhaled air away from, or towards, a preferred direction. In other examples, the partitions 7200 may be formed on only one part of the tube portion 8100, for example on a side of the tube portion 8100 facing generally away from the patient in use.

In some forms of the technology the partitions 7200 are formed on, or connected to, the outer surface 8125 of the inner tube portion 8120. For example, the partitions 7200 may be integrally formed as part of the inner tube portion 8120, for example in a moulding process. In alternative forms of the technology the partitions 7200 are formed on the inner surface 8115 of the outer tube portion 8110. In alternative forms of the technology the partitions may be formed as a separate component that is positioned between the inner tube portion 8120 and the outer tube portion 8110. In certain forms of the technology, the connection member 8000 is integrally formed in one-piece, for example by a moulding process.

When used with a full-face mask, the connection member 8000 may comprise, or be connected to, an AAV. The connection member 8000 may alternatively be used with other types of masks, examples of which are described herein.

The vent structure 7000 may comprise one or more of a number of sound reducing features as described below. These sound reducing features assist in reducing the sound associated with air flowing through and exiting the vent structure 7000.

### 4.6.1.1.1.2.1 Sealing member

In some forms of the technology the connection member 8000 further comprises a sealing member 8200 configured to form a seal to reduce any unwanted flow of air through any gaps or spaces (besides through the vent slots 7300) between the partitions 7200 and the vent housing 7100. Flow of air through such gaps or spaces may generate noise which may disturb the patient. The sealing member 8200 may form a seal at or proximate the vent inlet 7310 to hinder the flow of air through these gaps or spaces thereby preventing the generation of any additional noise. In forms of the technology where the partitions 7200 are formed as part of the inner tube portion 8120, the sealing member 8200 seals the space at the vent inlet 7310 between the partitions 7200 and the inner surface 8115 of the outer tube portion 8110. In forms of the technology where the partitions 7200 are formed as part of the outer tube portion 8110, the sealing member 8200 seals the space at the vent inlet 7310 between the partitions 7200 and the outer surface 8125 of the inner tube portion 8120.

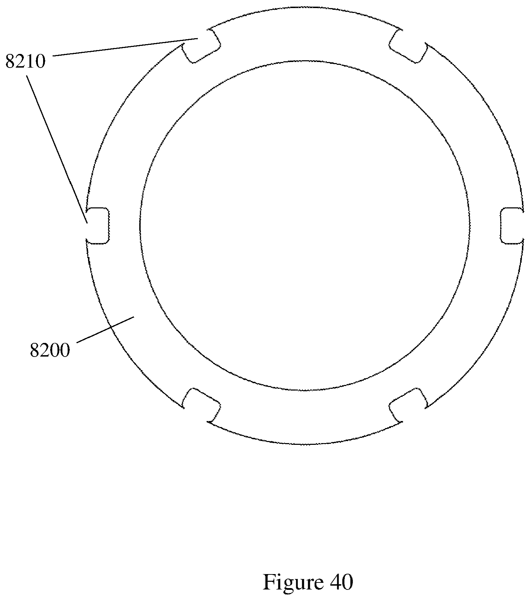

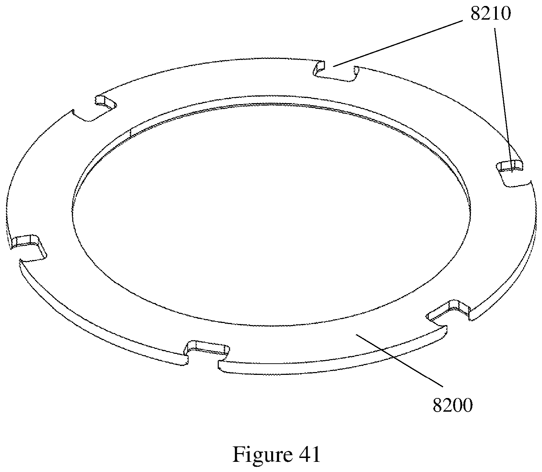

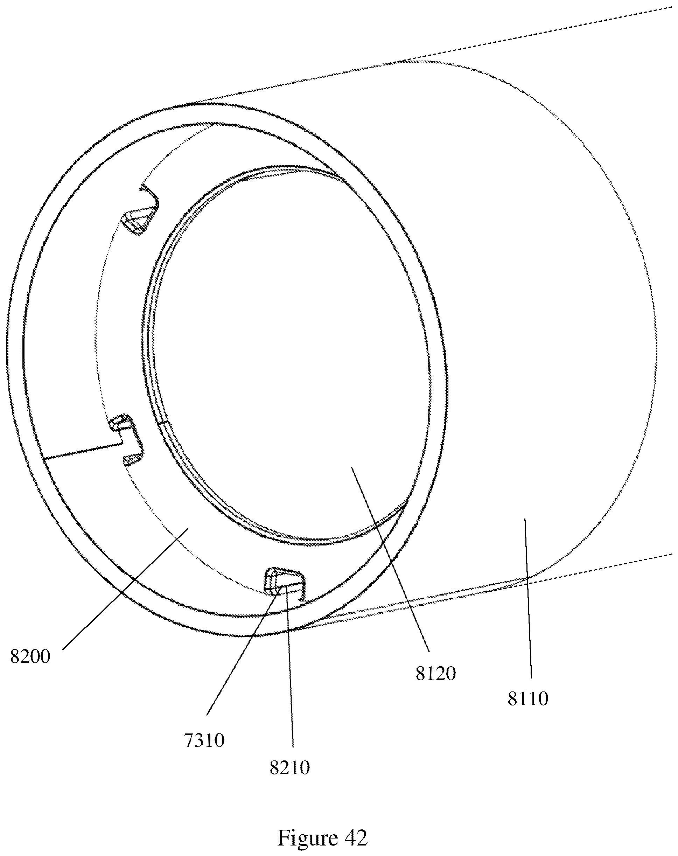

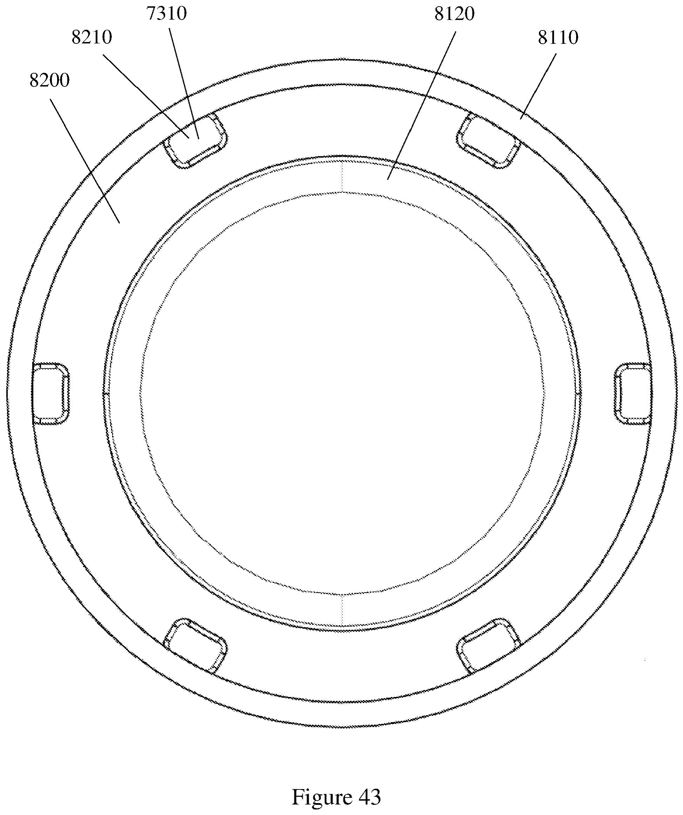

Figures 40 and 41 show a sealing member according to one form of the technology. Figures 42 and 43 show the inlet end of a connection member 8000 according to one form of the technology. The rest of the connection member may be similar to the other forms of the technology shown.

The sealing member 8200 as shown in Figures 40 to 43 is substantially annular in shape, its shape and size corresponding to the annular region between the inner tube portion 8120 and the outer tube portion 8110. In these forms the sealing member 8200 is positioned upstream, when considering the flow of exhaled air, of the partitions 7200 and therefore the vent inlets 7310, e.g. the sealing member 8200 may be closer to the patient interface 3000 than the vent inlets 7310. The sealing member 8200 is also positioned between the inner tube portion 8120 and the outer tube portion 8110, such that the hole in the centre of the annular shape aligns with the hollow portion of the inner tube portion to allow the flow of air from the flow generator through the inner tube portion 8120 to reach the patient interface 3000.

The sealing member 8200 comprises a plurality of apertures 8210. The number of apertures corresponds to the number of vent slots 7300. The shape and position of the apertures 8210 may correspond to the shape and position of the vent inlets 7310. For example, the apertures 8210 shown in Figures 40 to 43 are equally spaced around the sealing member 8200 so they align with the inlet of the respective vent slots 7300. The flow of exhaled air from the patient follows a path through the apertures 8210 and into the vent slots 7300.

In the form of the technology shown in Figures 40 to 43 the apertures 8210 are formed as openings at the outer rim of the sealing member 8200 (i.e. the edge adjacent the outer tube portion 8110 when assembled). In other forms the apertures 8210 may be formed as openings at the inner rim of the sealing member 8200 (i.e. the edge adjacent the inner tube portion 8120 when assembled). In other forms the apertures 8210 may be formed as openings through the central region of the sealing member 8200.

As can be seen in Figure 40 the apertures 8210 may have a substantially rectangular cross-sectional shape. This corresponds with the shape of the vent inlets 8310 as shown in Figure 42. In other forms of the technology the cross-sectional shape of the apertures 8210 may have another shape for example circular, square, or triangular.

The sealing member 8200 may be formed from a flexible material, for instance a silicone or rubber. The sealing member 8200 may be friction fit or interference fit between the inner tube portion 8120 and the outer tube portion 8110. In other forms of the technology the sealing member may comprise a snap fit connection between the inner tube portion 8120 and/or the outer tube portion. In other forms of the technology the sealing member may be adhered to or integrally formed with the inner tube portion 8120 and/or the outer tube portion 8110.

### 4.6.1.1.1.2.2 Change in cross sectional area along length of vent slot

In certain forms of the technology the vent structure 7000 comprises partitions 7200 which are formed such that each vent slot 7300 has a cross-sectional area at a first region 7330 of the vent slots 7300 that is smaller than a cross-sectional area at a second region 7340 of the vent slots 7300, the first region 7330 being closer to the vent inlet 7310 than the second region 7340. In some forms of the technology the first region 7330 is proximate the vent inlet 7310 and/or the second region 7340 is proximate the vent outlet 7320. For example, the partitions 7200 may be formed such that the vent inlet 7310 has a smaller cross-sectional area than the vent outlet 7320.

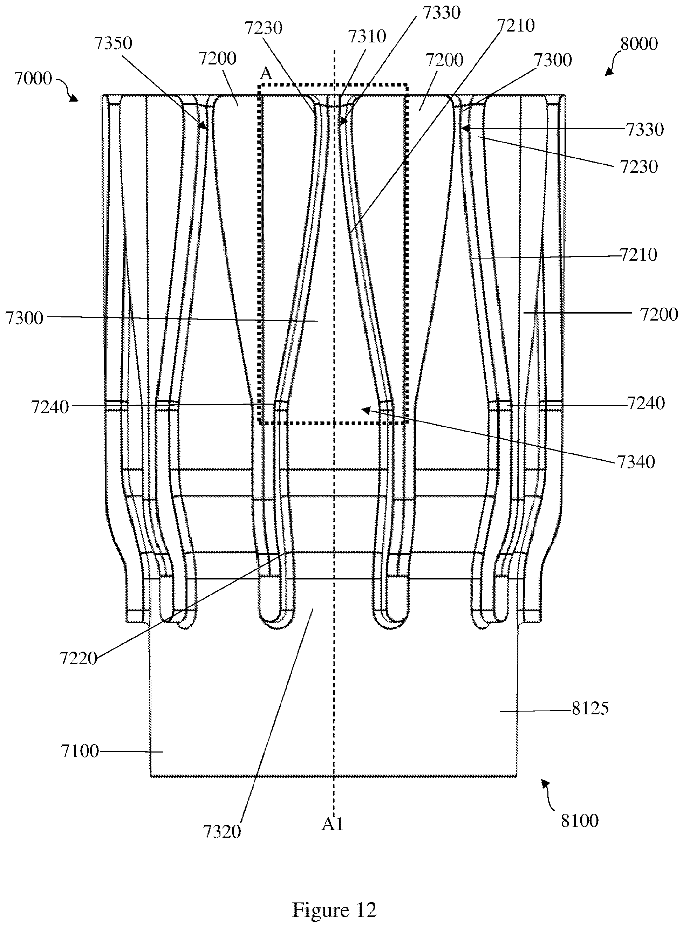

Each of the vent slots 7300 has a longitudinal axis along the length of the respective vent slot 7300, i.e. in the inlet-outlet direction. In Figures 12, 13, 16, 17, 22, 23, 26, 27, 32, 33, 36, 37 and 38, the longitudinal axis of one of the vent slots 7300 is labelled as A1. The longitudinal axis A1 is generally parallel to the net direction of flow of air through the vent slot 7300. In the examples shown the axis A1 is substantially parallel to the central axis A2 as described above. The first region 7330 is located at a longitudinal position upstream of the second region 7340. The vent slot 7300 expands, i.e. the cross-sectional area increases, from the first region 7330 to the second region 7340. The increase in cross-sectional area along the vent slot 7300 results in an expansion of the air flowing through the vent slot 7300 which reduces the speed of the air flow. A slower air flow through a vent slot 7300 produces less noise. Expansion of the airflow also results in some of the air flow changing direction, for example some of the air flow moves towards the walls of the vent slots 7300 thereby increasing the frictional or drag forces acting on the air flow which slows down the air flow further and further reduces noise.

In the forms of the present technology shown in Figures 8 to 39, the second region 7340 of each vent slot 7300 has a larger width compared to the first region 7330 of the respective vent slot 7300. In some forms of the technology the vent housing 7100 is formed such that the second region 7340 of each vent slot 7300 has a greater height compared to the first region 7330 of the respective vent slot 7300. In some forms of the technology there is both an increase in width and height of the vent slot 7300 between the first region 7330 and the second region 7340. In other forms of the technology there is only an increase in width or height of the vent slot 7300 between the first region 7330 and the second region 7340. These forms of the technology will now be described in more detail.

### 4.6.1.1.1.2.2.1 Change in width of the vent slot

In some forms of the technology, as shown in Figures 8-37, there may be an increase in width between a first region 7330 and second region 7340 of the vent slot 7300 and an increase in height between a third region 7335 and a fourth region 7345 of the vent slot 7300, the third region 7335 being located at a longitudinal position upstream of the fourth region 7345. In Figures 8-27 the third region 7335 and the fourth region 7345 are both located downstream of the first region 7330 and the second region 7340. In Figures 28-36 the third region 7335 and the fourth region 7345 are located upstream of the first region 7330 and the second region 7340. In some forms of the technology the first region 7330 and the third region 7335 are the same region of the vent slot 7300 and the second region 7340 and the fourth region 7345 are the same region of the vent slot 7300. In the illustrated forms of the technology, the vent slots 7300 also have a greater width at the vent outlet 7320 compared to the vent inlet 7310.

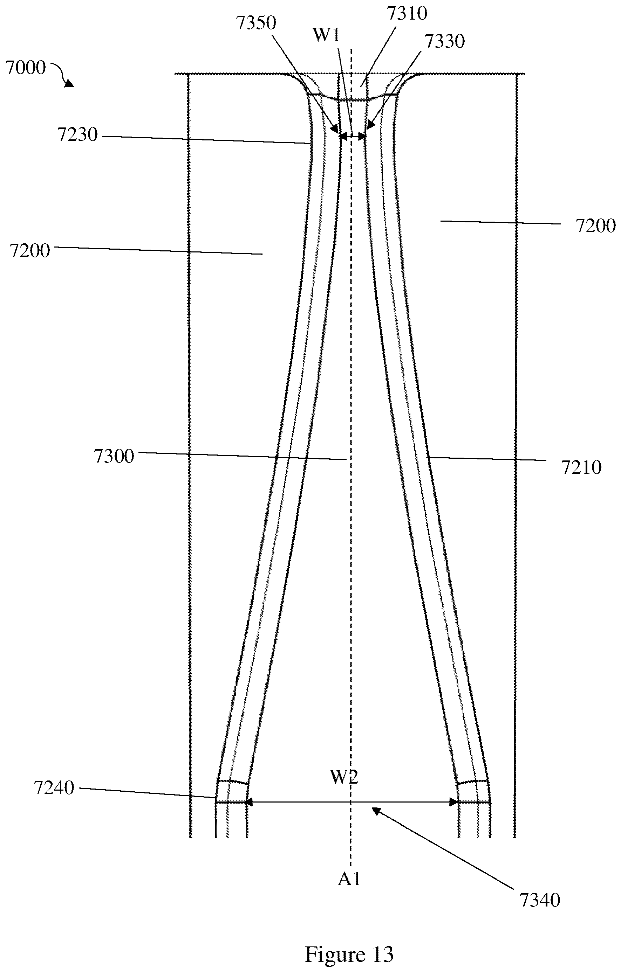

As seen in Figures 13, 23, and 33 a width, W1, of a vent slot 7300 in the first region 7330 is smaller than a width, W2, of the vent slot 7300 in the second region 7340. The width W1 may be in the range of 0.1mm to 0.5mm. In some forms of the technology the width W1 may be 0.2mm. The width W2 may in the range of 3.5mm to 5mm. In some forms of the technology the width W2 is 4.3mm. The width of the vent slot 7300 as shown is a distance between adjacent partitions 7200. The width is substantially perpendicular to the plane of the inner surface of the partitions 7200 that contacts the air in the vent slots 7300. In the form of the technology as shown in the Figures 8-39 where the vent structure 7000 is comprised as part of a connection member 8000 that is generally cylindrical in shape, the width of each vent slot 7300 is a distance measured in a circumferential direction. Therefore, the increase in width is an increase in the size of the vent slots in a circumferential direction or a circumferential expansion.

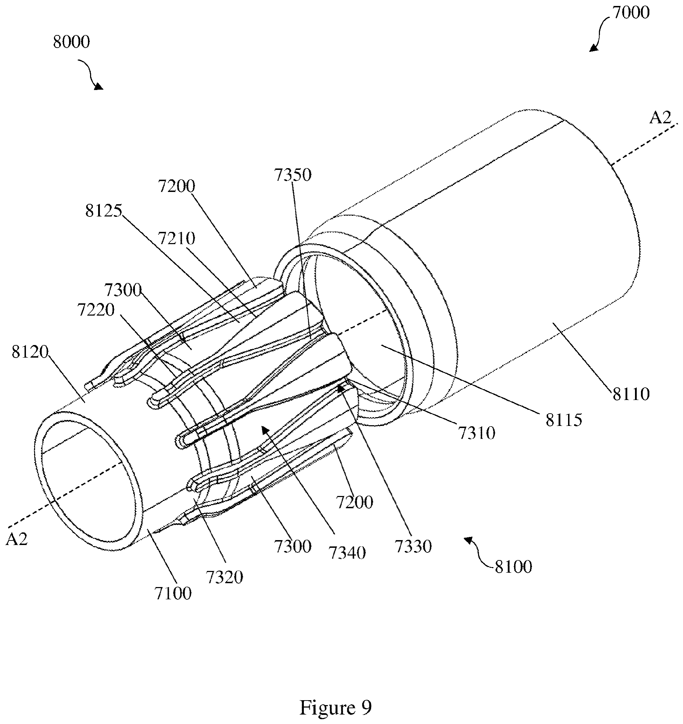

In some forms of the present technology, for example as illustrated in Figures 9, 10, 12, 13, 19, 20, 22, 23, 29, 30, 32, and 33, each partition 7200 comprises an angled straight wall portion 7210 between the first region 7330 and the second region 7340. The angled straight wall portion 7210 is oriented at an angle to the longitudinal axis A1. The angled straight wall portion 7210 forms part of the face of the partition 7300 which forms one side of the vent slot 7300. The angled straight wall portion 7210 is angled outwardly in the downstream direction away from the longitudinal axis A1 so that the width of the vent slot 7300 increases from the first region 7330 to the second region 7340. In some forms of the technology the angled straight wall portion 7210 forms between half and two thirds of the length of the vent slot 7300.

In Figures 8-17, the angled straight wall portions 7210 of each vent slot 7300 are oriented at a 7.5° angle from the longitudinal axis. This may be referred to as the angle of attack. In alternate embodiments the angle of attack may be smaller or larger. For example, in the form of the technology shown in Figures 18-27, the angled straight wall portions 7210 of each vent slot 7300 are oriented at a 5° angle from the longitudinal axis A1. In some forms of the technology some or all of the vent slots 7300 of the vent structure 7000 may have different angles of attack from other vent slots 7300.

In any given application or location of the vent structure 7000 (for example in a connection member 8000 between an air circuit 4170 and patient interface 3000), it may have an overall size that, irrespective of how the configuration of the vent slots 7300 are varied in other forms of the vent structure 7000 in the same application or location, the vent structure has the same or similar overall size. For example, in the case of the vent structure 7000 forming part of a connection member 8000 as shown in Figures 8 to 39, the circumference of the connection member 8000 may be the same between different connection members 8000 having different configurations of vent slots 7300. In such circumstances the number of vent slots 7300 is linked to the angle of attack in each vent slot. In the form of the technology shown in Figures 8-17 the connection member 8000 comprises ten vent slots 7300 and the vent slots 7300 have an angle of attack of 7.5°. In the form of the technology shown in Figures 18-27 the connection member 8000 comprises 15 vent slots 7300 and the angle of attack is 5°. In other forms of the technology, the angle of attack may be reduced further, for example to 2.5°, which would allow an even greater number of vent slots 7300. As noted above it may be advantageous to have a greater number of vent slots 7300 as this increases the overall surface area presenting frictional drag to the air flow through the vent and reduces the noise of the air flow. Having a greater angle of attack results in a greater expansion of the air flow through the vent slot 7300 due to the greater increase in width and therefore also results in slowing the air flow, however there may be room for fewer vent slots. For a given circumferential size of connection member there is a trade-off between increasing the number of vent slots and having a suitable angle of attack. The angle of attack may be selected to allow a high level (for example, maximum) flow expansion through the vent slot without causing excessive flow detachment or separation from the walls of the vent slots, which as described above increases the noise of the air exiting the vent. Additionally, increasing the number of vent slots 7300 also increases the complexity of the design. This may result in difficulties in manufacturing the vent structure 7000 which in turn can lead to an increase to the cost of machining the vent structure 7000. An increase in the number of vent slots 7300 may also lead to an increased difficulty in cleaning the vent structure 7000 as each vent slot may accumulate dirt and there are more slots to clean and each is narrower so more difficult to clean. These various factors are considered when determining the design of the vent structure 7000, in particular the number of vent slots 7300 and the angle of attack of the vent slots.

In some forms of the present technology, as can be seen in Figures 12, 22, and 32, each partition 7200 comprises a parallel straight wall portion 7220 which is substantially parallel to the longitudinal axis A1. In the form of the technology shown in Figures 8-27 the parallel straight wall portion 7220 is proximate the vent outlet 7320 and positioned downstream from the angled straight wall portion 7210 along the length of the vent slot 7300. The parallel straight wall portion 7220 continues to slow down the flow of air by providing frictional/drag forces to the flow of air.

In some forms of the technology, as can be seen in Figures 12, 13, 22, 23, 32 and 33, each of the partitions 7200 further comprise a negatively curved portion 7230 between the first region 7330 and the second region 7340. The negatively curved portion 7230 is adjacent the first region 7330 and is at a longitudinal position upstream of the angled straight wall portion 7210. The negatively curved portion 7230 forms part of the face of the partition 7200 which forms one side of the vent slot 7300. The negatively curved portion 7230 has a relatively small negative curvature and essentially creates the angle by which the angled straight wall portion 7210 is oriented away from the longitudinal axis A1 of the vent slot 7300.

In some forms of the technology, as can be seen in Figures 12, 13, 22, 23, 32 and 33, each of the partitions 7200 further comprises a positively curved portion 7240 between the first region 7330 and the second region 7340. The positively curved portion 7240 may be adjacent the second region 7340 and is positioned downstream of the angled straight wall portion 7210. The positively curved portion 7240 forms part of the face of the partition 7300 which forms one side of the vent slot 7300. The positively curved portion 7240 has a relatively small positive curvature from one upstream longitudinal position to a downstream longitudinal position. The straight wall portion 7210 of each of the partitions 7200 is located between the negatively curved portion 7230 and the positively curved portion 7240.

In some forms of the technology (not illustrated), each of the partitions 7200 may comprise a curved portion extending between the first region 7330 and the second region 7340. The curved portion may comprise a convex portion and a concave portion joined together such that the curve is continuous. The point of inflection, i.e. where the curved portion changes from convex to concave, may be located at substantially midway between the first region 7330 and the second region 7340. The concave portion may be closer to the vent inlet 7310 and the convex portion may be closer to the vent outlet 7320. The gradient of the concave and convex portions may be similar to the negatively curved portion 7230 and the positively curved portion 7240 as described above and shown in Figures 12, 13, 22, 23, 32 and 32. This curved portion may be configured to reduce the noise of the air as it flows through the vent slots. The shape may improve the attachment of the air flow to the sides of the vent slots. This increases the frictional or drag forces acting on the air flow thereby reducing the speed of the air flow. If the air separates from the sides of the vent slots a low pressure region forms where this occurs. This creates turbulence which is one of the main sources of aero-acoustic noise. Shaping the vent slots 7300 to increase attachment of the air flow to the sides of the vent slots 7300 reduces this source of noise. In some forms of the technology each partition 7200 is configured with an arrangement of wall portions between the first region 7330 and the second region 7340 to give the inner face of the partition 7200 presented to the air flow an aerofoil-like shape.

As shown in Figures 8-39 the vent slots 7300 may have an initial decrease in width to create a bottleneck region 7350. The bottleneck region 7350 may be understood to be a region of the vent slot of narrowest width compared to other regions of the vent slot. In the form of the technology shown in Figures 8-39 the bottleneck region 7350 is located proximate to the vent inlet 7310. The portion of the partition 7200 forming the vent inlet 7310 may have a relatively small negative curvature. The bottleneck region 7350 assists in maintaining the required positive pressure in a component upstream of the vent structure 7000, for example the patient interface 3000. The bottleneck region 7350 may also assist in ensuring that water droplets from the patient's exhaled air does not block the vent slots 7300. The local decrease in width of the vent slot 7300 in the bottleneck region 7350 causes the air flow to accelerate through that region to maintain a constant flow through the vent slot 7300. This increase in speed of the air flow will assist in ensuring that water droplets do not accumulate in the vent slots 7300. In certain forms of the technology, the narrowest width of the vent slots 7300 is 0.7mm. If the width is smaller than this the surface tension of the water droplets may cause the water droplets to block the vent slot 7300.

In the forms of the technology shown in Figures 8-39 adjacent partitions 7200 are formed so that the vent slots 7300 formed therebetween are substantially symmetrical along the central longitudinal axis of the vent slot A1. Also, in the forms of the technology shown in Figures 8-39, the shape and size of the partitions 7200 are all substantially the same. In alternative forms of the technology the partitions 7200 may be of different shapes or sizes.

### 4.6.1.1.1.2.2.2 Change in height of the vent slot

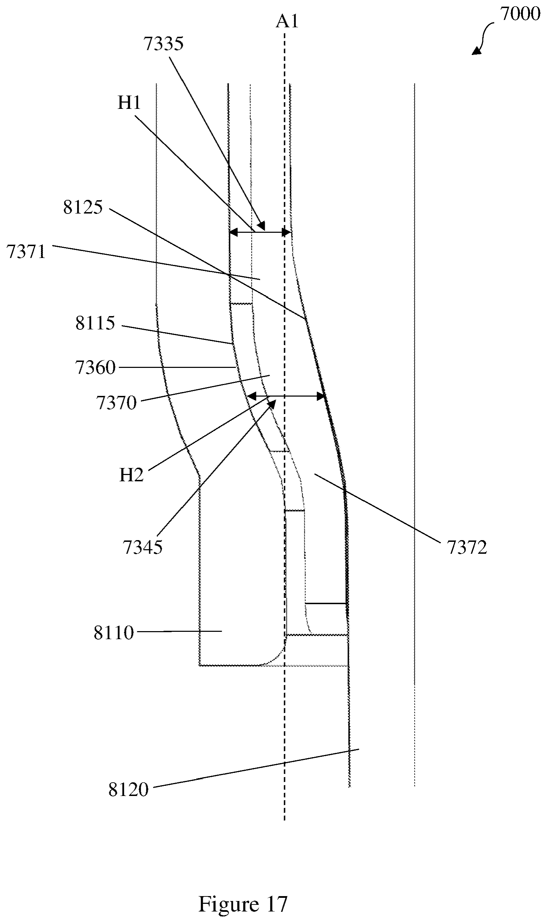

As seen in Figures 17, 27, and 37, in certain forms of the technology, a height, H1, in the third region 7335 of each vent slot 7300 is smaller than a height, H2, in a fourth region 7345 of each vent slot 7300 where the fourth region 7345 is located downstream of the third region 7335. The height H1 may be in the range 0.5mm to 1.5mm. In some forms of the technology the height H1 may be 1mm. The height H2 may be in the range 0.8mm to 1.5mm. In some forms of the technology the height H2 may be 1.2mm. The height of the vent slot 7300 may be understood to be a distance between walls of the vent slot 7300 in a direction perpendicular to the width and the longitudinal axis A1 of the vent slot 7300. The height of the vent slot 7300 may span from one part of the vent housing 7100 to another, for example as mentioned above in some forms of the technology the vent housing 7100 may define upper and lower walls of each vent slot 7300. In the form of the technology shown in the Figures 8-39, where the vent structure 7000 is comprised as part of a connection member 8000 that is generally cylindrical in shape, the height of each vent slot 7300 is a distance in the radial direction and the increase in height amounts to a radial expansion of the vent slot 7300 or an expansion of the vent slot in the radial direction. This results in an expansion of the air flow which slows down the flow of air as described above.

In some forms of the technology, as seen in Figures 17, 27, and 37, each vent slot 7300 comprises an expanding height portion 7360 having an upstream end and a downstream end. The upstream end of the expanding height portion has a lesser height than the downstream end of the expanding height portion 7360. The distance between the upstream end and the downstream end of the expanding height portion 7360 may be small compared to the distance between the vent inlet 7310 and the vent outlet 7320. The relatively small distance between the upstream end and the downstream end results in a rapid change in the height which results in a rapid expansion of the air flow moving through the vent slot. This leads to a quick and effective reduction in the speed of the air flow and therefore the noise that flow generates. The expanding height portion 7360 is preferably located at or proximate a region of minimum width of the respective vent slot 7300. For example, in the form of the technology shown in Figures 28-38 the expanding height portion 7360 is at the same location or proximate to the bottleneck region 7350.

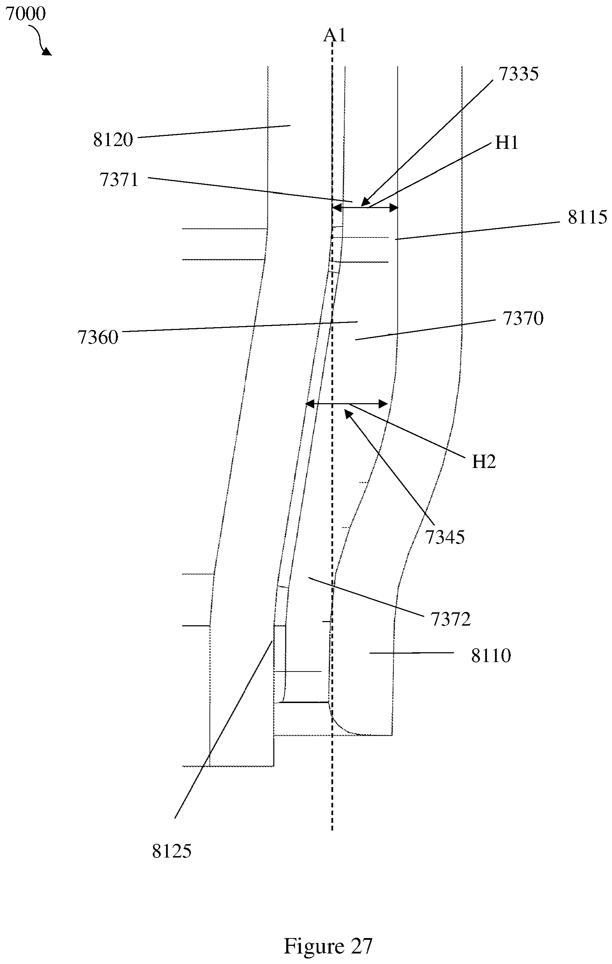

In some forms of the technology, as shown in Figures 8-37, the increase in height of the vent slot 7300, through the expanding height portion 7360 or otherwise, is due a divergence between the outer surface 8125 of the inner tube portion 8120 and the inner surface 8115 of the outer tube portion 8110 along the length of the vent slot 7300. This divergence may be achieved in a number of ways, including through a change, in the longitudinal direction, in the distance between the outer surface 8125 of the inner tube portion 8120 and the central longitudinal axis, A2, of the inner tube portion 8120 between the third region 7335 and the fourth region 7345, or a change in the distance between the inner surface 8115 of the outer tube portion 8110 and the central axis, A2, between the third region 7335 and the fourth region 7345, or a combination of changes to these distances. For example, as can be seen in Figures 17, 27, and 37 there is a decrease in diameter of the inner tube portion 8120 between the third region 7335 and the fourth region 7345. Additionally, there is a decrease in diameter of the outer tube portion 8110, between the third region 7335 and the fourth region 7345. The decrease in diameter of the inner surface 8115 of the outer tube portion 8110 less than the decrease in diameter of the outer surface 8125 of the inner tube portion 8120 between the third region 7335 and the fourth region 7345, which results in an increase in height of the vent slot 7300 along the vent slot. In the form of the technology shown in Figures 17 and 27 the outer surface 8125 of the inner tube portion 8120 in the expanding height portion 7360 is sloped at a constant angle with respect to the central axis A2. The inner surface 8115 of the outer tube portion 8110 in the expanding height portion 7360 has a negative curvature from the upstream end to the downstream end.

The inner surface 8115 of the outer tube portion 8110 may reduce in diameter along the vent slot 7300 in order to form part of a deflector 7400, as described below.

In alternative forms (not illustrated) the increase in height along the vent slot 7300 is achieved by increasing the diameter of the inner surface 8115 of the outer tube portion 8110 between the third region 7335 and fourth region 7345 whilst the inner tube portion 8120 remains unchanged. In alternative forms the increase in height along the vent slot 7300 is achieved by decreasing the diameter of the outer surface 8125 of the outer tube portion 8120 between the third region 7335 and the fourth region 7345 whilst the outer tube portion 8120 remains unchanged. In alternative forms, as described above and shown in Figures 17, 27, and 37, the change in height is achieved by a combination of a change in the inner tube portion 8120 and the outer tube portion 8110. In other forms of the technology, the increase in height of the vent slot is due to some other change in the shape of the vent housing 7100 which causes the vent slot to increase in height. For example, the outer surface 8125 of the inner tube portion 8120 may have a positive curvature with a gradient that forms an increase in height between the third region 7335 and the fourth region 7345. In other forms both the inner surface 8115 of the outer tube portion 8110 and the outer surface 8125 of the inner tube portion 8120 may be sloped at a constant angle with respect to the central axis A2, the inner surface 8115 having a shallower gradient than that of the outer surface 8125 to form an increase in height between the third region 7335 and the fourth region 7345.

In some forms of the technology, as shown in Figures 17, 27, and 37, after the expanding height portion 7360 the vent slot 7300 decreases in height until its height is the same as the initial height of the vent slot 7300. It will be appreciated how this decrease in height of the vent slot 7300 may be achieved using any one or more of the different ways to vary the height of the vent slot 7300 along its length discussed above in the context of an increase in the height of the vent slot 7300. In some forms, the vent slots 7300 have substantially the same height at the vent outlet 7320 compared to the vent inlet 7310. In such forms, the fourth region 7345 is not located at or closely proximate to the vent outlet 7320, i.e. there is a portion of the vent slot 7300 between the fourth region 7345 and the vent outlet 7320.

### 4.6.1.1.1.2.2.3 Acoustic attenuation chamber

In the forms of the technology shown in Figures 8-37, the vent structure 7000 is formed such that each vent slot 7300 comprises a chamber 7370 arranged to, in use, reflect sound waves and attenuate the sound of a flow of air through the respective vent slot 7300. In other forms of the technology (not shown), the vent structure 7000 may be formed such that each vent slot 7300 comprises more than one chamber 7370. In some forms two chambers 7370 may reduce the noise associated with air flowing through the vent more than one chamber. The two chambers may be longitudinally separated in the vent slot 7300 by a portion of the slot having a smaller height than each chamber.

The chamber 7370 may be formed as an enlarged region of the vent slot 7300, for example a region having a greater height and/or width than adjacent regions of the vent slot 7300. For example, in the forms of the technology shown in Figures 8-37, the chamber 7370 is formed at or proximate fourth region 7345, where the vent slot 7300 has an increased height compared to another region of the vent slot. Also in these forms the expanding height portion 7360 of the vent slot 7300 may form part of the chamber 7370. In the forms of the technology shown in Figures 8-37, the chamber 7370 is formed partially by the deflector 7400 as described below.

The chamber 7370 may be a form of acoustic attenuation chamber. The acoustic attenuation chamber reduces the noise of the air flow. In use, acoustic waves created by the air flowing through the vent slot reflect off the walls of the chamber 7370 and can interfere with other acoustic waves. For example, the acoustic chamber 7370 in the exemplary forms of the technology illustrated has an upper wall at an angle of approximately 15° to 20° from the axis of the tube portion A2. Interference of the acoustic waves reflected off this wall with the source of the acoustic waves (for example those generated on the lower wall) may result in the cancellation of some of the acoustic waves at certain wavelengths or frequencies. This will assist in reducing the noise of the air flow in the vent structure 7000.

In the forms of the technology shown in Figures 17, 27, and 37 at least a part of the chamber 7370, or walls forming part thereof, is formed to have a concave shape. This shape may increase the amount of interference of the acoustic waves and promote greater acoustic attenuation compared to a non-concave form.

An increased steepness of the walls of the chamber 7370 (i.e. the angle of the walls of the chamber 7370 relative to central axis A2) results in a more drastic change in the direction of the air flow compared to forms of the technology with shallower such angles. This enhances the acoustic attenuation effects of the chamber and reduces the flow velocity. In some forms of the technology, this may be achieved by providing a relatively steep outer surface 8125 of the inner tube portion 8120 and/or a relatively steep inner surface 8115 or the outer tube portion 8110. The need to balance this consideration against the advantages of a compact design may limit the steepness that the walls of the chamber 7370 are given in practice.

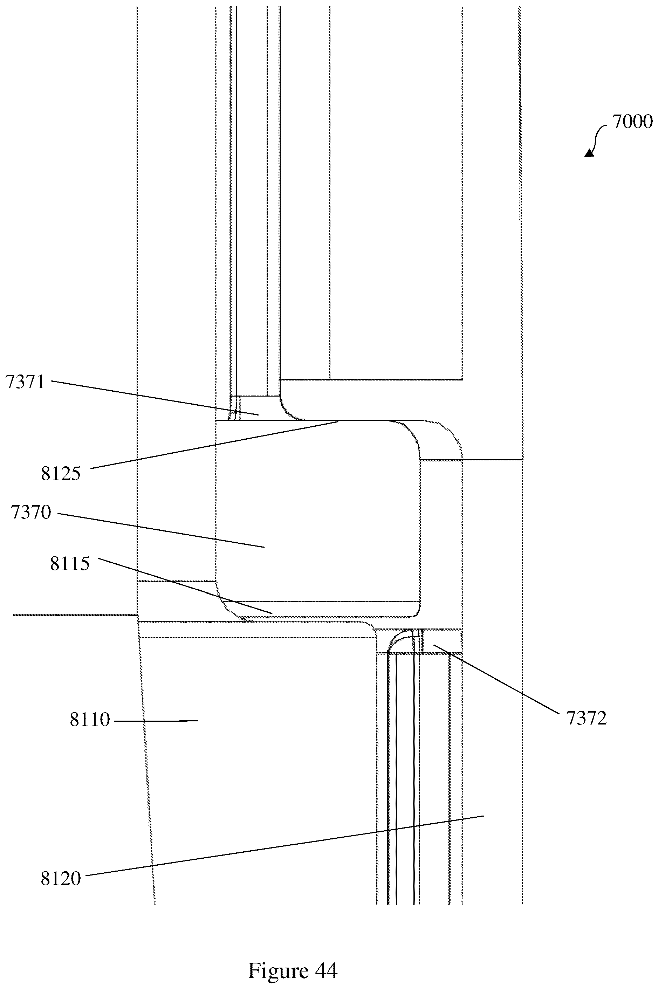

In the form of the technology shown in Figure 44, an alternative form of an acoustic attenuation chamber is shown with a vent slot 7300 having a chamber 7370 with a substantially square cross-section, the square having rounded corners. This shape increases the steepness of the walls of the chamber 7370 compared to those in the forms of the technology discussed above. A portion of the outer surface 8125 and the inner surface 8115 are perpendicular to the central axis A2. This results in a more drastic change in the direction of the air flow with the air flow turning 90° in two places within the chamber 7370 and assists in reducing the velocity of the air flow.

In some forms of the technology the inlet 7371 to the chamber 7370 is offset from the outlet 7372 to the chamber 7370. The offset between the inlet 7371 and the outlet 7372 results in a vent slot 7300 that is not straight, i.e. one longitudinal section of the vent slot 7300 has an axis that is offset from the axis of another longitudinal section of the vent slot 7300. This assists in preventing noise generated upstream of the chamber 7370 propagating downstream of the chamber 7370. If the inlet 7371 and the outlet 7372 are offset any acoustic waves travelling through the vent slots 7300 are more likely to encounter at least a portion of a wall of the vent slot 7300 which may result in interference of the acoustic waves as discussed above and therefore a reduction in noise.

In the forms of the technology shown in Figures 8-37, and 44 the inlet 7371 is radially offset from the outlet 7372. For example, as shown in Figures 17, 27, 37, and 44 the outlet 7372 may be closer to the central axis A2 than the inlet 7371. In other forms of the technology the outlet 7372 may be further from the central axis A2 than the inlet 7371. In other forms of the technology (not illustrated) the inlet 7371 to the chamber 7370 may be circumferentially offset from the outlet 7372 to the chamber 7370. In other forms of the technology (not illustrated) the inlet 7371 to the chamber 7370 may be both circumferentially and radially offset from the outlet to the chamber 7370. This configuration may further reduce the velocity of the air flow as it increases further the chance of the sound waves contacting a portion of the wall of the vent slot 7300. Having the inlet 7371 of the chamber 7370 radially offset from the outlet 7372 of the chamber 7370 may be easier to tool than having it circumferentially offset.

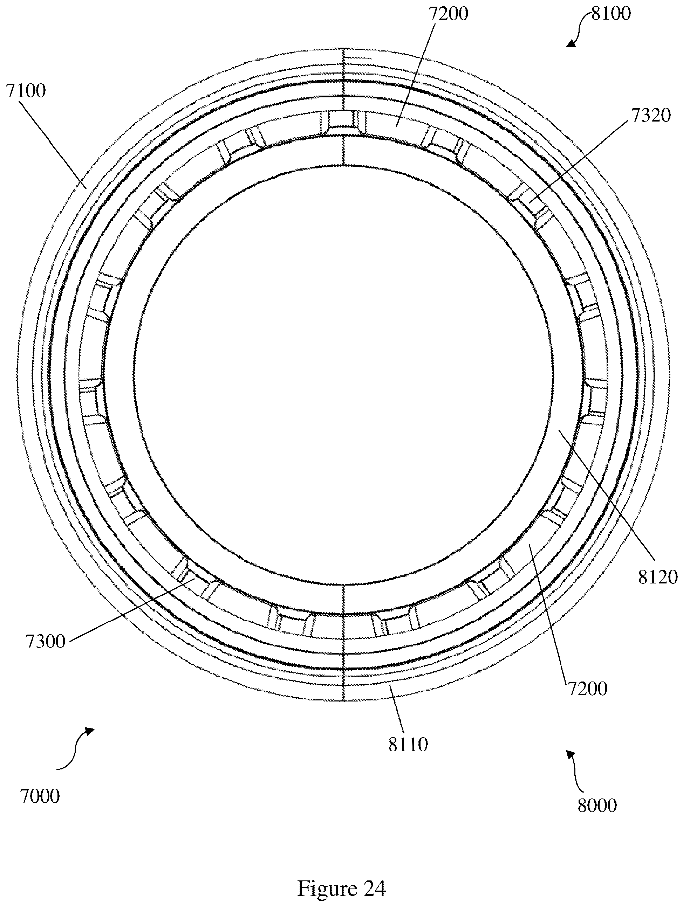

In the form of the technology shown in Figures 24-31, the chamber 7370 is located at or proximate the longitudinal position along the vent slot 7300 where the vent slot 7300 has the smallest width, i.e. the bottleneck region 7350. In these forms, this position is proximate the vent inlet 7310. In the forms of the technology shown in Figures 8-27, the chamber 7370 is located close to the vent outlet 7320. Locating the chamber 7370 closer to the vent inlet 7310 has, in some forms, been found to result in an increased noise reduction compared to forms of the technology where the chamber is located at a longitudinal position along the vent slot 7300 further from the vent inlet 7310. Such a location may position the chamber 7370 proximate the location of maximum air flow velocity through the vent slot 7300. As the chamber 7370 significantly changes the direction of air flow in the direction perpendicular to the flow, locating the chamber 7370 at or proximate this point may significantly reduce the velocity of the air flow. Locating the chamber 7370 closer to the vent inlet 7310 and having an increase in the width of the vent slot 7300 in a region of the vent slot 7300 after the chamber 7370 may reduce flow separation, i.e. the air flow is better attached downstream of the chamber 7370. A reduction in flow separation reduces the noise associated with the air flow through the vent as described above. Locating the chamber 7370 closer to the vent inlet 7310 decreases the velocity of the air flow prior to the increase in width of the vent slot 7300. A slower flow of air may result in better flow attachment to the side walls of the vent slot 7300 when the flow expands. As discussed herein, this further reduces the speed of the air flow due to the increased frictional forces acting on the flow of air.

In Figures 28-37 the chamber 7370 is followed by a section of the vent slot 7300 which increases in width as described above. In other forms of the technology, downstream of the chamber 7370 the vent slot 7300 has a constant width and height. This section of the vent slot 7300 slows down the speed of the air flow further and decreases the extent of flow separation at the vent outlet 7320. This section may be formed by a parallel straight wall portion 7220 as described above.

### 4.6.1.1.1.2.3 Deflector

In certain forms of the technology the vent structure 7000 comprises a plurality of deflectors 7400 configured to deflect the flow of air through each vent slot 7300 towards a part of the vent housing 7100 and/or another component in the respiratory system. In the forms of the technology shown in Figures 8-37, each vent slot 7300 has a respective deflector 7400 to deflect the flow through that respective vent slot 7300.

The deflector 7400 in each vent slot 7300 may be a wall of the vent slot 7300, or a separate component positioned in the path of the air flow through the vent slot 7300, positioned and arranged at an angle to the general direction of the air flow through the vent to cause the air flow to change direction when it contacts the deflector 7400. The deflector 7400 acts as a brake to slow down the flow through friction caused by the flow contacting the deflector 7400.

Each deflector 7400 is positioned and arranged to cause the exiting air flow to flow against a component downstream of the deflector 7400 and, in some forms of the technology, a component downstream of the vent outlet 7320. That downstream component may be another part of the vent structure 7000 or another part of the respiratory system. If the air exiting the vent outlet 7320 is directed towards another component, that component will apply frictional or drag forces to the flow of air, reducing the speed of the flow of air, and therefore the level of noise it creates. Directing the flow of air exiting the vent outlet 7320 towards a component that is, for example part of the vent structure 7000 or respiratory system, will also reduce jets of air being directed towards, and therefore disturbing, the patient 1000 or the bed partner 1100. Additionally, a reduction in the speed of the air flow will also reduce the disruptions to the patient 1000 or bed partner 1100.

The plurality of deflectors 7400 may be provided to, for example formed on or connected to, an inner surface 7110 of the vent housing 7100. In the form of the technology shown in Figures 8-39 the inner surface 8115 of the outer tube portion 8110 has a positive curvature which forms the deflector 7400 by presenting a surface in the path of the flow of air that is at an angle to the general direction of air flow towards the surface. The inner surface 8115 of the outer tube portion 8110 curves in a direction radially inwardly, i.e. towards the central axis A2. As the flow of air contacts, the deflector it also changes in direction radially inwardly towards the central axis A2. In some forms of the technology the curve of the inner surface 8115 of the outer tube portion 8110 may have a larger positive curvature compared to the forms of the technology shown in the Figures, i.e. the curve may have a steeper portion, to change the direction of the flow more rapidly or further upstream. The deflector 7400 may have an angle with respect to the longitudinal axis of the vent slot, A1, of between 5-10°.

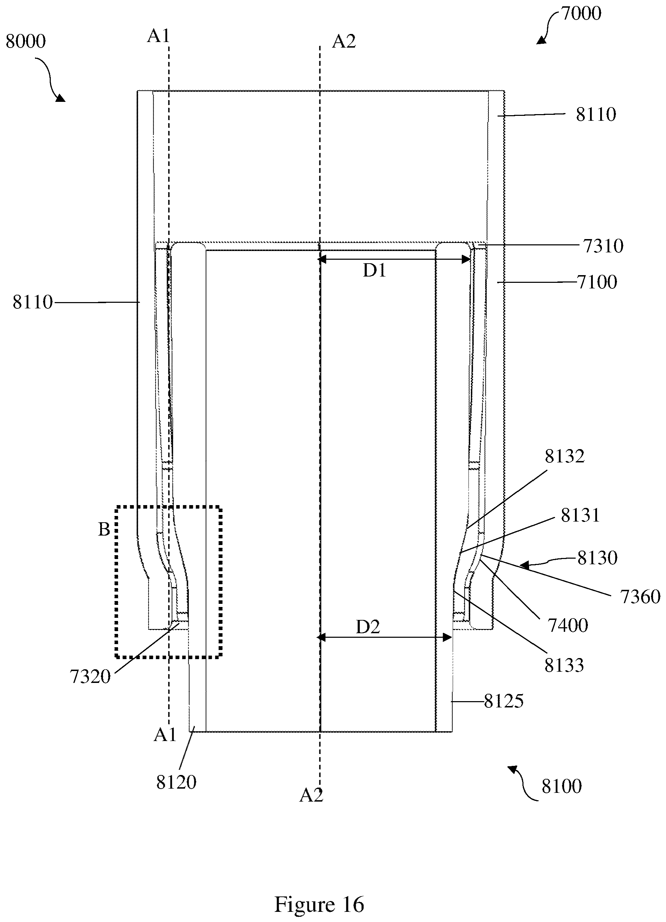

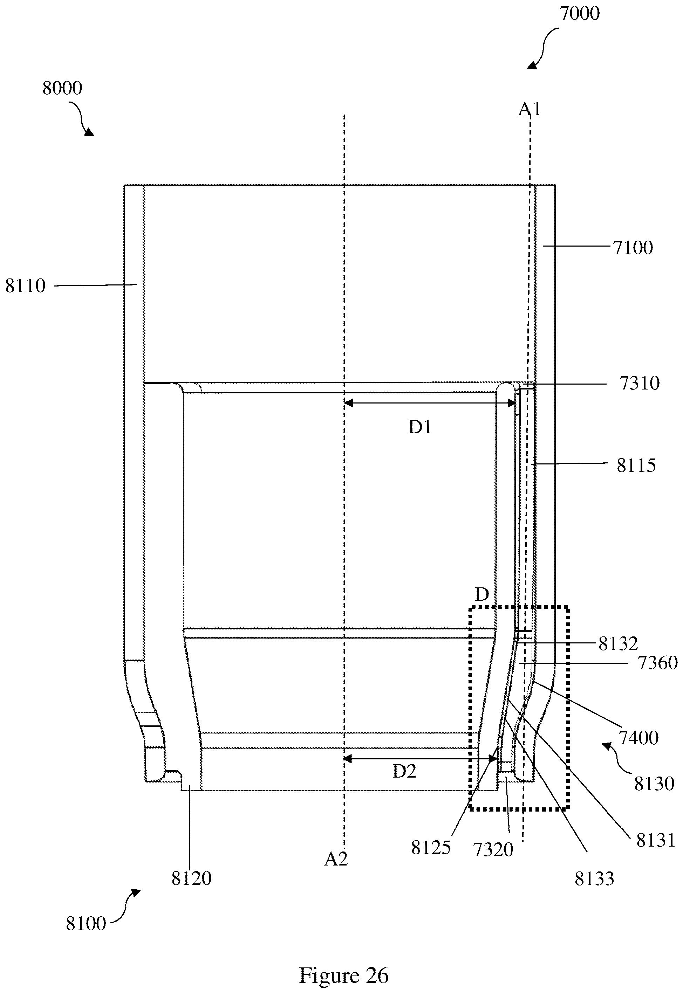

In the form of the technology shown in Figures 16 and 26, the deflectors 7400 are formed by the inwardly curving inner surface 8115 of the outer tube portion 7110. This part of the inner surface 8115 of the outer tube portion 8110 may also form part of the expanding height portion 7360 and the acoustic attenuation chamber 6370 as described above. That is, the same part of the vent structure 7000 may serve multiple purposes.

In the form of the technology shown in Figures 28-37, each deflector 7400 is located at a longitudinal position within one of the vent slots 7300 where the respective vent slot 7300 has minimum width, i.e. the deflector 7400 is located at the bottleneck region 7350. For example, each deflector 7400 may be positioned at a position along the length of each vent slot 7300 proximate the vent inlet 7310.

In the form of the technology shown in Figures 18 to 27 the downstream component that the air flow is deflected towards is the outer surface 8125 of the inner tube portion 8120 downstream of the deflector 7400 and/or the air circuit 4170 connected to the inner tube portion 8120 in use (not shown in the figures). The deflectors 7400 in this example may act to direct air towards the air circuit 4170 because they are located proximate the vent outlet 7320. In the form of the technology shown in Figures 8-17, the downstream component that the air flow is deflected towards is a portion of the connection member 8000, in this example the outer surface 8125 of the inner tube portion 8120, that is located downstream of the vent outlet 6320. The deflectors 7400 in this example may act to direct air towards these components because the deflectors 7400 are located adjacent the vent outlet 7320 and a portion of the inner tube portion 8120 extends from the vent outlet 7320. In the form of the technology shown in Figures 28-37, the downstream component that the air is deflected towards is the outer surface 8125 of the inner tube portion 8120 within the vent slot 7300. The deflectors 7400 are located at a longitudinal position in the vent slot 7300 closer to the vent inlet 7310 than the vent outlet 7320, for example approximately one-third along the length of the vent slots 7300. In other forms of the technology (not illustrated), the deflector may be configured to deflect the air flow towards the partitions 7200 forming the vent slots 7300 or the inner surface 8115 of the outer tube portion 8110.

In the form of the technology shown in Figures 8-37 the connection member 8000 comprises a step down region 8130 formed in a region of the tube portion 8100. In the step down region 8130 the diameter of the connection member 8000 decreases from one part of the connection member 8000 to a longitudinally adjacent part of the connection member 8000. In the form of technology shown in these figures, the distance between the central axis, A2, and both the inner surface 8115 of the outer tube portion 8110 and the outer surface 8125 of the inner tube portion 8120 decreases between longitudinally adjacent parts of the connection member 8000. For example, in the forms of the technology shown in Figures 16, 26, and 36 the vent structure 7000 is formed such that a radial distance, D1, of the outer surface 8125 of the inner tube portion 8120 at the vent inlet 7310 from the central axis A2 is greater than a radial distance, D2, of the outer surface 8125 of the inner tube portion 8120 from the central axis A2 at the vent outlet 7320. The distance, D2, may be varied to suit the diameter of the air circuit 4170. The distance, D1, may be in the range of 0.3-0.7mm greater than the distance D2. In some forms of the technology the distance D1 may be 0.5mm greater than the distance D2. The step down region 8130 may comprise the same region of the connection member 8000, or be comprised of the same components, as form the expanding height portion 7360 and the acoustic attenuation chamber 7370, as described above, and may contribute to forming these features.

The step down region 8130 may be formed by the outer surface 8125 of the inner tube portion 8120 comprising a radially inward step 8131 having an upstream end 8132 and a downstream end 8133, wherein the upstream end 8132 of the radially inwards step 8131 is at a greater radial distance from the central axis than the downstream end 8133 of the radially inward step 8131. In the forms of the technology shown in Figures 8-37 the radially inward step also contributes to forming an acoustic attenuation chamber 7370 (as described above) between the upstream end 8132 and the downstream end 8133. The radially inward step may also contribute to an offset between the inlet 7371 and the outlet 8372 of the acoustic attenuation chamber 7370 (as discussed above). In the forms of the technology shown in Figures 8-37 the radially inward step contributes to forming the expanding height portion 7360. As described above this is formed by a region of the outer surface 8125 of the inner tube portion 8120 sloped at a constant angle with respect to the central axis A2 and a region of the inner surface 8115 of the outer tube portion 8110 with a negative curvature.

In certain forms of the technology, such as shown in Figures 8-37, the step down region 8130 also comprises the deflector 7400 where the inner surface 8115 of the outer tube portion 8110 curves towards the central axis A2.

As noted above, in the form of the technology shown in Figures 28-37 the step down region 8130 is located where the width of the vent slots 7300 is smallest, i.e. at the bottleneck region 7350. In other forms of the technology, as shown in Figures 8-27, the step down 8130 may be located at another longitudinal position along the tube portion 8100, for example where the width of the vent slot 7300 is widest.

The step down region 8130 may form, or contribute to forming, a number of the sound reducing features already described, including any one or more of the expanding height portion 7360, the acoustic attenuation chamber 7370 and the deflector 7400.

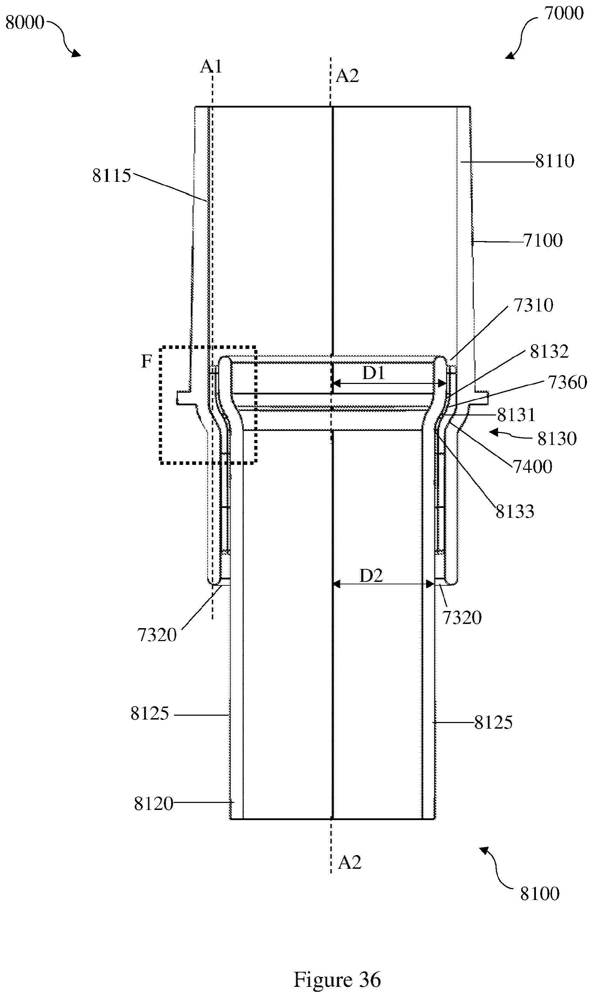

In the forms of the technology shown in Figures 26 and 36 the walls of the inner tube portion 8120 and the outer tube portion 8110 have substantially a constant thickness along the length of the connection member 8000. As a result, at the step down region 8130 there is both a decrease in diameter of the inner and outer surfaces of the outer tube portion 8110 and the inner tube portion 8120.

In the form of the technology shown in Figure 16, at the step down region 8130 there is a decrease in diameter of the inner tube portion 8120 but the diameter of the inner surface of the inner tube portion 8120 remains constant along the length of the connection member 8000. This may deter turbulence in the air passing through the inner tube portion 8120 from the air circuit 4170 to the patient interface 3000.

In other forms of the technology the outer surface of the outer tube portion 8110 may maintain a constant diameter along the length of the connection member 8000.

In other words, any bend or change in orientation of the vent slots 7300 may, in some forms of the technology, also be associated with corresponding bends or changes in direction of the outer and inner surfaces of the respective tube portions forming the vents slots 7300 while, in other forms of the technology, the bends or changes in orientation of the vent slots 7300 are internal features only and the outer surfaces of the respective tube portions have a constant diameter along their length, or have some other shape or configuration that is not sympathetic to the shape of the vent slots 7300. Generally speaking, thicker walls of components such as tube portions results in greater weight. A lighter tube portion 8100 will result in a lighter connection member 8000. A lighter connection member 8000 may be more comfortable for the patient 1000 and may be cheaper to manufacture if it has less material.

In some forms of the technology the deflectors 7400 result in the air flow exiting the vent flowing in adjacent the outer surface of the air circuit 4170 and in a direction that is along (i.e. parallel to) the outer surface of the air circuit 4170. This may either be because the deflectors are located adjacent the air circuit 4170 or because the outer surface of the connection member 8000 or inner tube portion 8120 is aligned with the outer surface of the air circuit 4170. The air circuit 4170 may be formed from a flexible tube that can bend during use. In some forms the Coanda effect may act to assist in ensuring that the air flow attaches to the air circuit even if the tube is bent. If there is a bend in the tube the air flowing along the outer curve of the bend will continue to follow the tube under the Coanda effect rather than continuing along a straight path in a direction away from the tube. This effect provides a number of advantages. It helps to avoid air flowing towards the patient 1000 or bed partner 1100 as the flow of air exiting the vent 3400 tends to stay attached to the outer surface of the air circuit 4170. The attachment of the flow to the outer surface of the air circuit 4170 where it is subject to friction from the air circuit 4170 also helps to further slow down the flow of air, reducing noise.

### 4.6.1.1.1.3 Projecting portion

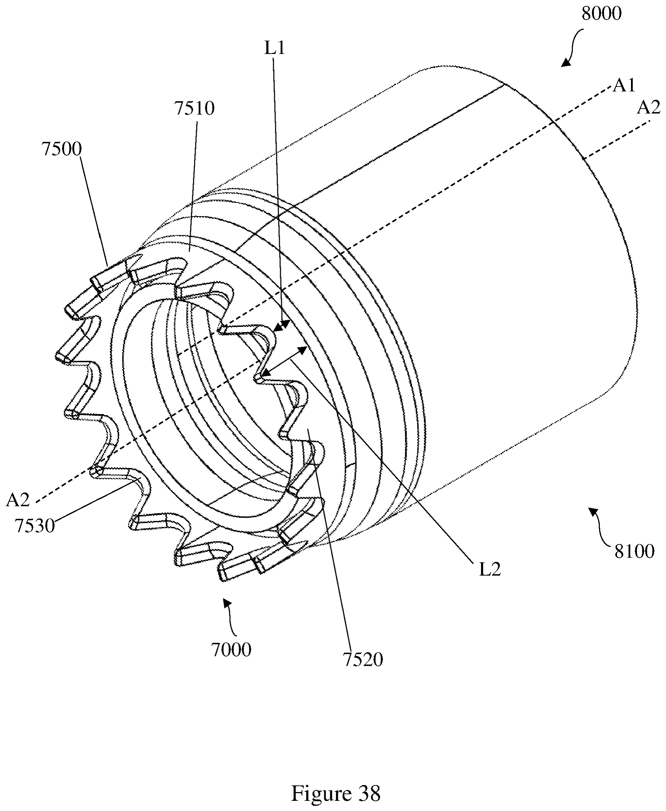

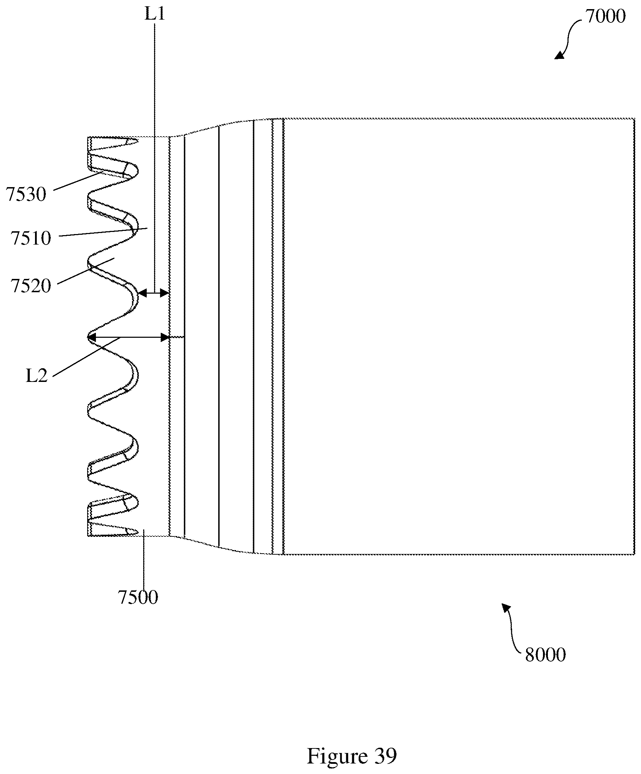

In one form of the technology shown in Figures 38 and 39, the vent structure 7000 further comprises a projecting portion 7500 structured and arranged to inhibit generation of shear layer instabilities in air flowing out of the vent outlet 7320. The projecting portion 7500 extends outwardly from the vent outlet 7320 so that the projecting portion 7500 presents a surface to the path of air flowing out of the vent outlet 7320. More particularly, the projecting portion 7500 extends outwardly from a part of the connection member that forms, or is adjacent to, the vent outlet 7320. As the flow of air exits the vent outlet 7320 the mixing between the warmer air exiting the vent and the colder ambient air causes shear layer instabilities, or turbulent mixing. The projecting portion 7500 assists in hindering air exiting the vent outlet 7320 from mixing directly with the ambient air. This reduces the shear layer instabilities and therefore the noise caused by air exiting the vent outlet 7320. The projecting portion 7500 may be more effective where the ambient air is cold and the difference in temperature between the air exiting the vent outlet 7320 and the ambient air is greater.

In the example shown in Figures 38 and 39 the projecting portion 7500 comprises an annular projection extending from the outer tube portion 8110, for example from the second end portion of the outer tube portion 8110 (the second end portion being opposite to the first end portion that connects to the patient interface 3000). In the example shown the projecting portion 7500 extends around the entire circumference of an end portion of the outer tube portion 8110. In some forms of the technology (not illustrated) there may be several projection portions 7500 which extend from different regions of the outer tube portion 8110, rather than a single annular projection.

In the forms of the technology shown in Figures 38 and 39 the projecting portion extends in a direction substantially parallel to the longitudinal axis, A1, of an adjacent vent slot 7300 (not shown). In these forms, the projecting portion 7500 comprises one or more first portions 7510 extending at a first length, L1, from the vent outlet 7320 and one or more second portions 7520 extending at a second length, L2, from the vent outlet 7320, the first length, L1, being less than the second length, L2. For example, as is the case with the form of projecting portion 7500 illustrated, a distal end of the projecting portion 7500 comprises a wave-shaped profile when viewed from a direction perpendicular to the plane in which the projecting portion 7500 extends from the vent outlet 7320. In certain forms, the wave-shaped profile is a sinusoidal wave which is particularly effective for reducing turbulent mixing of cold ambient air and the warmer air exiting the vents to reduce shear layer instabilities.

In the form of the technology shown in Figures 38 and 39, the projecting portion 7500 also comprises a negatively curved edge 7530 adjacent the exiting air flow. The negatively curved edge 7530 is formed at the distal end of the projecting portion 7500 on its inner edge, i.e. between the side of the projecting portion 7500 facing radially inwards towards the flow of air exiting the vent outlet 7320 and the end face of the projecting portion 7500. This creates a surface adjacent the air flow that is curved or rounded, e.g. in the form of a fillet edge. The negatively curved edge 7530 may act to further reduce shear layer instabilities. The negatively curved edge 7530 may reduce the flow separation of the warmer air exiting the vent that expands or flows in a direction perpendicular to the axis A2, thereby preventing mixing of the warmer air with the colder ambient air.

In other forms of the technology, the shape of the projecting portion 7500, including the size and shape of the wave-shaped profile, may be different. In some examples the wave shapes may be shallower or deeper, i.e. the difference between the first length, L1, and the second length, L2, may be greater or less than that shown in Figures 38 and 39. In other examples, the profile may be formed from a shape that instead of being wave-shaped is a series of triangular, square, or rectangular shapes formed by the projecting portion 7500 extending various lengths from the outer tube portion 8110.

### 4.6.1.1.1.4 Noise reduction testing

Testing of the form of the technology shown in Figures 8-17 has shown that the illustrated vent structure results in air flow slowing down to a speed of 6m/s within a distance of 55mm downstream of the vent outlet 7320 when used with a respiratory system with a patient undergoing CPAP therapy under typical conditions. The illustrated design also demonstrated a decay in acoustic power which decreases to 1e-12 W (the lowest sound level a person with good hearing can discern) within 80mm downstream of the vent outlet 7320.

### 4.6.1.1.1.5 Alternative forms of providing the vent structure to the connection member

The forms of the technology illustrated in Figures 8-39 are all connection members having an overall cylindrical symmetry. In other forms of the technology (not illustrated) the connection member 8000 and the vent structure 7000 may have a different configuration to that shown in the figures. In some examples, the tube portion 8100 of the connection member 8000 may be formed as a single hollow cylindrical shape and the vent structure 7000 may be provided to a region of the outer surface of the tube portion 8100 where that region does not span the entire outer circumference of the tube portion 8100. That is, the vent structure 7000 may, in some forms, be provided to one side of the tube portion 8100. In such examples, the plurality of partitions 7200 may be arranged generally in a part-cylindrical geometry or they may be arranged in a planar geometry. In such forms, the vent housing 7100 may be partly formed by the outer surface of the tube portion 8100 and may comprise a vent covering to enclose the plurality of partitions 7200 that form the vent slots 7300.

In some examples the axis A1 of the vent slots 7300 may not be parallel to the longitudinal central axis A2 of the connection member 8000. For example, the vent slots 7300 may be oriented on the tube portion 8100 to direct the flow of air exiting the vent outlet 7320 in a certain direction that, during typical use of the connection member 8000, may be likely to be away from the patient 1000 or bed partner 1100.

### 4.6.1.1.1.6 Patient interface

The preceding sections describe forms of the technology where the vent 3400 in the respiratory system is formed in, or as part of, a connection member 8000 connecting the air circuit 4170 to the patient interface 3000. In other forms of the technology, the vent 3400 may be comprised as part of another part of the respiratory system. When comprised in other parts of the respiratory system, the vent 3400 may include many of the same, or similar features to those described above. While the following paragraphs describe some features of exemplary vents 3400 formed as part of a patient interface 3000 and a positioning and stabilising structure 3300, it will be understood that any one or more of the features of a vent 3400 described above in the context of a connection member 8000 may be part of a vent 3400 forming part of a patient interface 3000 or a positioning and stabilising structure 3300, where suitable.

In some forms of the technology, a patient interface 3000 comprising a plenum chamber 3200 and a seal-forming structure 3100 as described above may further comprise a vent structure 7000. The vent structure 7000 allows a continuous flow of gases exhaled by the patient 1000 from an interior of the plenum chamber to ambient. The vent structure 7000 is sized and shaped to maintain the therapeutic pressure in the plenum chamber 3200 in use. The vent structure 7000 may comprise a vent housing 7100 and a plurality of partitions 7200 forming a plurality of vent slots 7300. In these forms, the vent structure 7000 may be structured and arranged in a form that is specifically adapted to the geometry of the patient interface 3000. The vent structure 7000 may comprise at least one of the noise reduction features, i.e. the increase in cross-sectional area, the deflector, or the projecting portion, as described above.

In some forms of the technology the vent structure 7000 is provided to a portion of the plenum chamber 3200, for example a portion that is on an anterior side of the plenum chamber 3200 when the patient interface 3000 is donned by a patient 1000. The vent structure 7000 may comprise a vent housing 7100 provided to the surface of the plenum chamber 3200 and vent slots 7300 that are arranged side-by-side and adopt a similar geometry to the surface of the plenum chamber 3200 proximate the vent structure 7000. The outer surface of the plenum chamber 3200 may form part of the vent housing 7100. The vent housing 7100 and the vent slots 7300 may be configured to follow the general shape, i.e. curvature, of the plenum chamber 3200. In some forms of the technology the vent structure 7000 may be provided to a central region of the plenum chamber 3200 and in alternative embodiments it may be located in a peripheral region of the plenum chamber 3200.

It has previously been described that the vent structure 7000 may comprise a plurality of deflectors 7400 configured to deflect the flow of air through each vent slot 7300 towards a part of the vent housing 7100 and/or another component in the respiratory system. In forms of the technology where the vent structure 7000 forms part of the patient interface 3000, the downstream component that the air flow is deflected towards may be part of the plenum chamber 3200, part of the air circuit 4170 or part of a component connecting the air circuit 4170 to a connection port 3600 in the patient interface 3000, for example an elbow.

### 4.6.1.1.1.7 Conduit Headgear

In some forms of the technology, a positioning and stabilising structure 3300 is configured to provide a force to hold a seal-forming structure 3100 in a therapeutically effective position on a patient's head in use. The positioning and stabilising structure 3300 comprises at least one gas delivery tube being constructed and arranged to contact at least a region of the patient's head superior to an otobasion superior of the patient's head. An end of the at least one gas delivery tube is configured to fluidly connect to a plenum chamber 3200. The positioning and stabilising structure 3300 further comprises a connection port to receive a flow of air from an air circuit 4170 and to deliver the flow of air to the entrance of the patient's airways via the seal-forming structure 3100, wherein the connection port is provided to a portion of the gas delivery tube superior to the otobasion superior of the patient's head. The positioning and stabilising structure 3300 further comprises a vent structure 7000 comprising a vent housing 7100 and a plurality of partitions 7200 forming a plurality of vent slots 7300. The vent structure 7000 preferably comprises at least one of the noise reduction features described above, i.e. the increase in cross-sectional area, the deflector, or the projecting portion, adapted into a form that is suitable within the overall arrangement and geometry of a vent structure in a positioning and stabilising structure 3300.

In certain forms, the vent structure 7000 may be comprised as part of a gas delivery tube in a positioning and stabilising structure 3300 and have a similar configuration to the connection member 8000 described above. In such forms, the outer tube portion 8110 and the inner tube portion 8120 may be configured to connect to upstream and downstream portions of the gas delivery tube respectively. In some forms of the technology the vent slots 7300 may be provided to a side of the connection member 8000 that faces away from the patient's face in use and a side of the connection member 8000 facing towards the patient's face in use has no vent slots 7300. In alternative forms of the technology, the vent structure 7000 may be provided to a region of the outer surface of the gas delivery tube.

The vent outlet 7320 may have a corresponding outlet in the positioning and stabilising structure 3300 to allow the flow of air to exit into the surrounding ambient air. In some forms of the technology, the outlet in the positioning and stabilising structure may be a hole or a plurality of holes in the region where the vent outlet 7320 is located. In other forms of the technology, the outlet in the positioning and stabilising structure may be a region with a reduced thickness, for example with only a thin layer of material, where the vent outlet 7320 is located.

In some forms of the technology the vent structure 7000 may be provided to a portion of the gas delivery tube that is positioned over the cheek region of the patient's face in use. This may reduce the amount of deadspace in the system by reducing the distance between the vent structure 7000 and the patient's mouth/nose where the air is exhaled compared to forms of the technology in which the vent structure 7000 is provided to a portion of the gas delivery tube further from the patient's mouth/nose, for example a portion positioned superior to the otobasion superior in use.

It has previously been described that the vent structure 7000 may comprise a plurality of deflectors 7400 configured to deflect the flow of air through each vent slot 7300 towards a part of the vent housing 7100 and/or another component in the respiratory system. In forms of the technology where the vent structure 7000 forms part of a positioning and stabilising structure 3300, as described above, the downstream component that the air flow is deflected towards may be another part of the positioning and stabilising structure 3300, for example a part of the gas delivery tube that is positioned in use superior to the vent structure 7000.

### 4.6.1.1.1.8 Split Venting

In some forms of the technology in addition to the vent 3400 constructed and arranged to allow for the washout of exhaled gases there may be a further second vent (not illustrated) configured to vent air received from the RPT device 4000 before the air is received by the patient 1000. The second vent may be configured and arranged to reduce the flow rate of air from the RPT device 4000 that is received by the patient 1000 whilst maintaining the pressure of the flow of air to the patient 1000. The second vent may be at a different location in the respiratory therapy system to vent 3400.

In some forms of the technology the second vent may be located along the air circuit 4170. In forms in which both the vent 3400 and the second vent are located along the air circuit 4170, the second vent may be provided to the air circuit 4170 at a different location to vent 3400, for example the second vent may be located on the air circuit 4170 at a distance closer to the RPT device 4000 than vent 3400.

In some forms of the technology the second vent may be in the form of a vent structure 7000 according to any of the forms of technology as described above. In some forms of the technology the vent structure may be in the form of a connection member 8000 as described above. The connection member 8000 may be provided to the air circuit 4170 such that the connection member 8000 is connected at respective ends to upstream and downstream sections of the air circuit 4170. The air circuit 4170 and the connection member 8000 may be connected in a similar manner as described above.

### 4.6.1.2 Anti-Asphyxia Valve (AAV)

In one form of the technology, the connection member 6000 comprises an anti-asphyxia valve (AAV) 6200 to provide a flow of air to the patient 1000 from the ambient air when the blower 4142 is not working. An example of a connection member 6000 with an AAV 6200 is shown in Figures 7A-7I and is described in the following paragraphs.

The connection member 6000 comprises an AAV 6200 comprising an opening 6210 to the ambient air and a closure 6220 that is configured to move between a first position in which the closure 6220 covers the opening 6210 and a second position in which the opening 6210 is uncovered. The AAV 6200 is configured so that, when a flow of pressurised gas is being supplied to the patient interface 3000 from the air circuit 4170, the closure 6220 is in the first position. For example, the flow of pressurised gas may exert a force on the closure 6220, moving it into the first position. The AAV 6200 is also configured so that, when there is no pressurised flow of gas being supplied to the patient interface 3000, the closure 6220 is in the second position. For example, the flow of gas caused by the patient's breathing may exert a force on the closure 6220, moving it into the second position. In the form of the technology shown in Figures 7A-7I, the closure 6220 is shown in the second position in which the blower 4142 is turned off or not functioning and is therefore not providing a supply of pressurised air. The opening 6210 is uncovered to allow the patient 1000 to breathe through the opening 6210.

In the form of the technology shown in Figures 7A-7I, the AAV 6200 is located proximate the patient-proximal end 6111 of the first tube portion 6110. The arrangement of the AAV 6200 and the vent structure 6100 and some advantages of this location of the AAV 6200 are described in section 4.6.3.

In the form of the technology shown in Figures 7A-7I, the closure 6220 comprises a hingedly mounted flap 6221. The flap 6221 is mounted to the first tube portion 6110 although in other forms of the technology it may be mounted to another part of the connection member 6000, for example the second tube portion 6120. The hingedly mounted flap 6221 comprises a base portion 6222 configured to fit into a first aperture 6117 in the first tube portion 6110. The base portion 6222 is configured to fit into the first aperture 6117 via a snap-fit connection. In other forms of the technology, the base portion 6222 may be mounted to the first tube portion 6110 by an alternative means, for instance an adhesive or friction fit connection.

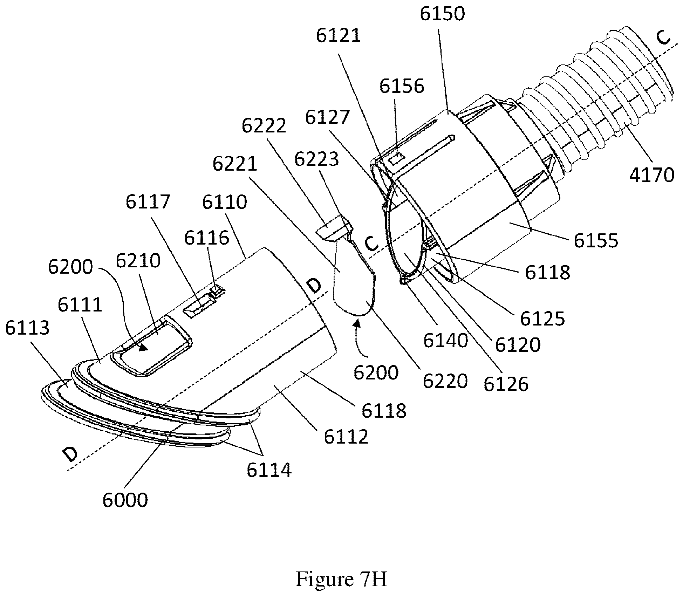

In the form of the technology shown in Figures 7A-7I the base portion 6222 comprises a hinge portion 6223, as shown in Figure 7H. This hinge portion 6223 is formed from a portion of the closure 6220 between the base portion 6222 and the flap 6221 that is relatively thin (e.g. a living hinge) and therefore enables the closure 6220 to bend such that the hingedly mounted flap 6221 moves between the first position and the second position. In other forms of the technology, the hinge may be formed by other means, for example a rotatable pin connection.

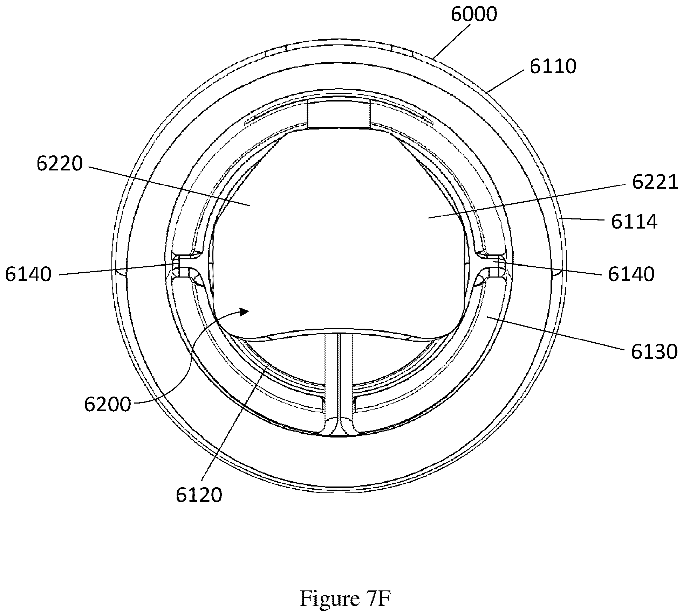

In the form of the technology shown in Figures 7A-7I, the hingedly mounted flap 6221 is relatively planar and may have a curved surface. The curved surface may be complementary to the inner surface 6115 of the first tube portion 6110 to help the flap 6221 abut against the inner surface 6115, which in the form shown in Figures 7A-7I is cylindrical in shape. The hingedly mounted flap 6221 therefore aligns with and covers the entire opening 6210 when in the first position. The shape of the plane surface of the hingedly mounted flap may be of an irregular hexagon with rounded corners as shown in Figure 7F. In other forms of the technology, the flap may have an alternative shape, for instance circular, square, or rectangular. The planar surface may be sized and shaped to substantially cover the opening 6127 of the second tube portion 6120.

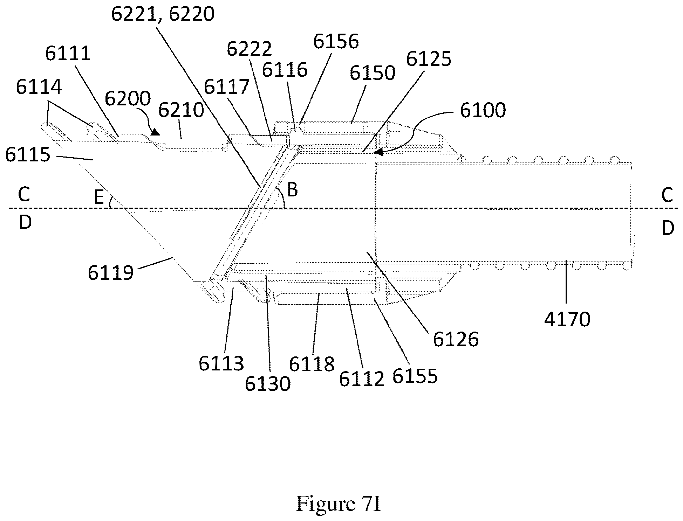

When the closure 6220 is in the second position the flap 6221 substantially covers the patient-proximal end 6121 of the second tube portion 6120 thereby preventing flow of gas between the first tube portion 6110 and the second tube portion 6120 during use. This assists in directing the flow of exhaled air from the patient 1000 through the opening 6210 and provides an air flow path for the patient 1000 to breathe the surrounding ambient air through the opening 6210, preventing the patient rebreathing exhaled CO₂. In the form of the technology shown in Figures 7A-7I the hingedly mounted flap 6221 contacts and is seated on the patient-proximal end 6121 of the second tube portion 6120 when the closure 6220 is in the second position. In the second position the flap 6221 at least partially covers the opening at the patient-proximal end 6121 of the second tube portion 6120. The patient-proximal end 6121 of the second tube portion 6120 may be disposed at an angle, indicated as B in Figure 7I, to the longitudinal central axis C-C of the second tube portion 6120. When the hingedly mounted flap 6221 is seated on the patient-proximal end 6121 of the second tube portion 6120, the hingedly mounted flap 6221 is orientated at substantially the angle B to the longitudinal central axis C-C, the angle B being substantially non-perpendicular.

In use, when the blower 4142 is supplying a flow of pressurised air, the supply of pressurised air through the second tube portion 6120 pushes the hingedly mounted flap 6221 from the second position into the first position where it covers the opening 6210. When the blower 4142 does not supply a flow of pressurised air, the patient's breathing draws air through opening 6210 from ambient, pushing the hingedly mounted flap 6221 against the patient-proximal end 6121 of the second tube portion 6120. In some forms of the technology, the hingedly mounted flap 6221 may be biased to the second position.

In use, the opening 6210 of the AAV 6200 is positioned on a side of the connection member 6000 facing generally away from the patient, e.g. in an anterior direction. By facing away from the patient 1000, the flow of air through the opening 6210 when in the second position will be less likely to disturb the patient 1000 than if the opening 6210 was on a side of the connection member 6000 facing towards the patient.

### 4.6.1.3 Connection to the patient interface

The first tube portion 6110 has a patient-proximal end 6111 configured to connect to the patient interface 3000, for example to the connection port 3600. In some forms of the technology, this is a removable connection such that the patient interface 3000 and the connection member 6000 can be disconnected from each other, for instance to assist in cleaning or replacing parts.

In the form of the technology shown in Figures 7A-7I, the patient proximal end 6111 of the first tube portion 6210 has two ridges 6114 provided to the outer surface 6118 of the first tube portion 6110. The ridges 6114 are formed around the perimeter of the first tube portion 6110 and a groove 6113 is formed between them. The groove 6113 is configured to receive the connection port 3600 of the patient interface 3000. In some forms the plenum chamber 3200 comprises a flexible material and the connection port 3600 is an opening in this flexible material. The flexible material around the connection port 3600 can then be fitted into groove 6113. In other forms of the technology, the connection port 3600 may connect to the patient-proximal end 6111 via a different mechanism, for instance: a snap-fit connection; a latch mechanism; or one or more protrusions configured to fit into corresponding apertures in the plenum chamber 3200. In other forms of the technology, the connection port 3600 may connect to the inside of the first tube portion 6110.

In the form of the technology shown in Figures 7A-7I, a plane of the patient-proximal end 6111 of the first tube portion 6110 is arranged at a substantially non-perpendicular angle E, shown in Figure 7I, to the first longitudinal central axis D-D. The angle E may be approximately 45° in some forms of the technology. As the connection member 6000 is substantially straight as described in section 4.6.2, when the connection member 6000 is connected to the patient interface 3000 in use, the connection member 6000 is consequently generally directed in an anterior-inferior direction away from the patient interface 3000 rather than in a directly anterior direction which would be more obtrusive and obstructive for the patient.

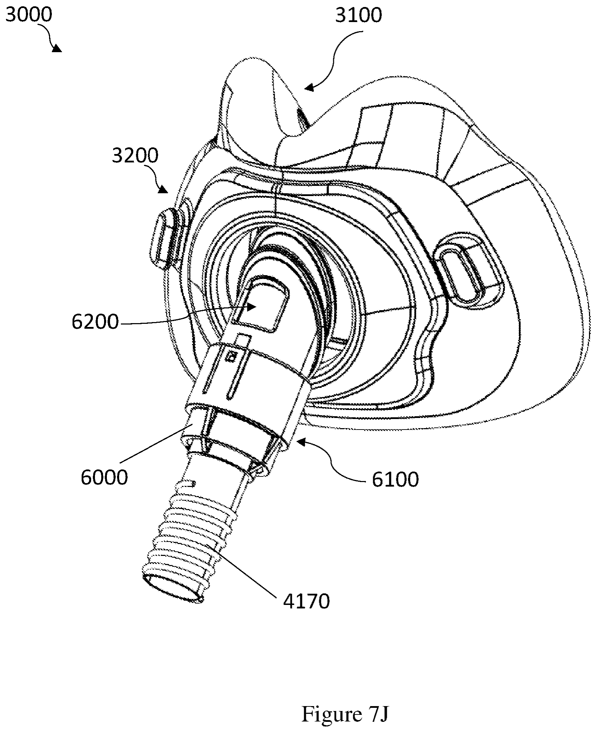

Figure 7J shows the connection member 6000 connected to the patient interface 3000, in particular the patient-proximal end 6111 of the first tube portion 6110 is connected to the plenum chamber 3200 of the patient interface 3000. For example, the patient-proximal end 6111 of the first tube portion 6110 may be connected to an anterior portion of the plenum chamber 3200 of the patient interface 3000, for example a central anterior portion of the plenum chamber 3200 (i.e. a part of the plenum chamber 3200 that intersects the mid-sagittal plane of the patient in use).

The connection member 6000 may be configured to be relatively short and may be as short as possible to minimise the amount of bulk and stiffness added to the patient interface by the connection member 6000. For example, in some forms, the connection member 6000 may have a length of between 35mm and 50mm, for example in some forms the length of the connection member 6000 may be approximately 40mm. In other forms the connection member 6000 may have a different length.

In some forms of the technology a short, compact connection member 6000 connected to a plenum chamber 3200 that is at least partly formed of a flexible material may mean that the patient interface assembly is configured to decouple forces between the air circuit 4170 and the patient interface 3000 sufficiently without other decoupling mechanisms being used, for example a rotatable elbow connecting the air circuit 4170 to the plenum chamber 3200. The connection member 6000 may be able to rotate to a sufficient extent, for example through angles up to approximately 10° from the normal, due to the flexibility and compliance in the plenum chamber 3200 to adequately decouple most forces likely to be exerted on the air circuit 4170 during use.

### 4.6.1.4 Connection to the air circuit

The patient-distal end 6122 of the second tube portion 6120 is configured to connect to the air circuit 4170. In some forms of the technology, this may be a removable connection such that the air circuit 4170 and the connection member 6000 can be disconnected. Examples of suitable removable connections include a screw- and-thread connection or a snap-fit connection. In other forms this may be a fixed or permanent connection for instance via an adhesive. In the form of the technology shown in Figures 7A-7I the air circuit 4170 is fitted to the inner surface 6126 of the patient-distal end 6122 of the second tube portion 6120.

### 4.6.1.5 Heat-moisture exchanger

In some forms of the technology, the connection member 6000 may also include a passive heat-moisture exchanger that may absorb heat and moisture from gas exhaled by the patient. The incoming flow of breathable gas to be supplied to the patient's airways may be heated and humidified by the heat and moisture held in the heat-moisture exchanger. An example of a heat-moisture exchanger is disclosed in Australian Patent Application No. 2014295910 which is incorporated here within in its entirety by reference.

In some forms of the technology, a heat-moisture exchanger may be positioned on a region of the connection member 6000 that is close to the patient 1000 in use, for instance adjacent the patient-proximal end 6111 of the first tube portion 6110. In other forms of the technology, a heat-moisture exchanger may be positioned within the connection member 6000 between the AAV 6200 and the vent structure 6100. This may increase the length of the connection member by approximately the length of the heat-moisture exchanger, which may be approximately at least 20mm in some forms.

### 4.6.2 Straight connection member

In some forms of the technology, the connection member 6000 is substantially straight. The connection member 6000 may have a substantially straight central longitudinal axis.

In one form of the technology the first tube portion 6110 has a first longitudinal central axis D-D that is substantially straight between the patient-proximal end 6111 and the patient-distal end 6112. The second tube portion 6120 has a second longitudinal central axis C-C that is substantially straight between the patient-proximal end 6121 and the patient-distal end 6122. The first tube portion 6110 and the second tube portion 6120 are arranged with the first longitudinal central axis D-D of the first tube portion 6110 substantially parallel to the second longitudinal central axis C-C of the second tube portion 6120. In some forms of the technology the first longitudinal central axis D-D and the second longitudinal central axis C-C are axially aligned. In such forms, the first tube portion 6110 and the second tube portion 6120 are arranged concentrically with regard their circular cross-sections. Similarly, the third tube portion 6155 may also be arranged concentrically with the first tube portion 6110 and the second tube portion 6120. In other forms, the first tube portion 6110 and the second tube portion 6120 may be arranged with the first longitudinal central axis D-D substantially parallel to, but offset from, the second longitudinal central axis C-C.

The first tube portion 6110 and the second tube portion 6120 may be arranged with their respective longitudinal central axes parallel or close-to-parallel. For example, in some forms of the technology the first tube portion 6110 and the second tube portion 6120 may be arranged so that the first longitudinal central axis D-D, when extended beyond the ends of the first tube portion 6110, passes through the opening 6127 at the patient-distal end 6122 of the second tube portion 6120, and the second longitudinal central axis C-C, when extended beyond the ends of the second tube portion 6110, passes through the opening at the patient-proximal end 6111 of the first tube portion 6110.

Having a substantially straight connection member 6000 may make the patient interface assembly less bulky and obtrusive to the patient 1000 compared to a patient interface assembly that includes an elbow. As the mask is less bulky and obtrusive to the patient 1000 it may also make the mask easier, more comfortable and less claustrophobic for the patient to wear. A substantially straight connection member may also be easier to manufacture and therefore cheaper to produce than an elbow.

### 4.6.3 Arrangement of the vent structure and the AAV

In some forms of the technology the AAV 6200 is located in a position on the connection member 6000 closer to the patient interface 3000 than the vent structure 6100 is to the patient interface 3000 when in use. Equivalently, the AAV 6200 is closer to the patient-proximal end 6111 of the first tube portion 6110 than the vent structure 6100 is to the patient-proximal end 6111 of the first tube portion 6110. That is, the AAV 6100 is located upstream, when considering the direction of the flow of air as the patient exhales, of the vent structure 6100.

An advantage of providing the AAV 6200 relatively close to the patient's airways is that this arrangement reduces the amount of exhaled air that is re-breathed by the patient 1000 compared to if the AAV 6200 is further from the patient's airways. In some jurisdictions, the patient interface assembly must pass a re-breath test, which requires that only a certain amount of exhaled air can be re-breathed by the patient 1000. By providing the AAV 6200 relatively close to the patient's airways, for example closer to the patient interface 3000 than the vent structure 6100 in the form of connection member 6000 shown in the figures, the volume between the patient's airways and the AAV 6200 is reduced. This may help patient interface assemblies comprising the connection member 6000 pass such re-breath tests.

The connection member 6000 may be configured for use with a patient interface 3000 having a relatively small plenum chamber 3200. Such a patient interface assembly has a relatively small functional dead space. Functional dead space may be defined as the volume of exhaled air that is trapped within the system and that the patient subsequently re-breathes. It is desirable that functional dead space is kept within a safe range, or in the most extreme cases this could lead to patient suffocation. In circumstances where the functional dead space is small, the vent structure 6100 can be located further from the patient's airways while still venting the necessary amount of exhaled air compared to if the connection member 6000 is used with a patient interface 3000 having a larger plenum chamber 3200. Therefore, the connection member 6000 according to the forms of the technology described herein is suitable for use with a patient interface 3000 having a relatively small plenum chamber 3200 because the vent structure 6100 is located at a position on the connection member 6000 further from the patient interface 3000 than the AAV 6200 in use.

Another advantage of the AAV 6200 being positioned relatively close to the patient interface 3000 (e.g. proximal the patient-proximal end 6111 of the first tube portion 6110) is that a relatively small opening 6210 still enables sufficient exhaled air to vent from the patient interface assembly when the AAV 6200 is open for the patient interface assembly to pass a re-breath test. An opening 6210 of an AAV 6200 generally needs to be larger the further the AAV 6200 is located in use from the patient's airways to be able to vent a sufficient amount of exhaled air. The smaller the opening 6210, the smaller the connection member 6000 may be made overall. Additionally, having a smaller opening 6210 makes the AAV 6200 less visually obtrusive to the overall design of the connection member 6000 and the patient interface assembly as a whole. In certain forms of the technology the area of the opening 6210 of the AAV 6200 may be between 70mm² and 90mm² and in some forms may be approximately 80mm². In other forms the opening 6210 may have a different area.

A further advantage of locating the AAV 6200 closer to the patient in use than the vent structure 6100 is that the flow of air exiting the vent structure 6100 may be able to remain attached to the air circuit 4170 (through the Coanda effect as described earlier) without any interruption to the flow of air that may be caused by passing any components forming the AAV 6200. Any disruption to the flow of air may deflect the flow of air towards the patient 1000 or bed partner 1100 causing discomfort. The flow of air remaining attached to the air circuit 4170 also reduces the speed and turbulence of the flow of air, which reduces the noise of the vent in use.

### 4.7 HUMIDIFIER

### 4.7.1 Humidifier overview

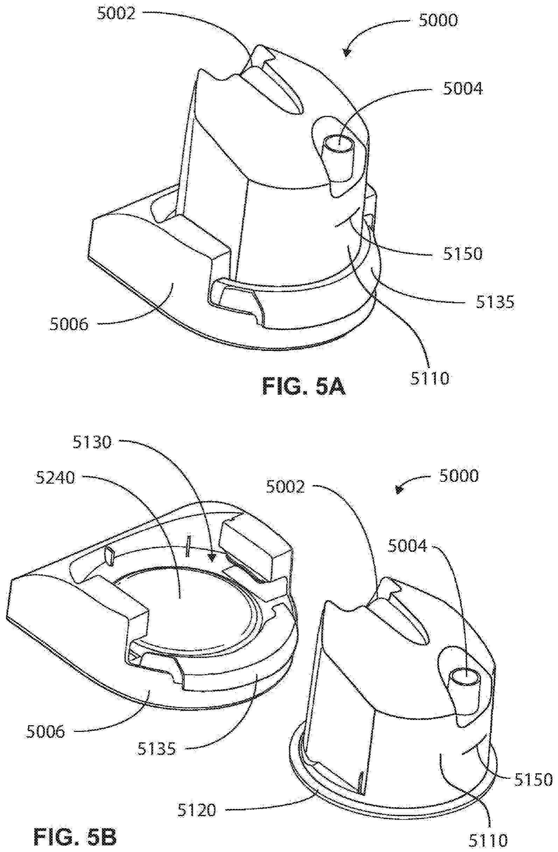

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 4.8 BREATHING WAVEFORMS

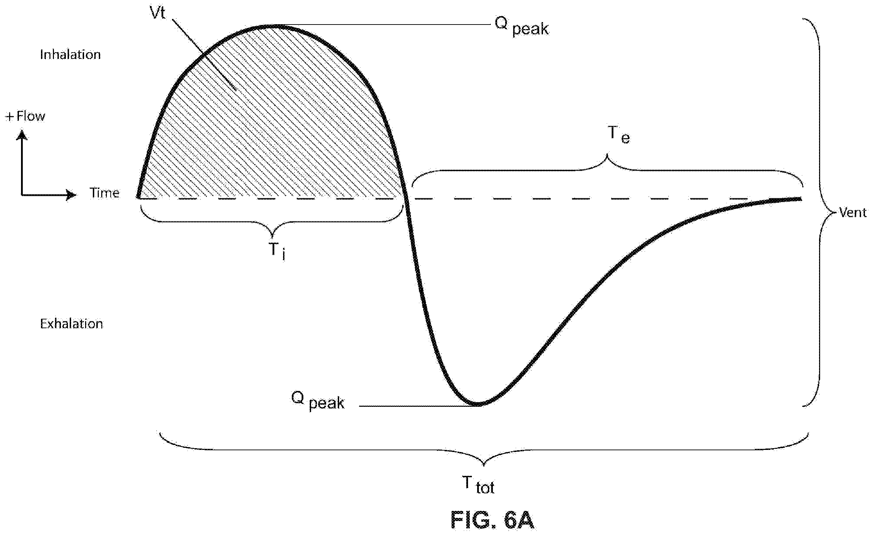

Fig. 6A shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak -0.5* L/s. The total duration of the breath, *Trot,* is about 4s. The person typically breathes at a rate of about 15 breaths per minute (BPM), with Ventilation *Vent* about 7.5 L/min. A typical duty cycle, the ratio of *Ti* to *Trot,* is about 40%.

### 4.9 RESPIRATORY THERAPY MODES

Various respiratory therapy modes may be implemented by the disclosed respiratory therapy system.

### 4.10 GLOSSARY

For the purposes of the present technology disclosure, in certain forms of the present technology, one or more of the following definitions may apply. In other forms of the present technology, alternative definitions may apply.

### 4.10.1 General

*Air:* In certain forms of the present technology, air may be taken to mean atmospheric air, and in other forms of the present technology air may be taken to mean some other combination of breathable gases, e.g. atmospheric air enriched with oxygen.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy (RPT):* The application of a supply of air to an entrance to the airways at a treatment pressure that is typically positive with respect to atmosphere.

*Ventilator:* A mechanical device that provides pressure support to a patient to perform some or all of the work of breathing.

### 4.10.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 4.10.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 4.10.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Trot.*

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea:* An increase in flow to a level higher than normal.

*Inspiratory portion of a breathing cycle:* The period from the start of inspiratory flow to the start of expiratory flow will be taken to be the inspiratory portion of a breathing cycle.

### 4.10.3 Anatomy

### 4.10.3.1 Anatomy of the face

### Ala: the external outer wall or "wing" of each nostril (plural: alar)

### Alar angle:

*Alare:* The most lateral point on the nasal *ala.*

*Alar curvature (or alar crest) point:* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

### Lip, lower (labrale inferius):

### Lip, upper (labrale superius):

*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold:* The skin fold or groove that runs from each side of the nose to the corners of the mouth, separating the cheeks from the upper lip.

*Naso-labial angle:* The angle between the columella and the upper lip, while intersecting subnasale.

*Otobasion inferior:* The lowest point of attachment of the auricle to the skin of the face.

*Otobasion superior:* The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare:* The point at the lower margin of the alar base, where the alar base joins with the skin of the superior (upper) lip.

*Subnasal point:* Located on the soft tissue, the point at which the columella merges with the upper lip in the midsagittal plane.

*Supramenton:* The point of greatest concavity in the midline of the lower lip between labrale inferius and soft tissue pogonion

### 4.10.3.2 Anatomy of the skull

*Frontal bone:* The frontal bone includes a large vertical portion, the *squama frontalis,* corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

### Orbit: The bony cavity in the skull to contain the eyeball.

*Parietal bones:* The parietal bones are the bones that, when joined together, form the roof and sides of the cranium.

*Temporal bones:* The temporal bones are situated on the bases and sides of the skull and support that part of the face known as the temple.

*Zygomatic bones:* The face includes two zygomatic bones, located in the upper and lateral parts of the face and forming the prominence of the cheek.

### 4.10.3.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 4.10.4 Patient interface

*Anti-asphyxia valve (AAV):* The component or sub-assembly of a mask system that, by opening to atmosphere in a failsafe manner, reduces the risk of excessive CO₂ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example, the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

### Tie (noun): A structure designed to resist tension.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 4.10.5 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example, a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 4.10.5.1 Curvature in one dimension

The curvature of a plane curve at p may be described as having a sign (e.g. positive, negative) and a magnitude (e.g. 1/radius of a circle that just touches the curve at *p*)*.*

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 4.10.5.2 Curvature of two-dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface and is analogous to a garden path).

*Path length:* In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 4.10.5.3 Space curves

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

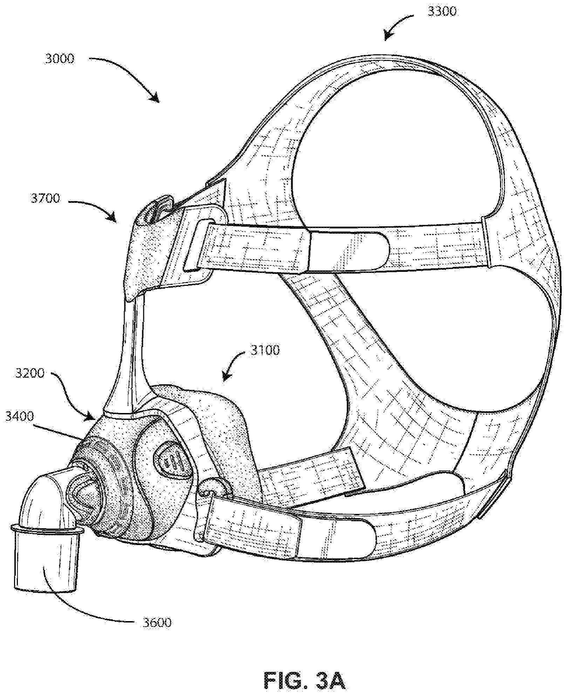

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 4.10.5.4 Holes

A surface may have a one-dimensional hole, e.g. a hole bounded by a plane curve or by a space curve. Thin structures (e.g. a membrane) with a hole, may be described as having a one-dimensional hole. See for example the one-dimensional hole in the surface of structure shown in Fig. 3I, bounded by a plane curve.

A structure may have a two-dimensional hole, e.g. a hole bounded by a surface. For example, an inflatable tyre has a two-dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two-dimensional hole indicated. In a yet another example, a conduit may comprise a one-dimension hole (e.g. at its entrance or at its exit), and a two-dimension hole bounded by the inside surface of the conduit. See also the two-dimensional hole through the structure shown in Fig. 3K, bounded by a surface as shown.

### 4.11 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein are incorporated herein by reference in their entirety to disclose and describe the methods and/or materials which are the subject of those publications. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the spirit and scope of the technology.

### 4.12 REFERENCE SIGNS LIST

- 1000: Patient
- 1100: Bed partner
- 3000: Patient interface
- 3100: Seal-forming structure
- 3200: Plenum chamber
- 3210: Chord
- 3220: Superior point
- 3230: Inferior point
- 3300: Positioning and stabilising structure
- 3400: Vent
- 3600: Connection port
- 3700: Forehead support
- 4000: RPT device
- 4010: External housing
- 4012: Upper portion
- 4014: Lower portion
- 4015: Panel(s)
- 4016: Chassis
- 4018: Handle
- 4020: Pneumatic block
- 4100: Pneumatic components
- 4110: Air filters
- 4112: Inlet air filter
- 4114: Outlet air filter
- 4120: Muffler
- 4122: Inlet muffler
- 4124: Outlet muffler
- 4140: Pressure generator
- 4142: Blower
- 4144: Brushless DC motor
- 4160: Anti-spill back valve
- 4170: Air circuit
- 4180: Supplemental oxygen
- 4200: Electrical components
- 4202: Printed Circuit Board Assembly (PCBA)
- 4210: Power Supply
- 4220: Input devices
- 4270: Transducers
- 5000: Humidifier
- 5002: Humidifier inlet
- 5004: Humidifier outlet
- 5006: Humidifier base
- 5110: Reservoir
- 5120: Conductive portion
- 5130: Humidifier reservoir dock
- 5135: Locking lever
- 5150: Water level indicator
- 5240: Heating element
- 6000: Connection member
- 6100: Vent structure
- 6110: First tube portion
- 6111: Patient-proximal end of the first tube portion
- 6112: Patient-distal end of the first tube portion
- 6113: Groove
- 6114: Ridges
- 6115: Inner surface of the first tube portion
- 6116: Connection portion
- 6117: First aperture
- 6118: Outer surface of the first tube portion
- 6119: Opening
- 6120: Second tube portion
- 6121: Patient-proximal end of the second tube portion
- 6122: Patient-distal end of the second tube portion
- 6125: Outer surface of the second tube portion
- 6126: Inner surface of the second tube portion
- 6127: Opening
- 6130: Vent slots
- 6140: Ribs
- 6150: Tube connector
- 6155: Third tube portion
- 6156: Second aperture
- 6200: Anti-asphyxia valve (AAV)
- 6210: Opening
- 6220: Closure
- 6221: Flap
- 6222: Base portion
- 6223: Hinge portion
- 7000: Vent structure
- 7100: Vent housing
- 7200: Partitions
- 7210: Angled straight wall portion
- 7220: Parallel straight wall portion
- 7230: Negatively curved portion
- 7240: Positively curved portion
- 7300: Vent slots
- 7310: Vent inlet
- 7320: Vent outlet
- 7330: First region
- 7335: Third region
- 7340: Second region
- 7345: Fourth region
- 7350: Bottleneck region
- 7360: Expanding height portion
- 7370: Chamber
- 7371: Inlet
- 7372: Outlet
- 7400: Deflector
- 7500: Projecting portion
- 7510: First portion
- 7520: Second portion
- 7530: Negatively curved edge
- 8000: Connection member
- 8100: Tube portion
- 8110: Outer tube portion
- 8115: Inner surface
- 8120: Inner tube portion
- 8125: Outer surface
- 8130: Step down region
- 8131: Radially inwards step
- 8132: Upstream end
- 8133: Downstream end
- 8200: Sealing member
- 8210: Apertures
Further preferred embodiments are decribed by the following items:
1. A connection member configured to connect an air circuit to a patient interface to convey a flow of pressurised breathable gas from the air circuit to the patient interface for breathing by a patient, the connection member comprising:
   a first tube portion having a patient-proximal end configured to connect to the patient interface and having a patient-distal end, the first tube portion having a first longitudinal central axis that is substantially straight between the patient-proximal end and the patient-distal end;
   a second tube portion having a patient-proximal end and a patient-distal end configured to connect to the air circuit, the second tube portion having a second longitudinal central axis that is substantially straight between the patient-proximal end and the patient-distal end, wherein the first tube portion and the second tube portion are arranged with the first longitudinal central axis of the first tube portion substantially parallel to the second longitudinal central axis of the second tube portion and at least a portion of the patient-proximal end of the second tube portion positioned inside at least a portion of the patient-distal end of the first tube portion, the flow of breathable gas being conveyed from the air circuit to the patient interface through the first tube portion and the second tube portion;
   a vent for venting gas exhaled by the patient to the ambient air, the vent formed between the first tube portion and the second tube portion; and
   an anti-asphyxia valve (AAV) comprising an opening to the ambient air and a closure configured to move between a first position in which the closure covers the opening and a second position in which the opening is uncovered.
2. The connection member of item 1 wherein the AAV is located in a position on the connection member closer to the patient-proximal end of the first tube portion than the vent.
3. The connection member of item 2 wherein the AAV is located proximate the patient-proximal end of the first tube portion.
4. The connection member of any one of items 2 to 3 wherein the vent is located proximate the patient-distal end of the first tube portion.
5. The connection member of any one of items 1 to 4 wherein the closure comprises a hingedly mounted flap.
6. The connection member of item 5 wherein the flap is mounted to the first tube portion.
7. The connection member of item 6 wherein the flap comprises a base portion configured to fit into a first aperture in the first tube portion.
8. The connection member of item 7 wherein the base portion is configured to fit into the first aperture via a snap-fit connection.
9. The connection member of any one of items 1 to 8 wherein the opening is positioned to face an anterior direction when in use.
10. The connection of member of any one of items 1 to 9 further comprising a tube connector configured to connect the second tube portion to an outer surface of the first tube portion.
11. The connection member of item 10, wherein the tube connector comprises a third tube portion mounted to an outer surface of the second tube portion, wherein the third tube portion surrounds the patient-distal end of the first tube portion.
12. The connection member of item 11 wherein the first tube portion comprises a connecting portion configured to fit into a second aperture in the third tube portion.
13. The connection member of item 12 wherein the third tube portion comprises a connecting portion configured to fit into a second aperture in the first tube portion.
14. The connection member of any one of items 12 to 13 wherein the connecting portion fits into the second aperture via a snap-fit connection.
15. The connection member of any one of items 1 to 14, wherein the vent comprises a plurality of vent slots arranged in a region between the first tube portion and the second tube portion.
16. The connection member of item 15, wherein the plurality of vent slots are arranged around the second tube portion.
17. The connection member of any one of items 15 to 16 wherein the vent slots are formed between a plurality of ribs extending between an outer surface of the second tube portion and an inner surface of the first tube portion.
18. The connection member of any one of items 15 to 17 wherein the vent comprises four vent slots.
19. The connection member of any one of items 15 to 18 wherein the plurality of vent slots are equally spaced apart.
20. The connection member of any one of items 1 to 19 wherein a plane of the patient-proximal end of the first tube portion is arranged at a substantially non-perpendicular angle to the first longitudinal central axis.
21. The connection member of item 20 wherein the plane of the patient-proximal end of the first tube portion is angled with respect to the first longitudinal central axis so that, in use, the connection member is directed in an anterior-inferior direction.
22. The connection member of any one of items 1 to 21 wherein the first tube portion is cylindrical in shape.
23. The connection member of any one of items 1 to 22 wherein the second tube portion is cylindrical in shape.
24. The connection member of item 23, when dependent on items 22 and 15, wherein the vent slots are arranged around an annular region between the first and second tube portions.
25. The connection member of any one of items 1 to 24 wherein the first longitudinal central axis and the second longitudinal central axis are axially aligned.
26. The connection member of any one of items 1 to 25 wherein, when the closure is in the second position, the closure substantially covers the patient-proximal end of the second tube portion thereby preventing flow of gas between the first tube portion and the second tube portion during use.
27. The connection member of item 26, when dependent on item 6, wherein in the second position the hingedly mounted flap contacts the patient-proximal end of the second tube portion.
28. The connection member of item 27 wherein the patient-proximal end of the second tube portion is at a second tube angle to the second longitudinal central axis and, when the hingedly mounted flap contacts the patient-proximal end of the second tube portion, the hingedly mounted flap is oriented at substantially the second tube angle to the second longitudinal central axis, the second tube angle being substantially non-perpendicular.
29. A connection member configured to connect an air circuit to a patient interface to convey a flow of pressurised breathable gas from the air circuit to the patient interface for breathing by a patient, the connection member comprising:
   a first tube portion having a patient-proximal end configured to connect to the patient interface and having a patient-distal end;
   a second tube portion having a patient-proximal end and a patient-distal end configured to connect to the air circuit, wherein at least a portion of the patient-proximal end of the second tube portion is positioned inside at least a portion of the patient-distal end of the first tube portion, the flow of breathable gas being conveyed from the air circuit to the patient interface through the first tube portion and the second tube portion;
   a vent for venting gas exhaled by the patient to the ambient air, the vent formed between the first tube portion and the second tube portion; and
   an anti-asphyxia valve (AAV) comprising an opening to the ambient air and a closure configured to move between a first position in which the closure covers the opening and a second position in which the opening is uncovered;
   wherein the AAV is located in a position on the connection member closer to the patient interface than the vent.
30. The connection member of item 29 wherein the AAV is located proximate the patient-proximal end of the first tube portion.
31. The connection member of any one of items 29 to 30 wherein the closure comprises a hingedly mounted flap.
32. The connection member of item 31 wherein the flap is mounted to the first tube portion.
33. The connection member of item 32 wherein the flap comprises a base portion configured to fit into a first aperture in the first tube portion.
34. The connection member of item 33 wherein the base portion is configured to fit into the first aperture via a snap-fit connection.
35. The connection member of any one of items 29 to 34 wherein the opening is positioned to face an anterior direction when in use.
36. The connection of member of any one of items 29 to 35 further comprising a tube connector configured to connect the second tube portion to an outer surface of the first tube portion.
37. The connection member of item 36 wherein the tube connector comprises a third tube portion mounted to an outer surface of the second tube portion, wherein the third tube portion surrounds the patient-distal end of the first tube portion.
38. The connection member of item 37 wherein the first tube portion comprises a connecting portion configured to fit into a second aperture in the third tube portion.
39. The connection member of item 38 wherein the third tube portion comprises a connecting portion configured to fit into a second aperture in the first tube portion.
40. The connection member of any one of items 38 to 39 wherein the connecting portion fits into the second aperture via a snap-fit connection.
41. The connection member of any one of items 29 to 40, wherein the vent comprises a plurality of vent slots arranged in a region between the first tube portion and the second tube portion.
42. The connection member of item 41, wherein the plurality of vent slots are arranged around the second tube portion.
43. The connection member of any one of items 41 to 42 wherein the vent slots are formed between a plurality of ribs extending between an outer surface of the second tube portion and an inner surface of the first tube portion.
44. The connection member of any one of items 41 to 43 wherein the vent comprises four vent slots.
45. The connection member of any one of items 41 to 44 wherein the plurality of vent slots are equally spaced apart.
46. The connection member of any one of items 29 to 45 wherein a plane of the patient-proximal end of the first tube portion is arranged at a substantially non-perpendicular angle to the first longitudinal central axis.
47. The connection member of item 46 wherein the plane of the patient-proximal end of the first tube portion is angled with respect to the first longitudinal central axis so that, in use, the connection member is directed in an anterior-inferior direction.
48. The connection member of any one of items 29 to 47 wherein the first tube portion is cylindrical in shape.
49. The connection member of any one of items 29 to 48 wherein the second tube portion is cylindrical in shape.
50. The connection member of item 49, when dependent on claims 48 and 41, wherein the vent slots are arranged around an annular region between the first and second tube portions.
51. The connection member of any one of items 29 to 50 wherein the first longitudinal central axis and the second longitudinal central axis are axially aligned.
52. The connection member of any one of items 29 to 51 wherein, when the closure is in the second position, the closure substantially covers the patient-proximal end of the second tube portion thereby preventing flow of gas between the first tube portion and the second tube portion during use.
53. The connection member of item 52, when dependent on item 32, wherein in the second position the hingedly mounted flap contacts the patient-proximal end of the second tube portion.
54. The connection member of item 53 wherein the patient-proximal end of the second tube portion is at a second tube angle to the second longitudinal central axis and, when the hingedly mounted flap contacts the patient-proximal end of the second tube portion, the hingedly mounted flap is oriented at substantially the second tube angle to the second longitudinal central axis, the second tube angle being substantially non-perpendicular.
55. An air circuit assembly configured to fluidly connect a respiratory therapy device to a patient interface, the air circuit assembly comprising
   an air circuit; and
   a connection member of any one of items 1 to 54.
56. A patient interface assembly comprising:
   a connection member of any one of items 1 to 54; and
   a patient interface, comprising:
      a plenum chamber pressurisable to a therapeutic pressure of at least 6 cmH₂O above ambient air pressure, said plenum chamber including a plenum chamber inlet port sized and structured to receive a flow of air at the therapeutic pressure for breathing by a patient; and
      a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use.
57. The patient interface assembly of item 56 wherein the plenum chamber is formed from a flexible material.
58. The patient interface of item 57 wherein the plenum chamber is formed from silicone.
59. The patient interface of any one of items 56 to 58 wherein the seal-forming structure is configured to form a seal around both the patient's nose and mouth in use.
60. The patient interface of item 59 wherein the seal-forming structure is configured to form a seal over the bridge of the patient's nose in use.
61. The patient interface of any one of items 59 or 60 wherein the seal-forming structure comprises an oral portion and a nasal portion.
62. The patient interface of item 61 wherein the nasal portion comprises a lip superior portion which contacts the lip superior of the patient in use.
63. The patient interface of item 62 wherein the nasal portion comprises a superior-facing medial portion which contacts the inferior and partially anterior surfaces of the patient's pronasale in use.
64. The patient interface of any one of items 61 to 63 wherein the oral portion comprises a lip inferior portion which contacts the chin region of the patient in use.
65. The patient interface of any one of items 61 to 64 wherein the oral portion comprises an oral hole peripheral portion which contacts the cheeks of the patient in use.

## Claims

1. A connection member (6000) configured to connect an air circuit (4170) to a patient interface (3000) to convey a flow of pressurised breathable gas from the air circuit (4170) to the patient interface (3000) for breathing by a patient (1000), the connection member (6000) comprising:
a first tube portion (6110) having a patient-proximal end configured to connect to the patient interface (3000) and having a patient-distal end;
a second tube portion (6120) having a patient-proximal end and a patient-distal end configured to connect to the air circuit (4170), wherein at least a portion of the patient-proximal end of the second tube portion (6120) is positioned inside at least a portion of the patient-distal end of the first tube portion (6110), the flow of breathable gas being conveyed from the air circuit (4170) to the patient interface (3000) through the first tube portion (6110) and the second tube portion (6120);
a vent (6100) for venting gas exhaled by the patient to the ambient air, the vent formed between the first tube portion (6110) and the second tube portion (6120); and
an anti-asphyxia valve (AAV) (6200) comprising an opening (6210) to the ambient air and a closure (6220) configured to move between a first position in which the closure covers the opening and a second position in which the opening is uncovered,
wherein the AAV (6200) is located closer to the patient-proximal end of the first tube portion (6110) than the vent (6100).

2. The connection member (6000) of claim 1 wherein the AAV (6200) is located proximate the patient-proximal end of the first tube portion (6110).

3. The connection member (6000) of any one of claims 1 to 2 wherein the closure (6220) comprises a hingedly mounted flap (6221), and preferably wherein the flap (6221) is mounted to the first tube portion (6110).

4. The connection member (6000) of claim 3 wherein the flap (6221) comprises a base portion (6222) configured to fit into a first aperture (6117) in the first tube portion (6110), and preferably wherein the base portion (6222) is configured to fit into the first aperture (6117) via a snap-fit connection.

5. The connection of member (6000) of any one of claims 1 to 4 further comprising a tube connector (6150) configured to connect the second tube portion (6120) to an outer surface of the first tube portion (6110).

6. The connection member (6000) of claim 5 wherein the tube connector (6150) comprises a third tube portion (6155) mounted to an outer surface of the second tube portion (6120), wherein the third tube portion (6155) surrounds the patient-distal end of the first tube portion (6110).

7. The connection member (6000) of claim 6 wherein the first tube portion (6110) comprises a connecting portion (6116) configured to fit into a second aperture in the third tube portion (6155).

8. The connection member (6000) of claim 7 wherein the third tube portion (6155) comprises a connecting portion configured to fit into a second aperture (6156) in the first tube portion (6110), and preferably wherein the connecting portion fits into the second aperture (6156) via a snap-fit connection.

9. The connection member (6000) of any one of claims 1 to 8, wherein the vent (6100) comprises a plurality of vent slots (7300) arranged in a region between the first tube portion (6110) and the second tube portion (6120).

10. The connection member (6000) of claim 9, wherein the plurality of vent slots (7300) are arranged around the second tube portion (6120), and wherein:
a) the plurality of vent slots (7300) are formed between a plurality of ribs extending between an outer surface of the second tube portion (6120) and an inner surface of the first tube portion (6110);
b) the vent (6100) comprises four vent slots (7300);
c) the plurality of vent slots (7300) are equally spaced apart; and/or
d) the vent slots (7300) are arranged around an annular region between the first and second tube portions.

11. The connection member (6000) of any one of claims 1 to 10 wherein a plane of the patient-proximal end of the first tube portion (6110) is arranged at a substantially non-perpendicular angle to the first longitudinal central axis, and preferably wherein the plane of the patient-proximal end of the first tube portion (6110) is angled with respect to the first longitudinal central axis so that, in use, the connection member (6000) is directed in an anterior-inferior direction.

12. The connection member (6000) of any one of claims 1 to 11 wherein the first tube portion (6110) and/or the second tube portion (6120) is cylindrical in shape.

13. The connection member (6000) of any one of claims 1 to 12 wherein the first longitudinal central axis and the second longitudinal central axis are axially aligned.

14. The connection member (6000) of any one of claims 1 to 13 wherein, when the closure (6220) is in the second position, the closure (6220) substantially covers the patient-proximal end of the second tube portion (6120) thereby preventing flow of gas between the first tube portion (6110) and the second tube portion (6120) during use.
The connection member of claim 14, when dependent on claim 3, wherein in the second position the hingedly mounted flap (6221) contacts the patient-proximal end of the second tube portion (6120), and preferably wherein the patient-proximal end of the second tube portion (6120) is at a second tube angle to the second longitudinal central axis and, when the hingedly mounted flap (6221) contacts the patient-proximal end of the second tube portion (6120), the hingedly mounted flap (6221) is oriented at substantially the second tube angle to the second longitudinal central axis, the second tube angle being substantially non-perpendicular.
